(19) Europäisches Patentamt / European Patent Office / Office européen des brevets

(11) **EP 4 786 452 A1**

(12) **EUROPEAN PATENT APPLICATION**
published in accordance with Art. 153(4) EPC

(43) Date of publication:
**05.08.2026 Bulletin 2026/32**

(21) Application number: **24871018.8**

(22) Date of filing: **27.09.2024**

(51) International Patent Classification (IPC):
***C07D 213/84*** (2006.01) ***C07D 213/75*** (2006.01)
***C07C 255/60*** (2006.01) ***C07C 235/38*** (2006.01)
***A61K 31/277*** (2006.01) ***A61K 31/44*** (2006.01)
***A61P 5/28*** (2006.01) ***A61P 17/14*** (2006.01)

(52) Cooperative Patent Classification (CPC):
**A61K 31/277; A61K 31/44; A61P 5/28; A61P 17/14; C07C 235/38; C07C 255/60; C07D 213/75; C07D 213/84**

(86) International application number:
**PCT/CN2024/121910**

(87) International publication number:
**WO 2025/067483 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311282820**
**05.07.2024 CN 202410900706**

(71) Applicants:
- **China Pharmaceutical University**
**Nanjing, Jiangsu 211198 (CN)**
- **Jiangsu Simcere Pharmaceutical Co., Ltd.**
**Nanjing, Jiangsu 210042 (CN)**

(72) Inventors:
- **ZHANG, Yan**
**Nanjing, Jiangsu 211198 (CN)**
- **JIAO, Yu**
**Nanjing, Jiangsu 211198 (CN)**
- **HUANG, Liancheng**
**Nanjing, Jiangsu 210042 (CN)**
- **ZHAO, Siqi**
**Beijing 102206 (CN)**
- **TANG, Feng**
**Shanghai 201318 (CN)**
- **LIU, Le**
**Nanjing, Jiangsu 210042 (CN)**
- **CHEN, Yadong**
**Nanjing, Jiangsu 211198 (CN)**
- **LU, Tao**
**Nanjing, Jiangsu 211198 (CN)**
- **PENG, Shaoping**
**Nanjing, Jiangsu 210042 (CN)**
- **WANG, Feng**
**Nanjing, Jiangsu 210042 (CN)**

(74) Representative: **V.O.**
**P.O. Box 87930**
**2508 DH Den Haag (NL)**

# (54) AMIDE COMPOUNDS

(57) Provided are compounds of formula (I) or stereoisomers or pharmaceutically acceptable salts thereof, pharmaceutical compositions comprising same, and the use thereof as androgen receptor (AR) antagonists in the treatment or prevention of androgen receptor-mediated diseases, preferably for the treatment of androgen receptor-mediated alopecia, such as androgenetic alopecia.

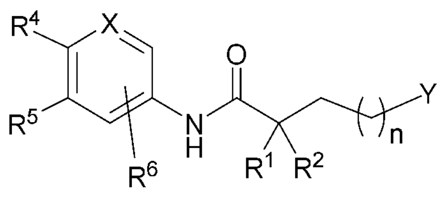

(I)

EP 4 786 452 A1

## Description

### CROSS-REFERENCE TO RELATED APPLICATIONS

The present disclosure claims the priority and benefit to the following Chinese patent applications filed with China National Intellectual Property Administration: Chinese Patent Application No. 202311282820.X filed on September 28, 2023 and Chinese Patent Application No. 202410900706.7 filed on July 5, 2024, which are incorporated herein by reference in their entirety.

### TECHNICAL FIELD

The present disclosure relates to the field of pharmaceutical chemistry, and in particular to an amide compound or a stereoisomer thereof or a pharmaceutically acceptable salt thereof, a pharmaceutical composition comprising same, and use thereof as an androgen receptor (AR) antagonist.

### BACKGROUND

Androgen receptor (AR) is a member of the nuclear receptor family. AR comprises four main regions: an N-terminal domain (NTD) for transcriptional control, a DNA binding domain (DBD), a hinge region, and a C-terminal ligand binding domain (LBD). Its activation is closely associated with the development of diseases such as benign prostate hyperplasia, prostate cancer, seborrhea, acne, premenstrual syndrome, lung cancer, polycystic ovarian syndrome, hirsutism, and alopecia. Therefore, the androgen receptor is an important target in various fields of drug discovery.

Androgenetic alopecia (AGA) is the most common type of alopecia in clinic practice at present. Although its specific pathogenesis has not been completely revealed yet, most researchers believe that it is related to androgen metabolism. Studies have shown that the effect of androgen on susceptible hair follicles is increased due to the increased expression of androgen receptor gene and/or type II 5$\alpha$ reductase gene in hair follicles of the alopecia area. For androgenetic alopecia, the dermal cells in susceptible hair follicles contain specific 5$\alpha$ reductase type II that can convert androgen testosterone circulating in the blood to this region into dihydrotestosterone, which causes a series of reactions by binding to the androgen receptor in the cells, resulting in progressive miniaturization and alopecia in the hair follicles and even atrichia. Currently, the FDA has only approved minoxidil and finasteride for the treatment of androgenetic alopecia. Minoxidil is a potassium channel opener for topical use, with its exact mechanism in androgenetic alopecia incompletely elucidated. However, minoxidil has a long time to onset, as well as adverse reactions such as pruritus, contact dermatitis, and aggravated alopecia after discontinuation. Finasteride is an inhibitor of the 5-$\alpha$ reductase type II that decreases the level of dihydrotestosterone in the serum and scalp for the treatment of AGA in males. However, erectile dysfunction, ejaculation disorder, and hyposexuality due to the long-term use of finasteride deter patients with alopecia. Therefore, the research on the antagonistic activity of novel molecules against AR is of great significance for research on androgenetic alopecia.

### SUMMARY

In one aspect, the present disclosure provides a compound of formula (I) or a stereoisomer thereof or a pharmaceutically acceptable salt thereof,

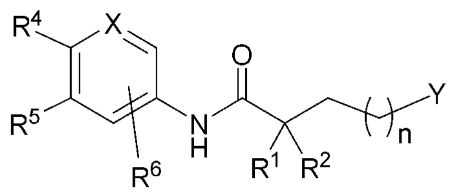

(I)

wherein,

X is selected from CH or N;
Y is selected from OH, COOH, $-CONH_2$, $-COOR^3$, or $-CONR^{3'}R^3$;
$R^1$ is selected from OH or $-O-C_1-C_6$ alkyl;

$R^2$ is selected from H, deuterium, $C_1$-$C_6$ alkyl, $C_1$-$C_6$ deuterated alkyl, or $C_1$-$C_6$ haloalkyl;

$R^3$ is selected from $C_1$-$C_6$ alkyl, $C_1$-$C_6$ deuterated alkyl, $C_3$-$C_6$ cycloalkyl, 4- to 10-membered heterocyclyl, $C_6$-$C_{10}$ aryl, or 5- to 10-membered heteroaryl, wherein the $C_1$-$C_6$ alkyl, $C_3$-$C_6$ cycloalkyl, 4- to 10-membered heterocyclyl, $C_6$-$C_{10}$ aryl, or 5- to 10-membered heteroaryl is optionally substituted with $R^{3a}$;

$R^{3a}$ is selected from halogen, OH, CN, $NH_2$, $-COR^{3b}$, $-COOR^{3b}$, $-NHCOR^{3b}$, $-CONHR^{3b}$, -O-$C_1$-$C_6$ alkyl, $C_1$-$C_6$ alkyl, phenyl, 4- to 6-membered heterocyclyl, or 5- to 6-membered heteroaryl, wherein the $C_1$-$C_6$ alkyl, phenyl, 4- to 6-membered heterocyclyl, or 5- to 6-membered heteroaryl is optionally substituted with $R^{3c}$;

$R^{3b}$ is selected from H or $C_1$-$C_6$ alkyl;

$R^{3c}$ is selected from halogen, OH, $C_1$-$C_6$ alkyl, or -O-$C_1$-$C_6$ alkyl;

$R^{3'}$ is selected from H or $C_1$-$C_6$ alkyl;

$R^4$ is selected from $NO_2$, CN, halogen, $C_1$-$C_6$ alkyl, $C_1$-$C_6$ haloalkyl, or -O-$C_1$-$C_6$ alkyl;

$R^5$ is selected from halogen, $C_1$-$C_6$ alkyl, $C_1$-$C_6$ haloalkyl, -COO$C_1$-$C_6$ alkyl, -S-$C_1$-$C_6$ alkyl, -O-$C_1$-$C_6$ alkyl, -Se-$C_1$-$C_6$ alkyl, -S(O)-$C_1$-$C_6$ alkyl, or -$S(O)_2$-$C_1$-$C_6$ alkyl;

$R^6$ is selected from H, halogen, $C_1$-$C_6$ alkyl, $C_1$-$C_6$ haloalkyl, or -O-$C_1$-$C_6$ alkyl;

n is selected from 1, 2, 3, 4, 5, 6, 7, 8, or 9;

with the provision that, when Y is selected from OH, n is selected from 1, 2, or 3.

In another aspect, the present disclosure provides a pharmaceutical composition, comprising the compound of formula (I) or the stereoisomer thereof or the pharmaceutically acceptable salt thereof and a pharmaceutically acceptable excipient.

In yet another aspect, the present disclosure provides a method for preventing or treating an androgen receptor-mediated disease in a mammal, comprising administering to a mammal in need of the treatment a therapeutically effective amount of the compound of formula (I) or the stereoisomer thereof or the pharmaceutically acceptable salt thereof, or the pharmaceutical composition thereof. In yet another aspect, the present disclosure provides use of the compound of formula (I) or the stereoisomer thereof or the pharmaceutically acceptable salt thereof or the pharmaceutical composition thereof in preparing a medicament for preventing or treating an androgen receptor-mediated disease.

In still another aspect, the present disclosure provides the compound of formula (I) or the stereoisomer thereof or the pharmaceutically acceptable salt thereof, or the pharmaceutical composition thereof for use in preventing or treating an androgen receptor-mediated disease.

In still another aspect, the present disclosure provides use of the compound of formula (I) or the stereoisomer thereof or the pharmaceutically acceptable salt thereof, or the pharmaceutical composition thereof in preventing or treating an androgen receptor-mediated disease.

## DETAILED DESCRIPTION

The present disclosure provides a compound of formula (I) or a stereoisomer thereof or a pharmaceutically acceptable salt thereof,

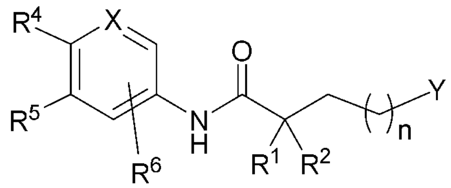

(I)

wherein,

X is selected from CH or N;

Y is selected from OH, COOH, $-CONH_2$, $-COOR^3$, or $-CONR^{3'}R^3$;

$R^1$ is selected from OH or -O-$C_1$-$C_6$ alkyl;

$R^2$ is selected from H, deuterium, $C_1$-$C_6$ alkyl, $C_1$-$C_6$ deuterated alkyl, or $C_1$-$C_6$ haloalkyl;

$R^3$ is selected from $C_1$-$C_6$ alkyl, $C_1$-$C_6$ deuterated alkyl, $C_3$-$C_6$ cycloalkyl, 4- to 10-membered heterocyclyl, $C_6$-$C_{10}$ aryl, or 5- to 10-membered heteroaryl, wherein the $C_1$-$C_6$ alkyl, $C_3$-$C_6$ cycloalkyl, 4- to 10-membered heterocyclyl, $C_6$-$C_{10}$ aryl, or 5- to 10-membered heteroaryl is optionally substituted with $R^{3a}$;

$R^{3a}$ is selected from halogen, OH, CN, $NH_2$, $-COR^{3b}$, $-COOR^{3b}$, $-NHCOR^{3b}$, $-CONHR^{3b}$, -O-$C_1$-$C_6$ alkyl, $C_1$-$C_6$ alkyl,

phenyl, 4- to 6-membered heterocyclyl, or 5- to 6-membered heteroaryl, wherein the $C_1$-$C_6$ alkyl, phenyl, 4- to 6-membered heterocyclyl, or 5- to 6-membered heteroaryl is optionally substituted with $R^{3c}$;

$R^{3b}$ is selected from H or $C_1$-$C_6$ alkyl;

$R^{3c}$ is selected from halogen, OH, $C_1$-$C_6$ alkyl, or -O-$C_1$-$C_6$ alkyl;

$R^{3'}$ is selected from H or $C_1$-$C_6$ alkyl;

$R^4$ is selected from $NO_2$, CN, halogen, $C_1$-$C_6$ alkyl, $C_1$-$C_6$ haloalkyl, or -O-$C_1$-$C_6$ alkyl;

$R^5$ is selected from halogen, $C_1$-$C_6$ alkyl, $C_1$-$C_6$ haloalkyl, -COO$C_1$-$C_6$ alkyl, -S-$C_1$-$C_6$ alkyl, -O-$C_1$-$C_6$ alkyl, -Se-$C_1$-$C_6$ alkyl, -S(O)-$C_1$-$C_6$ alkyl, or -$S(O)_2$-$C_1$-$C_6$ alkyl;

$R^6$ is selected from H, halogen, $C_1$-$C_6$ alkyl, $C_1$-$C_6$ haloalkyl, or -O-$C_1$-$C_6$ alkyl;

n is selected from 1, 2, 3, 4, 5, 6, 7, 8, or 9;

with the provision that, when Y is selected from OH, n is selected from 1, 2, or 3.

In some embodiments, X is N.

In some embodiments, Y is selected from COOH, -$CONH_2$, -$COOR^3$, or -$CONR^{3'}R^3$. In some embodiments, Y is selected from -$CONH_2$, -$COOR^3$, or -$CONR^{3'}R^3$.

In some embodiments, Y is selected from -$COOR^3$ or -$CONR^{3'}R^3$.

In some embodiments, Y is -$COOR^3$.

In some embodiments, Y is selected from OH, COOH, -$COOCH_2CH_3$, -$COOCH_3$, -$COOCH(CH_3)_2$, -$COOC(CH_3)_3$, -$CONHCH_3$, or -$CONH_2$. In some embodiments, Y is selected from -$COOCH_2CH_3$, -$COOCH_3$, -$COOCH(CH_3)_2$, -$COOC(CH_3)_3$, or -$CONHCH_3$.

In some embodiments, Y is selected from -$COOCH_2CH_3$, -$COOCH_3$, -$COOCH(CH_3)_2$, or -$COO(CH_3)_3$.

In some embodiments, $R^1$ is OH.

In some embodiments, $R^2$ is selected from $C_1$-$C_6$ alkyl, $C_1$-$C_6$ deuterated alkyl, or $C_1$-$C_6$ haloalkyl.

In some embodiments, $R^2$ is selected from H, $C_1$-$C_6$ alkyl, or $C_1$-$C_6$ haloalkyl. In some embodiments, $R^2$ is selected from H, $C_1$-$C_3$ alkyl, or $C_1$-$C_3$ haloalkyl.

In some embodiments, $R^2$ is selected from $C_1$-$C_6$ alkyl or $C_1$-$C_6$ haloalkyl. In some embodiments, $R^2$ is selected from $C_1$-$C_3$ alkyl or $C_1$-$C_3$ haloalkyl.

In some embodiments, $R^2$ is selected from H, $CH_3$, or $CF_3$.

In some embodiments, $R^2$ is selected from $CH_3$ or $CF_3$. In some embodiments, $R^2$ is selected from $CH_3$.

In some embodiments, $R^1$ is OH, and $R^2$ is $CH_3$.

In some embodiments, $R^3$ is selected from $C_1$-$C_6$ alkyl, $C_1$-$C_6$ deuterated alkyl, $C_3$-$C_6$ cycloalkyl, 4-to 6-membered heterocyclyl, $C_6$-$C_{10}$ aryl, or 5- to 6-membered heteroaryl, wherein the $C_1$-$C_6$ alkyl, $C_3$-$C_6$ cycloalkyl, 4- to 6-membered heterocyclyl, $C_6$-$C_{10}$ aryl, or 5- to 6-membered heteroaryl is optionally substituted with $R^{3a}$.

In some embodiments, $R^3$ is selected from $C_1$-$C_6$ alkyl or $C_1$-$C_6$ deuterated alkyl, wherein the $C_1$-$C_6$ alkyl is optionally substituted with $R^{3a}$.

In some embodiments, $R^3$ is $C_1$-$C_6$ alkyl. In some embodiments, $R^3$ is $C_1$-$C_4$ alkyl, wherein the $C_1$-$C_4$ alkyl is optionally substituted with $R^{3a}$.

In some embodiments, $R^3$ is unsubstituted $C_1$-$C_6$ alkyl. In some embodiments, $R^3$ is unsubstituted $C_1$-$C_4$ alkyl.

In some embodiments, $R^3$ is selected from methyl, ethyl, isopropyl, or tert-butyl. In some embodiments, $R^3$ is selected from unsubstituted methyl, unsubstituted ethyl, unsubstituted isopropyl, or unsubstituted tert-butyl.

In some embodiments, $R^{3'}$ is H or $C_1$-$C_4$ alkyl. In some embodiments, $R^{3'}$ is H.

In some embodiments, $R^{3a}$ is selected from halogen, OH, CN, $NH_2$, -$COR^{3b}$, -$COOR^{3b}$, -$NHCOR^{3b}$, -$CONHR^{3b}$, -O-$C_1$-$C_6$ alkyl, or $C_1$-$C_6$ alkyl, wherein the $C_1$-$C_6$ alkyl is optionally substituted with $R^{3c}$. In some embodiments, $R^{3a}$ is selected from halogen, OH, CN, $NH_2$, -O-$C_1$-$C_6$ alkyl, or $C_1$-$C_6$ alkyl, wherein the $C_1$-$C_6$ alkyl is optionally substituted with $R^{3c}$.

In some embodiments, $R^4$ is selected from halogen or CN.

In some embodiments, $R^4$ is selected from F, Cl, or CN.

In some embodiments, $R^4$ is CN.

In some embodiments, $R^5$ is selected from halogen, $C_1$-$C_6$ alkyl, $C_1$-$C_6$ haloalkyl, -COO$C_1$-$C_6$ alkyl, -S-$C_1$-$C_6$ alkyl, or -O-$C_1$-$C_6$ alkyl.

In some embodiments, $R^5$ is selected from $C_1$-$C_6$ haloalkyl, -S-$C_1$-$C_6$ alkyl, -O-$C_1$-$C_6$ alkyl, -Se-$C_1$-$C_6$ alkyl, -S(O)-$C_1$-$C_6$ alkyl, or -$S(O)_2$-$C_1$-$C_6$ alkyl. In some embodiments, $R^5$ is selected from $C_1$-$C_3$ haloalkyl, -S-$C_1$-$C_3$ alkyl, -O-$C_1$-$C_3$ alkyl, -Se-$C_1$-$C_3$ alkyl, -S(O)-$C_1$-$C_3$ alkyl, or -$S(O)_2$-$C_1$-$C_3$ alkyl. In some embodiments, $R^5$ is selected from $C_1$-$C_3$ haloalkyl, -S-$C_1$-$C_3$ alkyl, -O-$C_1$-$C_3$ alkyl, or -Se-$C_1$-$C_3$ alkyl.

In some embodiments, $R^5$ is selected from $C_1$-$C_6$ haloalkyl, -S-$C_1$-$C_6$ alkyl, or -O-$C_1$-$C_6$ alkyl. In some embodiments, $R^5$ is selected from $C_1$-$C_3$ haloalkyl, -S-$C_1$-$C_3$ alkyl, or -O-$C_1$-$C_3$ alkyl.

In some embodiments, $R^5$ is selected from $CF_3$, -S-$CH_3$, -O-$CH_3$, -Se-$CH_3$, -S(O)-$CH_3$, or -$S(O)_2$-$CH_3$.

In some embodiments, $R^5$ is selected from $CF_3$, -S-$CH_3$, or -O-$CH_3$. In some embodiments, $R^5$ is selected from

$CF_3$ or $-S-CH_3$. In some embodiments, $R^5$ is $CF_3$.

In some embodiments, $R^6$ is selected from H or halogen.

In some embodiments, $R^6$ is selected from H, F, or Cl.

In some embodiments, $R^6$ is H.

In some embodiments, n is selected from 1, 2, or 3.

In some embodiments, n is selected from 1 or 2.

In some embodiments, n is 2.

In some embodiments, X is selected from CH or N; Y is selected from OH, COOH, $-CONH_2$, $-COOR^3$, or $-CONR^{3'}R^3$, wherein $R^3$ is unsubstituted $C_1$-$C_6$ alkyl, and $R^{3'}$ is H or $C_1$-$C_6$ alkyl; $R^1$ is OH; $R^2$ is selected from H, $C_1$-$C_3$ alkyl, or $C_1$-$C_3$ haloalkyl; $R^4$ is CN; $R^5$ is selected from $C_1$-$C_3$ haloalkyl, $-S-C_1$-$C_3$ alkyl, $-O-C_1$-$C_3$ alkyl, $-Se-C_1$-$C_3$ alkyl, $-S(O)-C_1$-$C_3$ alkyl, or $-S(O)_2-C_1$-$C_3$ alkyl; and $R^6$ is H.

In some embodiments, X is selected from CH or N; Y is selected from OH, COOH, $-COOCH_2CH_3$, $-COOCH_3$, $-COOCH(CH_3)_2$, $-COOC(CH_3)_3$, $-CONHCH_3$, or $-CONH_2$; $R^1$ is OH; $R^2$ is selected from H, $CH_3$, or $CF_3$; $R^4$ is CN; $R^5$ is selected from $CF_3$, $-S-CH_3$, $-O-CH_3$, $-Se-CH_3$, $-S(O)-CH_3$, or $-S(O)_2-CH_3$; and $R^6$ is H.

In some embodiments, X is selected from CH or N; Y is selected from OH or $-COOR^3$, wherein $R^3$ is unsubstituted $C_1$-$C_6$ alkyl; $R^1$ is OH; $R^2$ is selected from H or $C_1$-$C_3$ alkyl; $R^4$ is CN; $R^5$ is selected from $C_1$-$C_3$ haloalkyl, $-S-C_1$-$C_3$ alkyl, $-O-C_1$-$C_3$ alkyl, or $-Se-C_1$-$C_3$ alkyl; and $R^6$ is H.

In some embodiments, X is selected from CH or N; Y is selected from OH, $-COOCH_2CH_3$, $-COOCH_3$, $-COOCH(CH_3)_2$, or $-COOC(CH_3)_3$; $R^1$ is OH; $R^2$ is selected from H or $CH_3$; $R^4$ is CN; $R^5$ is selected from $CF_3$, $-S-CH_3$, $-O-CH_3$, or $-Se-CH_3$; and $R^6$ is H.

In some embodiments, the compound of formula (I) or the stereoisomer thereof or the pharmaceutically acceptable salt thereof is selected from a compound of formula (II) or a stereoisomer thereof or a pharmaceutically acceptable salt thereof,

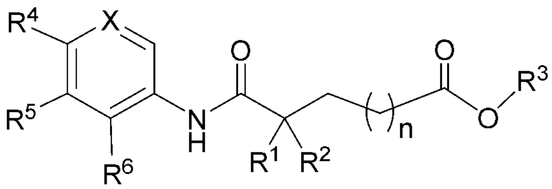

(II)

wherein X, $R^1$, $R^2$, $R^3$, $R^4$, $R^5$, $R^6$, and n are as defined above.

In some embodiments, the compound of formula (I) or the stereoisomer thereof or the pharmaceutically acceptable salt thereof is selected from a compound below or a stereoisomer thereof or a pharmaceutically acceptable salt thereof:

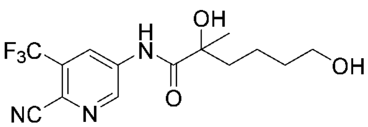
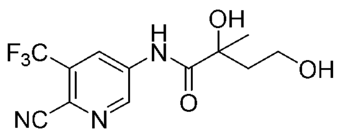
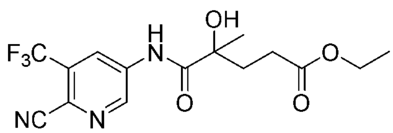

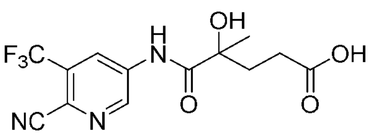
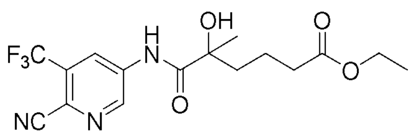
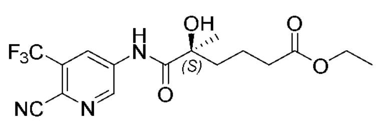

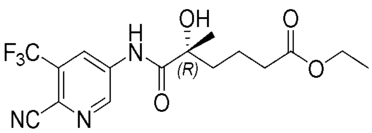
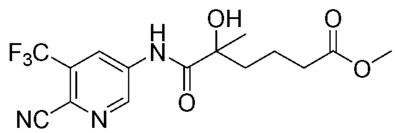
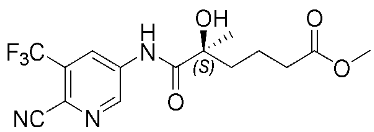

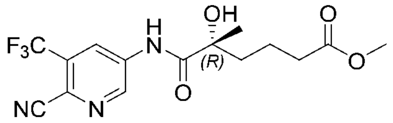
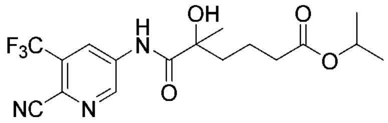
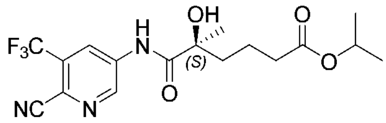

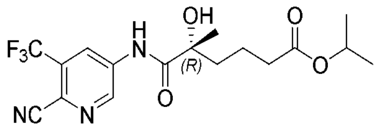
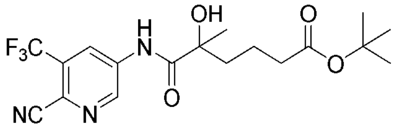
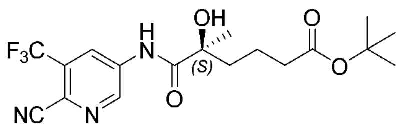

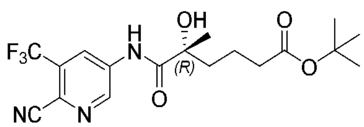
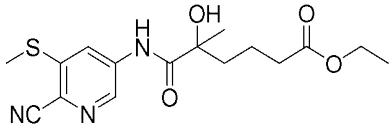
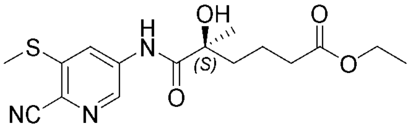

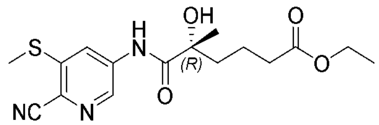
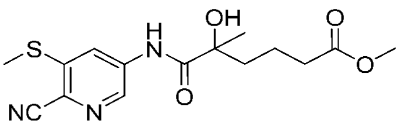
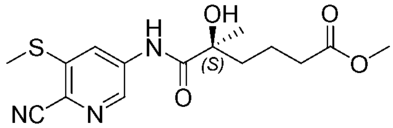

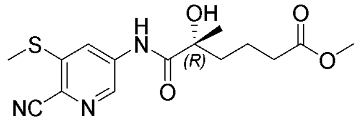
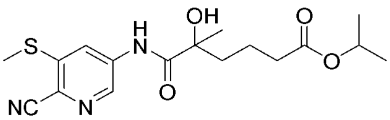
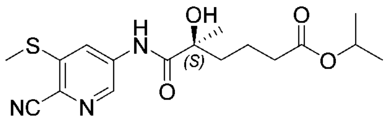

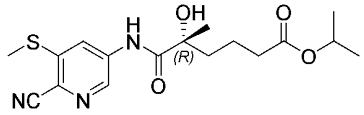
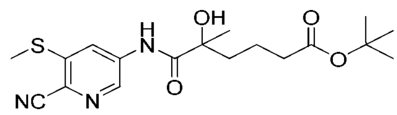
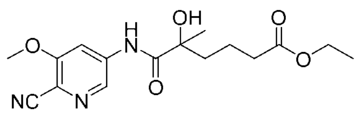

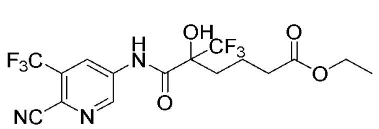
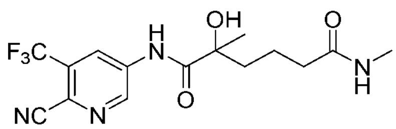
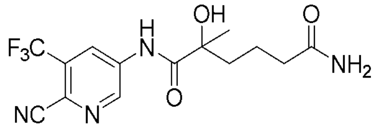

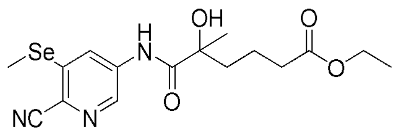
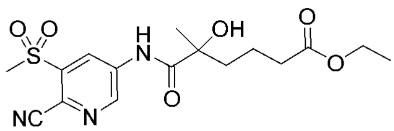
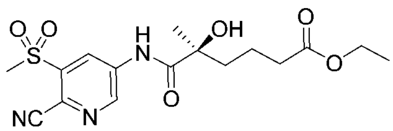

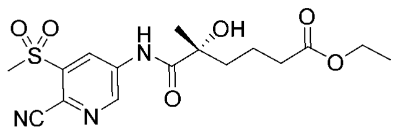
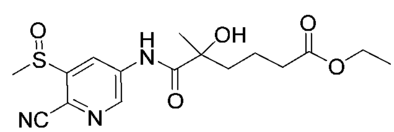
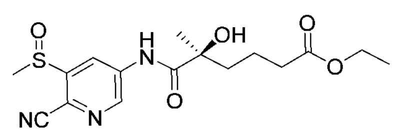

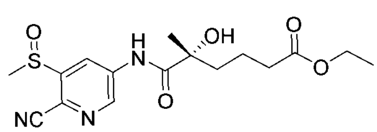
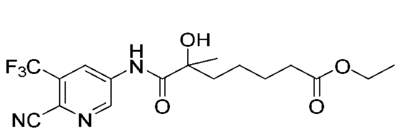
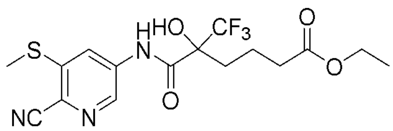

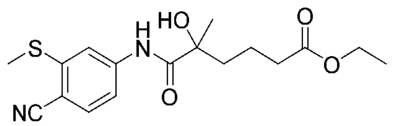
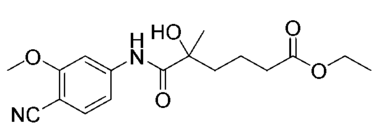
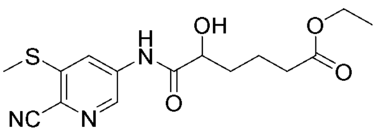

or

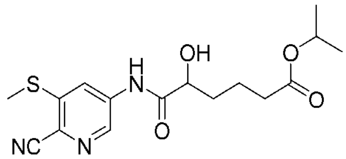
.

In another aspect, the present disclosure provides a pharmaceutical composition, comprising the compound of formula (I) or (II) or the stereoisomer thereof or the pharmaceutically acceptable salt thereof and a pharmaceutically acceptable excipient.

In another aspect, the present disclosure provides a method for preventing or treating an androgen receptor-mediated disease in a mammal, comprising administering to a mammal, preferably a human, in need of the treatment a therapeutically effective amount of the compound of formula (I) or (II) or the stereoisomer thereof or the pharmaceutically acceptable salt thereof, or the pharmaceutical composition thereof.

In another aspect, the present disclosure provides use of the compound of formula (I) or (II) or the stereoisomer thereof or the pharmaceutically acceptable salt thereof or the pharmaceutical composition thereof in preparing a medicament for preventing or treating an androgen receptor-mediated disease.

In another aspect, the present disclosure provides use of the compound of formula (I) or (II) or the stereoisomer thereof or the pharmaceutically acceptable salt thereof, or the pharmaceutical composition thereof in preventing or treating an androgen receptor-mediated disease.

In another aspect, the present disclosure provides the compound of formula (I) or (II) or the stereoisomer thereof or the pharmaceutically acceptable salt thereof, or the pharmaceutical composition thereof for use in preventing or treating an androgen receptor-mediated disease.

In some embodiments, the androgen receptor-mediated disease is selected from androgenetic alopecia. Any embodiment of any aspect of the present disclosure may be combined with other embodiments in the case of no contradictions. In addition, in any embodiment of any aspect of the present disclosure, any technical feature is applicable to the technical feature in other embodiments in the case of no contradictions.

Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be interpreted according to its common meaning in the field. When referring to a trade name, it is intended to refer to its corresponding commercial product or its active ingredient.

The illustration method for the racemic or enantiomerically pure compounds herein is from Maehr, J. Chem. Ed. 1985, 62:114-120. Unless otherwise stated, the absolute configuration of a stereogenic center is represented by a wedged bond and a wedged dashed bond (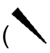 and 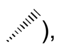), and the relative configuration of a stereogenic center (e.g., *cis*- or *trans*-configuration of alicyclic compounds) is represented by a black solid bond and a dashed bond (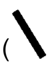 and 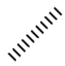).

The term "tautomer" refers to functional isomers resulting from the rapid movement of an atom in a molecule between two positions. The compounds of the present disclosure may exhibit tautomerism. Tautomeric compounds may have two or more interconvertible forms. Tautomers are generally present in an equilibrated form. Trying to separate a single tautomer usually leads to a mixture, the physicochemical properties of which are consistent with the mixture of the compound. The equilibrium depends on the chemical properties of the molecule. For example, in many aliphatic aldehydes and ketones, such as acetaldehyde, the keto form predominates; whereas in phenol, the enol form predominates. In the present disclosure, all tautomeric forms of the compounds are included. The term "stereoisomer" refers to isomers resulting from different spatial arrangements of atoms in a molecule, including *cis*-*trans* isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may be present in the form of a particular geometric isomer or stereoisomer. The form of a particular geometric isomer or stereoisomer may be *cis* and *trans* isomers, *E* and *Z* geometric isomers, (-)- and (+)-enantiomers, (*R*)- and (*S*)-enantiomers, diastereoisomers, (*D*)-isomers, (*L*)-isomers, and racemic mixtures or other mixtures thereof, such as an

enantiomer or diastereoisomer enriched mixture, and all of the above isomers, as well as mixtures thereof, are encompassed within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The asterisk "*" herein denotes a chiral center, indicating that the absolute configuration at the position is one of the S-configuration or the R-configuration.

The term "substituted" means that any one or more hydrogen atoms on a specific atom are replaced by substituents, as long as the valence of the specific atom is normal and the compound resulting from the substitution is stable. When the substituent is oxo (i.e., =O), it means that two hydrogen atoms are replaced, and oxo will not be present on an aromatic group.

The term "optional" or "optionally" means that the subsequently described event or circumstance may or may not occur. The description includes instances where the event or circumstance occurs and instances where the event or circumstance does not. For example, ethyl being "optionally" substituted with halogen means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). Those skilled in the art will appreciate that for any group containing one or more substituents, no substitution or substituting pattern that is spatially impossible and/or cannot be synthesized will be introduced.

When any variable (e.g., $R^a$ or $R^b$) occurs one or more times in the constitution or structure of a compound, the variable is independently defined in each case. For example, if a group is substituted with 2 $R^b$, the definition of each $R^b$ is independent.

When a bond of a substituent is cross-linked to two atoms on a ring, the substituent can be bonded to any atom on the ring. For example, structural unit

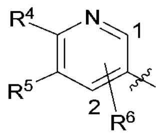

denotes that the H at any one of positions 1 or 2 on the ring is substituted by $R^6$.

Herein, "

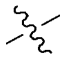

" denotes a linking site.

$C_m$-$C_n$ used herein means that the portion has an integer number of carbon atoms in the range of m-n. For example, the "$C_1$-$C_{10}$" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to a hydrocarbyl group with a general formula of $C_nH_{2n+1}$. The alkyl may be linear or branched. The term "$C_1$-$C_{10}$ alkyl" may be interpreted as a linear or branched saturated hydrocarbon group having 1, 2, 3, 4, 5, 6, 7, 8, 9, or 10 carbon atoms. Specific examples of the alkyl include, but are not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "$C_1$-$C_6$ alkyl" may be interpreted as an alkyl group having 1, 2, 3, 4, 5, or 6 carbon atoms. Specific examples include, but are not limited to, methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, and the like. The term "$C_1$-$C_3$ alkyl" may be interpreted as a linear or branched saturated alkyl group having 1, 2, or 3 carbon atoms. The "$C_1$-$C_{10}$ alkyl" may include the ranges of "$C_1$-$C_6$ alkyl", "$C_1$-$C_3$ alkyl", and the like, and the "$C_1$-$C_6$ alkyl" may further include "$C_1$-$C_3$ alkyl".

The term "deuterated alkyl" refers to an alkyl in which a hydrogen is substituted with deuterium, including monodeuterated alkyl and polydeuterated alkyl. For example, the term "$C_1$-$C_6$ deuterated alkyl" refers to a $C_1$-$C_6$ alkyl as defined above substituted with one or more deuterium, including but not limited to $CD_3$, $CH_2CD_3$, and the like.

The term "haloalkyl" includes monohaloalkyl and polyhaloalkyl. For example, the term "$C_1$-$C_6$ haloalkyl" refers to $C_1$-$C_6$ alkyl as defined above substituted with one or more halogens, including but not limited to trifluoromethyl, 2,2,2-trifluoroethyl, 4-chlorobutyl, 3-bromopropyl, trichloromethyl, pentafluoroethyl, pentachloroethyl, and the like.

The term "cycloalkyl" refers to a fully saturated carbon ring that is present in the form of a monocyclic ring, a fused ring, a bridged ring, a spiro ring, or the like. Unless otherwise specified, the carbon ring is generally a 3- to 10-membered ring. The term "$C_3$-$C_{10}$ cycloalkyl" may be interpreted as a saturated monocyclic, fused, spiro, or bridged ring having 3, 4, 5, 6, 7, 8, 9, or 10 carbon atoms. Specific examples of the cycloalkyl include, but are not limited to, cyclopropyl, cyclobutyl, cyclopentyl, cyclohexyl, cycloheptyl, cyclooctyl, cyclononyl, cyclodecyl, norbornyl (bicyclo[2.2.1]heptyl), bicyclo[2.2.2]octyl, adamantyl, spiro[4.5]decyl, and the like. The term "$C_3$-$C_{10}$ cycloalkyl" may include "$C_3$-$C_6$ cycloalkyl", and the term "$C_3$-$C_6$ cycloalkyl" may be interpreted as a saturated monocyclic or bicyclic hydrocarbon ring having 3, 4, 5, or 6 carbon atoms. Specific examples include, but are not limited to, cyclopropyl, cyclobutyl, cyclopentyl, cyclohexyl, and the like.

The term "heterocyclyl" refers to a fully saturated or partially saturated (non-aromatic heteroaromatic group on the whole) monocyclic, fused cyclic, spiro cyclic, or bridged cyclic group, and ring atoms of the group include 1, 2, 3, 4, or 5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), $-S(=O)_2-$, -S(=O)-, $-P(=O)_2-$, -P(=O)-, -NH-, -S(=O)(=NH)-, -C(=O)NH-, -NHC(=O)NH-, and the like. The term "3- to 10-membered heterocyclyl" refers to a heterocyclyl group with 3, 4, 5, 6, 7, 8, 9, or 10 ring atoms, and ring atoms of the group include 1, 2, 3, 4, or 5 heteroatoms or heteroatom groups independently selected from those described above. The "3- to 10-membered heterocyclyl" includes "4- to 7-membered heterocyclyl", wherein specific examples of 4-membered heterocyclyl include, but are not limited to, azetidinyl, thietanyl, and oxetanyl; specific examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, and 2,5-dihydro-1*H*-pyrrolyl; specific examples of 6-membered heterocyclyl include, but are not limited to, tetrahydropyranyl, piperidyl, morpholinyl, dithianyl, thiomorpholinyl, piperazinyl, trithianyl, tetrahydropyridinyl, and 4*H*-[1,3,4]thiadiazinyl; specific examples of 7-membered heterocyclyl include, but are not limited to, diazepanyl. The heterocyclyl may also be a bicyclic group, wherein specific examples of 5,5-membered bicyclic groups include, but are not limited to, hexahydrocyclopenta[*c*]pyrrol-2(1*H*)-yl; specific examples of 5,6-membered bicyclic groups include, but are not limited to, hexahydropyrrolo[1,2-*a*]pyrazin-2(1*H*)-yl, 5,6,7,8-tetrahydro-[1,2,4]triazolo[4,3-*a*]pyrazinyl, and 5,6,7,8-tetrahydroimidazo[1,5-*a*]pyrazinyl. Optionally, the heterocyclyl may be a benzo-fused ring group of the 4- to 7-membered heterocyclyl described above. Specific examples include, but are not limited to, dihydroisoquinolyl and the like. The "4- to 10-membered heterocyclyl" may include the ranges of "5- to 10-membered heterocyclyl", "4- to 7-membered heterocyclyl", "5- to 6-membered heterocyclyl", "6- to 8-membered heterocyclyl", "4- to 10-membered heterocycloalkyl", "5- to 10-membered heterocycloalkyl", "4- to 7-membered heterocycloalkyl", "5- to 6-membered heterocycloalkyl", "6-to 8-membered heterocycloalkyl", and the like, and the "4- to 7-membered heterocyclyl" may further include the ranges of "4- to 6-membered heterocyclyl", "5- to 6-membered heterocyclyl", "4- to 7-membered heterocycloalkyl", "4- to 6-membered heterocycloalkyl", "5- to 6-membered heterocycloalkyl", and the like. Although some bicyclic heterocyclyl groups herein comprise, in part, one benzene ring or one heteroaromatic ring, the heterocyclyl is still non-aromatic on the whole.

The term "aryl" refers to an aromatic all-carbon monocyclic or fused polycyclic group with a conjugated $\pi$-electron system. The aryl may have 6-20 carbon atoms, 6-14 carbon atoms, or 6-12 carbon atoms. The term "$C_6$-$C_{20}$ aryl" may be interpreted as an aryl group having 6 to 20 carbon atoms, particularly a ring having 6 carbon atoms ("$C_6$ aryl"), such as phenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthryl. The term "$C_6$-$C_{10}$ aryl" may be interpreted as an aryl group having 6 to 10 carbon atoms, particularly a ring having 6 carbon atoms ("$C_6$ aryl"), such as phenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl. The term "$C_6$-$C_{20}$ aryl" may include "$C_6$-$C_{10}$ aryl".

The term "heteroaryl" refers to an aromatic cyclic group having an aromatic monocyclic or fused polycyclic system, which contains at least one ring atom selected from N, O, and S, with the remaining ring atoms being C. The term "5- to 10-membered heteroaryl" may be interpreted as including an aromatic monocyclic or bicyclic ring system, which has 5, 6, 7, 8, 9, or 10 ring atoms, particularly 5, 6, 9, or 10 ring atoms, and comprises 1, 2, 3, 4, or 5, preferably 1, 2, or 3, heteroatoms independently selected from N, O, and S. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, or the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, isoindolyl, or the like; and pyridyl, pyridazinyl, pyrimidinyl, pyrazinyl, triazinyl, or the like, and benzo derivatives thereof, such as quinolyl, quinazolinyl, isoquinolyl, and the like; and azocinyl, indolizinyl, purinyl, or the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, or the like. The term "5- to 6-membered heteroaryl" refers to an aromatic ring system, which has 5 or 6 ring atoms and comprises 1, 2, or 3, preferably 1-2, heteroatoms independently selected from N, O, and S.

The term "halo" or "halogen" refers to fluorine, chlorine, bromine, or iodine.

The term "hydroxy" refers to the -OH group.

The term "cyano" refers to the -CN group.

The term "amino" refers to the $-NH_2$ group.

The term "therapeutically effective amount" means
an amount of the compound of the present disclosure for (i) treating a specific disease, condition, or disorder; (ii) alleviating, ameliorating, or eliminating one or more symptoms of a specific disease, condition, or disorder; or (iii) delaying the onset of the one or more symptoms of the specific disease, condition, or disorder described herein.

The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the content of the present disclosure.

The term "treat" or "treatment" means administering the compound or formulation described herein to ameliorate or eliminate a disease or one or more symptoms associated with the disease, and includes:

(i) inhibiting a disease or a disease state, i.e., arresting its development; and
(ii) alleviating a disease or a disease state, i.e., causing its regression.

The term "prevent" or "prevention" means administering the compound or formulation described herein to prevent a disease or one or more symptoms associated with the disease, and includes: preventing the development of the disease or disease state in a mammal, particularly when such a mammal is predisposed to the disease state but has not yet been diagnosed with it.

In the present disclosure, examples of the term "mammal" include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acid addition or base addition salts, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The word "comprise" and variations thereof such as "comprises" or "comprising" may be interpreted in an open and non-exclusive sense, i.e., "including but not limited to".

The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritiation (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present disclosure include, but are not limited to, oral, rectal, local, inhalation, parenteral, sublingual, intravaginal, intranasal, intraocular, intraperitoneal, intramuscular, subcutaneous, and intravenous

administration.

The pharmaceutical composition of the present disclosure can be manufactured by methods well known in the art, such as conventional methods of mixing, dissolving, granulating, emulsifying, freeze-drying, and the like.

In some embodiments, the pharmaceutical composition is in an oral form. For oral administration, the pharmaceutical composition can be formulated by mixing the active compound with pharmaceutically acceptable excipients well known in the art. These excipients enable the compounds of the present disclosure to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compound with a solid excipient, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, flavoring agents, and the like.

The pharmaceutical composition may also be suitable for parenteral administration, such as a sterile solution, suspension, or lyophilized product in a suitable unit dosage form.

In all of the administration methods for the compound of general formula (I) described herein, the daily dose is from 0.01 mg/kg to 500 mg/kg of body weight, given in individual or separated doses. The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

## Examples

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure.

All reagents used in the present disclosure are commercially available and can be used without further purification.

Unless otherwise stated, the ratios expressed for mixed solvents are volume mixing ratios. The following eluents may be mixed eluents formed by two or more solvents in a certain volume ratio. For example, "ethyl acetate/petroleum ether = 5/1" represents that the volume ratio of ethyl acetate to petroleum ether in the mixed eluent is 5:1 during the gradient elution.

Unless otherwise stated, % refers to wt%.

Compounds are named either manually or by ChemDraw® software, and supplier's product names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS). The "$IC_{50}$" refers to the half inhibitory concentration, which is the concentration at which half of the maximal inhibitory effect is achieved.

Abbreviations:

TBAF: tetrabutylammonium fluoride; THF: tetrahydrofuran; TsOH: p-toluenesulfonic acid; BnBr: benzyl bromide; DMF: N,N-dimethylformamide; $Cy_2NH$: dicyclohexylamine; AcCl: acetyl chloride; EtOH: ethanol; EA: ethyl acetate; DMAc: N,N-dimethylacetamide; $LiOH \cdot H_2O$: lithium hydroxide monohydrate; BnOH: benzyl alcohol; DCC: N,N'-dicyclohexylcarbodiimide; DMAP: 4-dimethylaminopyridine; DCM: dichloromethane; ACN: acetonitrile; $RuCl_3$: ruthenium trichloride; MeOH: methanol; i-PrOH: isopropanol; t-BuOH: tert-butanol; $TMSCHN_2$: trimethylsilyldiazomethane; Toluene: toluene; TEA: triethylamine; $TMSCF_3$: (trifluoromethyl)trimethylsilane; CsF: cesium fluoride; $Pd_2(dba)_3$: tris(dibenzylideneacetone)dipalladium; dppf: 1,1'-bis(diphenylphosphino)ferrocene; DIEA: N,N-diisopropylethylamine; $SOCl_2$: thionyl chloride; MeMgBr: methylmagnesium bromide; m-CPBA: m-chloroperoxybenzoic acid; $NaBH_4$: sodium borohydride; EtONa: sodium ethoxide; Acetone: acetone; $NaIO_4$: sodium periodate; DMSO: dimethyl sulfoxide.

**Example 1: Synthesis of N-[6-cyano-5-(trifluoromethyl)pyridin-3-yl]-2,6-dihydroxy-2-methyl-hexanamide (compound 001)**

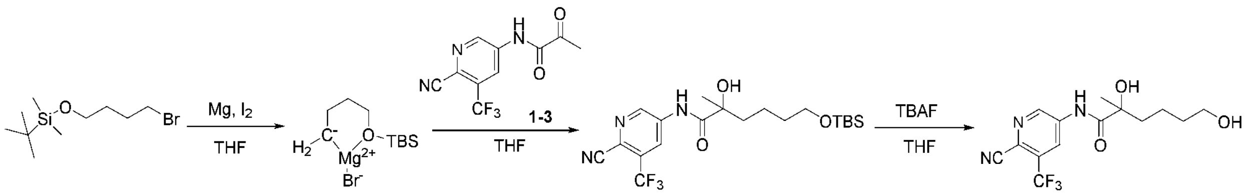


1-1 1-2 1-4 001

**Step 1: Synthesis of bromo-[4-[tert-butyl(dimethyl)silyl]oxybutyl]magnesium**

Magnesium (545 mg, 22.42 mmol) and 4-bromobutoxy-tert-butyl-dimethylsilane (5.0 g, 18.71 mmol) were dissolved in tetrahydrofuran (50 mL) in a nitrogen atmosphere, and iodine (1 piece) was added at 25 °C. After the addition was completed, the reaction mixture was stirred at 70 °C for 4 h, cooled to room temperature, and directly used in the next step.

**Step 2: Synthesis of 6-[tert-butyl(dimethyl)silyl]oxy-N-[6-cyano-5-(trifluoromethyl)pyridin-3-yl]-2-hydroxy-2-methyl-hexanamide**

In a nitrogen atmosphere at -78 °C, the reaction mixture (33.3 mL) from the previous step was slowly added dropwise to a solution of N-[6-cyano-5-(trifluoromethyl)pyridin-3-yl]-2-oxo-propionamide (compound **1-3**, 1.0 g, 3.89 mmol) in tetrahydrofuran (20 mL) within 15 min. After the dropwise addition was completed, the reaction mixture was stirred at -78 °C for 1 h. After LCMS indicated the completion of the reaction, a saturated aqueous ammonium chloride solution was added to the reaction mixture to quench the reaction, and the mixture was extracted with ethyl acetate (50 mL $\times$ 2). The organic phase was washed once with saturated brine, dried over anhydrous sodium sulfate, filtered, and concentrated. The crude product was purified by column chromatography (silica, ethyl acetate/petroleum ether = 1/1) to give compound **1-4** (550 mg).
MS (ESI): m/z = 446.20 $[M+H]^+$.

**Step 3: Synthesis of N-[6-cyano-5-(trifluoromethyl)pyridin-3-yl]-2,6-dihydroxy-2-methyl-hexanamide**

Tetrabutylammonium fluoride (1.11 mL, 1 M in THF) and intermediate compound **1-4** (450 mg, 1.01 mmol) were dissolved in tetrahydrofuran (15 mL), and the reaction mixture was stirred at 25 °C for 1 h and concentrated, and the crude product was purified by column chromatography (silica, ethyl acetate/petroleum ether = 1/1) to give target compound **001** (115 mg).
MS (ESI): m/z = 332.10 $[M+H]^+$.
$^1H$ NMR (400 MHz, DMSO-$d_6$)$\delta$10.70 (s, 1H), 9.40 (d, $J$ = 2.2 Hz, 1H), 8.92 (d, $J$ = 2.2 Hz, 1H), 5.85 (s, 1H), 4.33 (t, $J$ = 5.1 Hz, 1H), 3.36 (d, $J$ = 5.7 Hz, 2H), 1.81 - 1.69 (m, 1H), 1.67 - 1.53 (m, 1H), 1.53 - 1.43 (m, 1H), 1.43 - 1.32 (m, 5H), 1.28 - 1.14 (m, 1H).

**Example 2: Synthesis of N-[6-cyano-5-(trifluoromethyl)pyridin-3-yl]-2,4-dihydroxy-2-methylbutanamide (compound 002)**

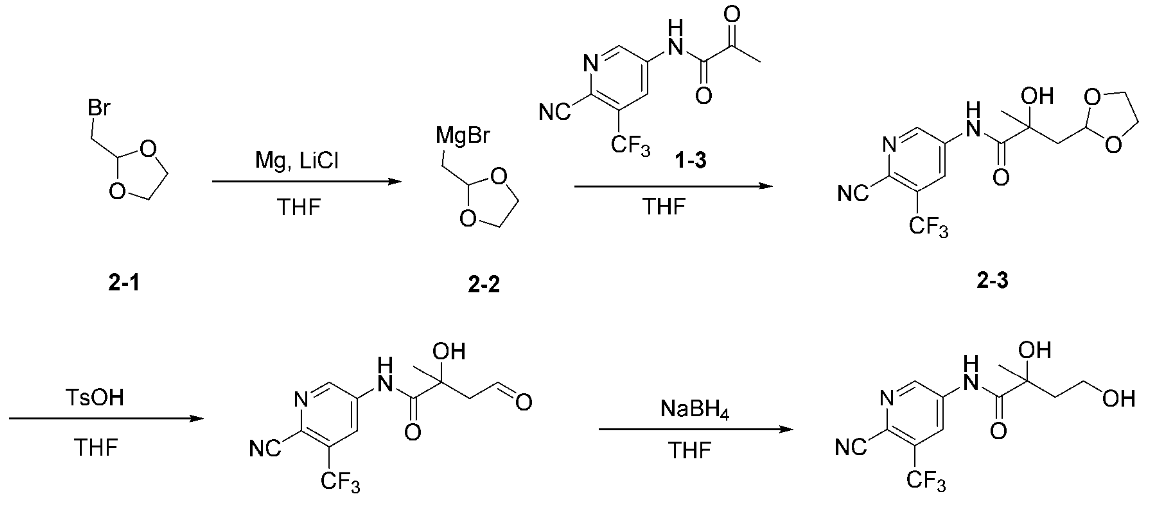


1-3

2-1 2-2 2-3

2-4 002

**Step 1: Synthesis of ((1,3-dioxolan-2-yl)methyl)magnesium bromide**

In a nitrogen atmosphere, 2-(bromomethyl)-1,3-dioxolane (20 g, 119.76 mmol), lithium chloride (10.15 g, 239.52 mmol), and magnesium powder (3.49 g, 143.71 mmol) were added to tetrahydrofuran (200 mL). The reaction mixture was stirred at 70 °C for 4 h and directly used in the next step.

**Step 2: Synthesis of N-[6-cyano-5-(trifluoromethyl)pyridin-3-yl]-3-(1,3-dioxolan-2-yl)-2-hydroxy-2-methylpropanamide**

In a nitrogen atmosphere at -78 °C, the reaction mixture (200 mL) from the previous step was slowly added dropwise to a solution of N-[6-cyano-5-(trifluoromethyl)pyridin-3-yl]-2-oxo-propionamide (compound **1-3**, 2.0 g, 7.78 mmol) in tetrahydrofuran (20 mL). After the dropwise addition was completed, the reaction mixture was heated to 70 °C and stirred for 18 h. After LCMS indicated the completion of the reaction, a saturated aqueous ammonium chloride solution was added to the reaction mixture to quench the reaction, and the mixture was extracted with ethyl acetate (50 mL × 2). The organic phase was washed once with saturated brine, dried over anhydrous sodium sulfate, filtered, and concentrated. The crude product was purified by column chromatography (silica, ethyl acetate/petroleum ether = 1/1) to give target compound **2-3** (220 mg).

MS (ESI): m/z = 346.10 $[M+H]^+$.

$^1H$ NMR (400 MHz, DMSO-$d_6$)δ 10.67 (s, 1H), 9.36 (d, *J* = 2.2 Hz, 1H), 8.87 (d, *J* = 2.2 Hz, 1H), 6.06 (s, 1H), 5.06 - 4.98 (m, 1H), 3.89 - 3.62 (m, 4H), 2.12 - 2.05 (m, 1H), 1.98 - 1.89 (m, 1H), 1.40 (s, 3H).

**Step 3: Synthesis of N-[6-cyano-5-(trifluoromethyl)pyridin-3-yl]-2-hydroxy-2-methyl-4-oxobutanamide**

Intermediate compound **2-3** (150 mg, 434.44 μmol) and p-toluenesulfonic acid (22.63 mg, 434.44 μmol) were added to tetrahydrofuran (10 mL). The reaction mixture was stirred at room temperature for 18 h. After LCMS indicated the completion of the reaction, water (60 mL) and ethyl acetate (80 mL) were added sequentially. The organic phase was washed with water (30 mL × 2), dried over anhydrous sodium sulfate, and filtered. The filtrate was concentrated to give compound **2-4** (120 mg).

MS(ESI): m/z = 302.1$[M+H]^+$.

**Step 4: Synthesis of N-[6-cyano-5-(trifluoromethyl)pyridin-3-yl]-2,4-dihydroxy-2-methylbutanamide**

Intermediate compound **2-4** (100 mg, 331.98 μmol) was added to tetrahydrofuran (1 mL). Sodium borohydride (18.84 mg, 497.97 μmol) was added in an ice-water bath. The reaction mixture was stirred in an ice-water bath for 18 h. 2 mL of water was added in an ice-water bath to quench the reaction. The reaction mixture was concentrated, and the residue was purified by Prep-HPLC [Phenomenex C18, 5 μm silica, 30 mm diameter, 80 mm length; a mixture of water (containing 7 mmol/L $NH_4HCO_3$) and acetonitrile with decreasing polarity was used as the eluent, acetonitrile gradient: 60%-70%, elution time: 9 min] to give target compound **002** (8.2 mg).

MS(ESI): m/z= 304.10 $[M+H]^+$.

$^1H$ NMR (400 MHz, DMSO-$d_6$)δ 9.36 (d, *J* = 2.2 Hz, 1H), 8.89 (d, *J* = 2.2 Hz, 1H), 6.05 (s, 1H), 3.62 - 3.43 (m, 2H), 2.05 - 1.93 (m, 1H), 1.85 - 1.73(m, 1H), 1.37 (s, 3H).

**Example 3: Synthesis of ethyl 5-((6-cyano-5-(trifluoromethyl)pyridin-3-yl)amino)-4-hydroxy-4-methyl-5-oxopentanoate (compound 003)**

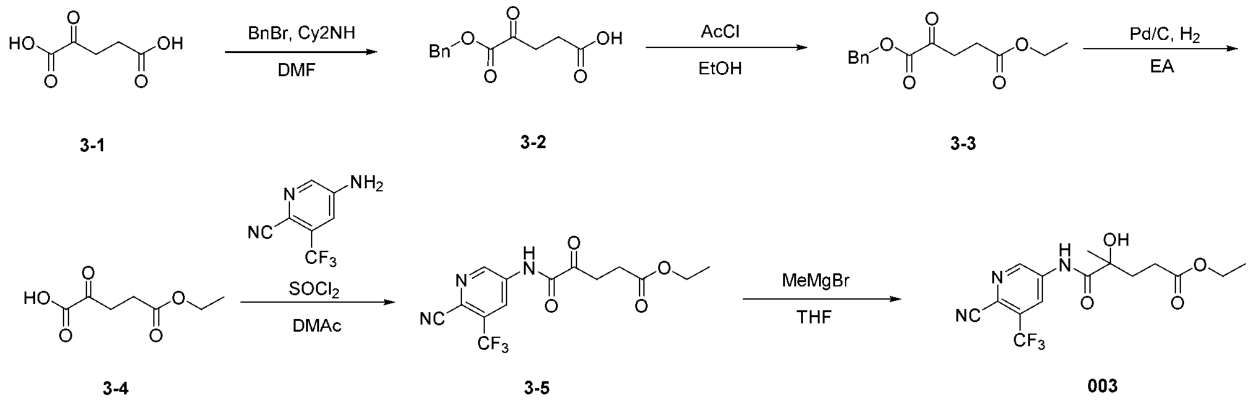


3-1 3-2 3-3 3-4 3-5 003

**Step 1: Synthesis of 5-(benzyloxy)-4,5-dioxopentanoic acid**

2-Ketoglutaric acid (2 g, 13.69 mmol) was dissolved in N,N-dimethylformamide (80 mL), and dicyclohexylamine (1.24 g, 6.84 mmol, 1.36 mL) and benzyl bromide (1.17 g, 6.84 mmol) were sequentially added. The mixture was heated to 50 °C and stirred overnight. After LC-MS indicated the completion of the reaction, the reaction mixture was poured into methyl tert-butyl ether (300 mL), and the mixture was washed three times with water (100 mL × 3). The organic phase was dried over anhydrous sodium sulfate and filtered, and the filtrate was concentrated. The residue was separated and purified by column chromatography (silica, ethyl acetate/petroleum ether = 1/2) to give compound **3-2** (1.1 g).
MS (ESI): m/z = 235.00 $[M-H]^-$.
$^1H$ NMR (400 MHz, DMSO-$d_6$)δ 12.27 (s, 1H), 7.48 - 7.32 (m, 5H), 5.26 (s, 2H), 3.09-3.01 (m, 2H), 2.52 - 2.46 (m, 2H).

**Step 2: Synthesis of ethyl 5-(benzyloxy)-4,5-dioxopentanoate**

Intermediate compound **3-2** (1 g, 4.23 mmol) was dissolved in absolute ethanol (10 mL), and acetyl chloride (365 mg, 4.66 mmol) was added in an ice-water bath. The mixture was stirred for 3 h with the temperature maintained. After LC-MS indicated the completion of the reaction, the reaction mixture was diluted with ethyl acetate (100 mL) and washed three times with a saturated aqueous sodium bicarbonate solution (50 mL × 3). The organic phase was dried over anhydrous sodium sulfate and filtered, and the filtrate was concentrated. The residue was separated and purified by column chromatography (silica, ethyl acetate/petroleum ether = 1/5) to give compound **3-3** (700 mg).
MS (ESI): m/z = 282.1 $[M+NH_4]^+$.
$^1H$ NMR (400 MHz, Chloroform-*d*)δ 7.45 - 7.25 (m, 5H), 5.30 (s, 2H), 4.20 - 4.05 (m, 2H), 3.20 - 3.12 (m, 2H), 2.70 - 2.62 (m, 2H), 1.29 - 1.21 (m, 3H).

**Step 3: Synthesis of 5-ethoxy-2,5-dioxopentanoic acid**

Intermediate compound **3-3** (500 mg, 1.89 mmol) was dissolved in ethyl acetate (10 mL), and Pd/C (46 mg) was added. The mixture was stirred for 15 min in a hydrogen atmosphere. After TLC (development solvent: dichloromethane/methanol = 10/1, Rf = 0.4) indicated the completion of the reaction, the reaction mixture was filtered, and the filtrate was concentrated to give compound **3-4** (300 mg).
$^1H$ NMR (400 MHz, Chloroform-*d*)δ 4.2 - 4.08 (m, 2H), 3.26 - 3.12 (m, 2H), 2.77 - 2.62 (m, 2H), 1.31 - 1.20 (m, 3H).

**Step 4: Synthesis of ethyl 5-((6-cyano-5-(trifluoromethyl)pyridin-3-yl)amino)-4,5-dioxopentanoate**

Intermediate compound **3-4** (300 mg, 2.05 mmol) was dissolved in N,N-dimethylacetamide (12 mL), and the solution was cooled to -10 °C. Thionyl chloride (244.22 mg, 2.05 mmol) was added, and the mixture was stirred for 10 min.

5-Amino-3-(trifluoromethyl)pyridine-2-carbonitrile (384 mg, 2.05 mmol) was added, and the mixture was stirred for another 3 h with the temperature maintained. After LC-MS indicated the completion of the reaction, the reaction mixture was poured into ice water (100 mL), and the mixture was extracted with ethyl acetate (30 mL × 3). The organic phases were combined, dried over anhydrous sodium sulfate, and filtered, and the filtrate was concentrated. The residue was separated and purified by column chromatography (silica, ethyl acetate/petroleum ether = 1/2) to give compound **3-5** (300 mg).

MS (ESI): m/z = 344.0 $[M+H]^+$.

$^1H$ NMR (400 MHz, DMSO-$d_6$) δ 10.73 (s, 1H), 9.40 (d, *J* = 2.2 Hz, 1H), 8.91 (d, *J* = 2.3 Hz, 1H), 4.11 - 4.02 (m, 2H), 3.32 - 3.16 (m, 2H), 2.68 - 2.60 (m, 2H), 1.25 - 1.15 (m, 3H).

**Step 5: Synthesis of ethyl 5-((6-cyano-5-(trifluoromethyl)pyridin-3-yl)amino)-4-hydroxy-4-methyl-5-oxopentanoate**

Intermediate compound **3-5** (100 mg, 291.32 μmol) was dissolved in tetrahydrofuran (10 mL), and the solution was cooled to -78 °C. Methylmagnesium bromide (0.5 M in THF, 1.40 mL) was added dropwise. The mixture was left to react for 2 h with the temperature maintained, and then slowly warmed to room temperature and stirred overnight. After LC-MS indicated the completion of the reaction, the reaction mixture was poured into ice water (40 mL), and the resulting mixture was extracted with ethyl acetate (15 mL × 3). The organic phases were combined, dried over anhydrous sodium sulfate, and filtered. The filtrate was concentrated, and the residue was purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 45%-75%, elution time: 9 min] to give target compound **003** (15 mg).

MS (ESI): m/z = 360.10 $[M+H]^+$.

$^1H$ NMR (400 MHz, DMSO-$d_6$) δ 9.37 (d, *J* = 2.3 Hz, 1H), 8.89 (d, *J* = 2.3 Hz, 1H), 6.01 (s, 1H), 4.06 - 3.95 (m, 2H), 2.47 - 2.39 (m, 1H), 2.29 - 2.18 (m, 1H), 2.10 - 1.97 (m, 1H), 1.94 - 1.81 (m, 1H), 1.37 (s, 3H), 1.18 - 1.10 (m, 3H).

**Example 4: Synthesis of 5-((6-cyano-5-(trifluoromethyl)pyridin-3-yl)amino)-4-hydroxy-4-methyl-5-oxopentanoic acid (compound 004)**

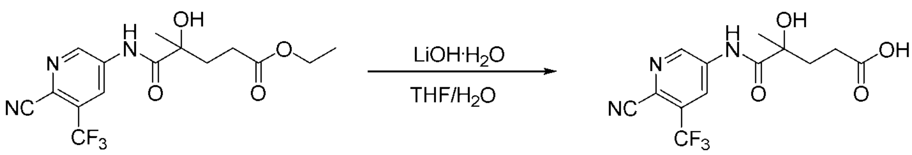


**003** **004**

Compound **003** (80 mg, 222.65 μmol) was dispersed in a mixed solution of tetrahydrofuran (3 mL) and water (1 mL), and lithium hydroxide monohydrate (8.55 mg, 203.76 μmol) was added. The reaction mixture was stirred at room temperature for 3 h. After LC-MS indicated the completion of the reaction, the reaction mixture was poured into a 0.5 M hydrochloric acid solution (15 mL), and the mixture was extracted three times with ethyl acetate (5 mL × 3). The organic phases were combined, dried over anhydrous sodium sulfate, and filtered, and the filtrate was concentrated. The residue was purified by RP-FLASH [column: 40 g C18 Column, 20-35 μm, 100 Å; mobile phase A: purified water, mobile phase B: acetonitrile; flow rate: 18 mL/min; gradient: 30% B to 50% B, elution time: 10 min] to give target compound **004** (15 mg).

MS (ESI): m/z = 332.0 $[M+H]^+$.

$^1H$ NMR (400 MHz, DMSO-$d_6$) δ 10.71 (s, 1H), 9.38 (d, *J* = 2.2 Hz, 1H), 8.92 - 8.88 (m, 1H), 2.43 - 2.32 (m, 1H), 2.22 - 2.12 (m, 1H), 2.06 - 1.93 (m, 1H), 1.91 - 1.80 (m, 1H), 1.37 (s, 3H).

**Examples 5 & 6: Synthesis of ethyl (6S)/(6R)-6-((6-cyano-5-(trifluoromethyl)pyridin-3-yl)amino)-5-hydroxy-5-methyl-6-oxohexanoate (compounds 005 & 006)**

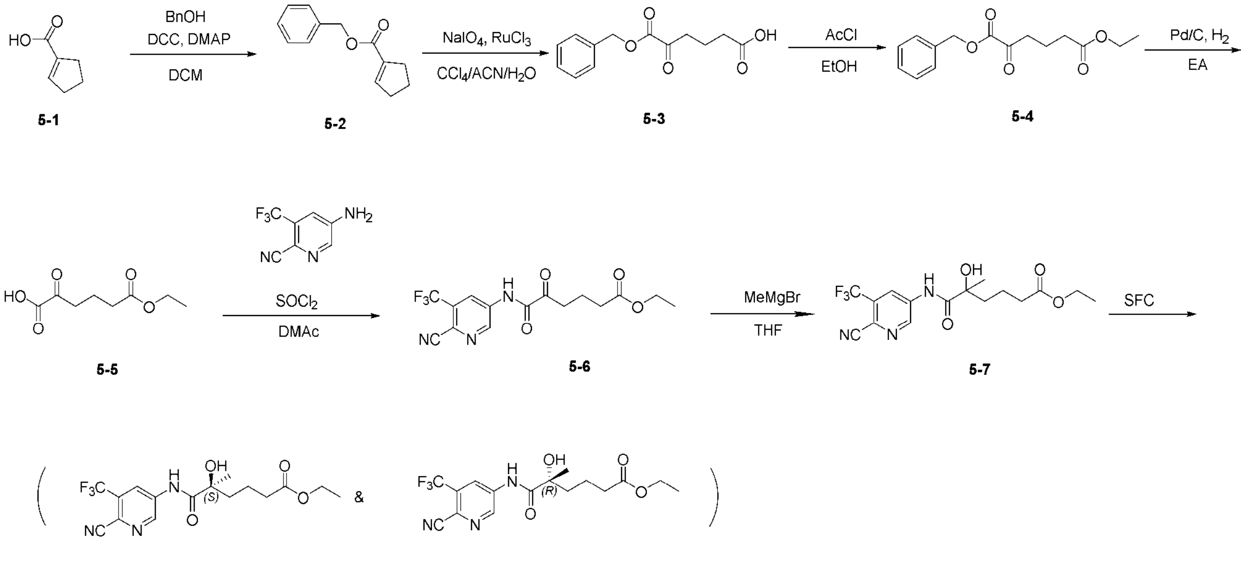


5-1 5-2 5-3 5-4

5-5 5-6 5-7

005/006

**Step 1: Synthesis of benzyl cyclopent-1-ene-1-carboxylate**

The starting material **5-1** (8 g, 71.35 mmol) was dissolved in dichloromethane (200 mL), and benzyl alcohol (9.26 g, 85.62 mmol), N,N'-dicyclohexylcarbodiimide (17.67 g, 85.62 mmol), and 4-dimethylaminopyridine (871.64 mg, 7.13 mmol) were added. The mixture was stirred at room temperature overnight. After TLC (development solvent: ethyl acetate/petroleum ether = 1/3, Rf = 0.6) indicated the completion of the reaction, the reaction mixture was concentrated, and the residue was purified by column chromatography (silica, ethyl acetate/petroleum ether = 0/1-1/4) to give title compound **5-2** (13 g).

$^1$H NMR (400 MHz, Chloroform-*d*) δ 7.42 - 7.27 (m, 5H), 6.87 - 6.79 (m, 1H), 5.19 (s, 2H), 2.65 - 2.55 (m, 2H), 2.55 - 2.45 (m, 2H), 2.02 - 1.90 (m, 2H).

**Step 2: Synthesis of 6-(benzyloxy)-5,6-dioxohexanoic acid**

Intermediate compound **5-2** (5 g, 24.72 mmol) was dissolved in a mixed solvent of carbon tetrachloride (120 mL) and acetonitrile (120 mL), and an aqueous solution (120 mL) of sodium periodate (10.58 g, 49.44 mmol) was added dropwise at 0 °C. After the dropwise addition was completed, solid ruthenium trichloride (512.79 mg, 2.47 mmol) was added, and the mixture was slowly warmed to room temperature and stirred for another 6 h. After LC-MS indicated the completion of the reaction, the reaction mixture was poured into water (300 mL), and the mixture was extracted with dichloromethane (150 mL × 3). The organic phases were combined, washed three times with water (100 mL × 3), dried over anhydrous sodium sulfate, and filtered, and the filtrate was concentrated. The residue was purified by column chromatography (silica, ethyl acetate/petroleum ether = 0/1-1/1) to give title compound **5-3** (2.5 g).

MS (ESI): m/z = 249.10 $[M-H]^-$.

$^1$H NMR (400 MHz, Chloroform-*d*) δ 7.44 - 7.31 (m, 5H), 5.28 (s, 2H), 2.99 - 2.88 (m, 2H), 2.47 - 2.38 (m, 2H), 2.07 - 1.90 (m, 2H).

**Step 3: Synthesis of 1-benzyl-6-ethyl 2-oxoadipate**

Intermediate compound **5-3** (1 g, 4.00 mmol) was dissolved in absolute ethanol (10 mL), and acetyl chloride (378.83 mg, 4.82 mmol) was dropwise added in an ice-water bath. The mixture was left to react for 5 h with the temperature maintained. After LC-MS indicated the completion of the reaction, the reaction mixture was poured into dichloromethane (30 mL), washed twice with water (10 mL × 2), dried over anhydrous sodium sulfate, and filtered, and the filtrate was concentrated. The residue was separated and purified by column chromatography (silica, ethyl acetate/petroleum ether = 0/1-1/1) to give title compound **5-4** (800 mg).

MS (ESI): m/z = 279.10 $[M+H]^+$.

$^1$H NMR (400 MHz, Chloroform-*d*) δ 7.44 - 7.31 (m, 5H), 5.28 (s, 2H), 4.17 - 4.07 (m, 2H), 2.97 - 2.89 (m, 2H), 2.42 - 2.32 (m, 2H), 2.03 - 1.90 (m, 2H), 1.41 - 1.20 (m, 3H).

**Step 4: Synthesis of 6-ethoxy-2,6-dioxohexanoic acid**

Intermediate compound **5-4** (350 mg, 1.26 mmol) was dissolved in ethyl acetate (4 mL), and Pd/C (30 mg) was added. The mixture was stirred for 15 min in a hydrogen atmosphere. After TLC (development solvent: methanol/dichloromethane = 1/10, Rf = 0.3) indicated the depletion of the starting materials, the mixture was filtered, and the filtrate was concentrated to give title compound **5-5** (220 mg).

$^1$H NMR (400 MHz, Chloroform-*d*)δ 4.19 - 4.09 (m, 2H), 3.07 - 2.99 (m, 2H), 2.44 - 2.35 (m, 2H), 2.07 - 1.94 (m, 2H), 1.30 - 1.22 (m, 3H).

**Step 5: Synthesis of ethyl 6-((6-cyano-5-(trifluoromethyl)pyridin-3-yl)amino)-5,6-dioxohexanoate**

Intermediate compound **5-5** (360 mg, 1.91 mmol) was dissolved in N,N-dimethylacetamide (7.97 mL). The solution was cooled to -10 °C, and thionyl chloride (273.12 mg, 2.30 mmol) was added dropwise. After the dropwise addition was completed, solid 5-amino-3-(trifluoromethyl)pyridine-2-carbonitrile (357.98 mg, 1.91 mmol) was added. The mixture was stirred for 2 h with the temperature maintained. After LC-MS indicated the completion of the reaction, the reaction mixture was diluted with ethyl acetate (50 mL), washed three times with water (20 mL × 3), dried over anhydrous sodium sulfate, and filtered, and the filtrate was concentrated. The residue was separated and purified by column chromatography (silica, ethyl acetate/petroleum ether = 0/1-1/1) to give title compound **5-6** (450 mg).

MS (ESI): m/z =358.10 $[M+H]^+$.

$^1$H NMR (400 MHz, DMSO-$d_6$)δ 11.43 (s, 1H), 9.39 (d, *J* = 2.3 Hz, 1H), 8.85 (d, *J* = 2.3 Hz, 1H), 4.11 - 3.98 (m, 2H), 3.02 - 2.94 (m, 2H), 2.42 - 2.33 (m, 2H), 1.88 - 1.76 (m, 2H), 1.21 - 1.15 (m, 3H).

**Step 6: Synthesis of ethyl 6-((6-cyano-5-(trifluoromethyl)pyridin-3-yl)amino)-5-hydroxy-5-methyl-6-oxohexanoate**

Intermediate **5-6** was prepared on a greater scale according to the method described in steps 1-5. Intermediate compound **5-6** (17 g, 47.58 mmol) was dissolved in tetrahydrofuran (200 mL), and the solution was cooled to -78 °C in a nitrogen atmosphere. A solution of methylmagnesium bromide in tetrahydrofuran (3 M, 47.58 mL) was added dropwise, and the mixture was stirred overnight with the temperature maintained. After LC-MS indicated the completion of the reaction, at -78 °C, a saturated aqueous ammonium chloride solution (350 mL) was slowly added to quench the reaction, and the mixture was extracted with ethyl acetate (100 mL × 3). The organic phases were combined, dried over anhydrous sodium sulfate, and filtered. The filtrate was concentrated, and the residue was separated and purified by column chromatography (silica, ethyl acetate/petroleum ether = 0/1-1/1) to give title compound **5-7** (11 g).

MS (ESI): m/z = 374.10 $[M+H]^+$.

$^1$H NMR (400 MHz, DMSO-$d_6$)δ 10.72 (s, 1H), 9.40 (d, *J* = 2.2 Hz, 1H), 8.91 (d, *J* = 2.3 Hz, 1H), 5.92 (s, 1H), 4.07 - 3.97 (m, 2H), 2.31 - 2.22 (m, 2H), 1.81 - 1.65 (m, 3H), 1.50 - 1.38 (m, 1H), 1.36 (s, 3H), 1.20 - 1.11 (m, 3H).

**Step 7: Synthesis of ethyl (6S)/(6R)-6-((6-cyano-5-(trifluoromethyl)pyridin-3-yl)amino)-5-hydroxy-5-methyl-6-oxohexanoate**

Compound **5-7** (1 g) was separated by SFC [column: Chiral Cel OJ-H 150 × 4.6 mm I.D., 5 μm; mobile phase: A: carbon dioxide; B: methanol (0.05% diethanolamine), gradient: mobile phase B: 5% to 40%, elution time: 4.5 min; mobile phase B: 5%, elution for 1.5 min; flow rate: 2.5 mL/min; column temperature: 40 °C] to give compound **005** (310 mg) and compound **006** (330 mg).

Compound **005**:

SFC retention time: 2.247 min.

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 24.980 min.

MS (ESI): m/z = 374.10 $[M+H]^+$.

$^1$H NMR (400 MHz, DMSO-$d_6$)δ 10.72 (s, 1H), 9.40 (d, *J* = 2.2 Hz, 1H), 8.91 (d, *J* = 2.3 Hz, 1H), 5.92 (s, 1H), 4.07 - 3.97 (m, 2H), 2.31 - 2.22 (m, 2H), 1.81 - 1.65 (m, 3H), 1.50 - 1.38 (m, 1H), 1.36 (s, 3H), 1.20 - 1.11 (m, 3H).

Compound **006**:

SFC retention time: 3.064 min.

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 16.224 min.

MS (ESI): m/z = 374.10 $[M+H]^+$.

$^1$H NMR (400 MHz, DMSO-$d_6$)δ 10.72 (s, 1H), 9.40 (d, *J* = 2.2 Hz, 1H), 8.91 (d, *J* = 2.3 Hz, 1H), 5.92 (s, 1H), 4.07 - 3.97 (m, 2H), 2.31 - 2.22 (m, 2H), 1.81 - 1.65 (m, 3H), 1.50 - 1.38 (m, 1H), 1.36 (s, 3H), 1.20 - 1.11 (m, 3H).

**Examples 7 & 8: Synthesis of methyl (6S)/(6R)-6-((6-cyano-5-(trifluoromethyl)pyridin-3-yl)amino)-5-hydroxy-5-methyl-6-oxohexanoate (compounds 007 & 008)**

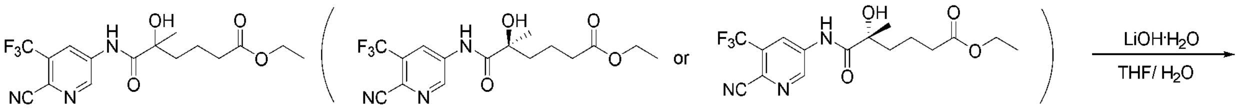


**005/006**

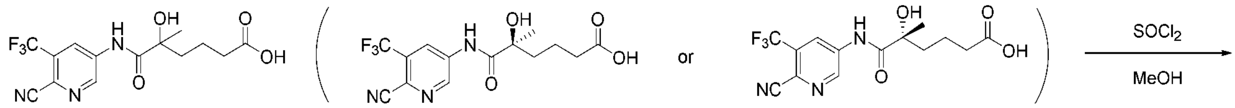


**7-1/8-1**

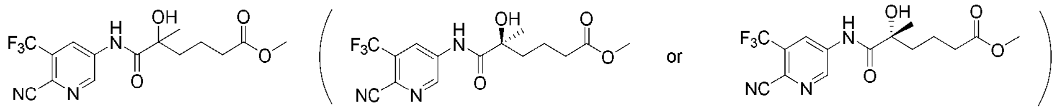

**007/008**

**Step 1: Synthesis of (6S)/(6R)-6-((6-cyano-5-(trifluoromethyl)pyridin-3-yl)amino)-5-hydroxy-5-methyl-6-oxohexanoic acid**

Compound **005** (or **006**) (270 mg, 723.23 μmol) was dispersed in a mixed solvent of tetrahydrofuran (3 mL) and water (1 mL), and lithium hydroxide monohydrate (34.66 mg, 826.02 μmol) was added. The reaction mixture was stirred at room temperature overnight. After LC-MS indicated the completion of the reaction, the reaction mixture was poured into a 0.1 M hydrochloric acid solution (15 mL), and the mixture was extracted with ethyl acetate (5 mL × 3). The organic phases were combined, dried over anhydrous sodium sulfate, and filtered, and the filtrate was concentrated to give compound 7-1 (or 8-1) (240 mg).

**Compound 7-1:**

MS (ESI): m/z = 346.10 $[M+H]^+$.

$^1$H NMR (400 MHz, DMSO-$d_6$)δ 11.97 (s, 1H), 10.70 (s, 1H), 9.40 (d, *J* = 2.2 Hz, 1H), 8.91 (d, *J* = 2.3 Hz, 1H), 5.90 (s, 1H), 2.19 (t, *J= 7.1* Hz, 2H), 1.81 - 1.54 (m, 3H), 1.48 - 1.37 (m, 1H), 1.37 (s, 3H).

**Compound 8-1:**

MS (ESI): m/z = 346.10 $[M+H]^+$.

$^1$H NMR (400 MHz, DMSO-$d_6$)δ 11.97 (s, 1H), 10.69 (s, 1H), 9.39 (d, *J* = 2.2 Hz, 1H), 8.91 (d, *J*= 2.2 Hz, 1H), 5.90 (s, 1H), 2.19 (t, *J= 7.1* Hz, 2H), 1.82 - 1.54 (m, 3H), 1.48 - 1.38 (m, 1H), 1.36 (s, 3H).

Step 2: **Synthesis of methyl (6S)/(6R)-6-((6-cyano-5-(trifluoromethyl)pyridin-3-yl)amino)-5-hydroxy-5-methyl-6-oxohexanoate**

Intermediate compound 7-1 (or 8-1) (50 mg, 144.81 μmol) was dissolved in methanol (4 mL), and thionyl chloride (51.69 mg, 434.44 μmol) was added dropwise at room temperature. The mixture was heated to 60 °C and stirred for 2 h.

After LC-MS indicated the completion of the reaction, the mixture was subjected to rotary evaporation at reduced pressure to remove the solvent, and the residue was purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-70%, elution time: 9.5 min] to give target compound 007 (12 mg) (or 008 (15 mg)).

**Compound 007:**

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 23.394 min.

MS (ESI): m/z = 360.10 $[M+H]^+$.

$^1H$ NMR (400 MHz, DMSO-$d_6$)δ 10.71 (s, 1H), 9.40 (d, *J* = 2.2 Hz, 1H), 8.91 (d, *J* = 2.2 Hz, 1H), 5.91 (s, 1H), 3.56 (s, 3H), 2.33 - 2.25 (m, 2H), 1.83 -1.55 (m, 3H), 1.51 - 1.39 (m, 1H), 1.36 (s, 3H).

**Compound 008:**

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 15.201 min.

MS (ESI): m/z = 360.10 $[M+H]^+$.

$^1H$ NMR (400 MHz, DMSO-$d_6$)δ 10.73 (s, 1H), 9.40 (d, *J* = 2.2 Hz, 1H), 8.91 (d, *J* = 2.3 Hz, 1H), 5.94 (s, 1H), 3.56 (s, 3H), 2.32- 2.24 (m, 2H), 1.81- 1.53 (m, 3H), 1.50 - 1.38 (m, 1H), 1.36 (s, 3H).

**Examples 9 & 10: Synthesis of isopropyl (6S)/(6R)-6-((6-cyano-5-(trifluoromethyl)pyridin-3-yl)amino)-5-hydroxy-5-methyl-6-oxohexanoate (compounds 009 & 010)**

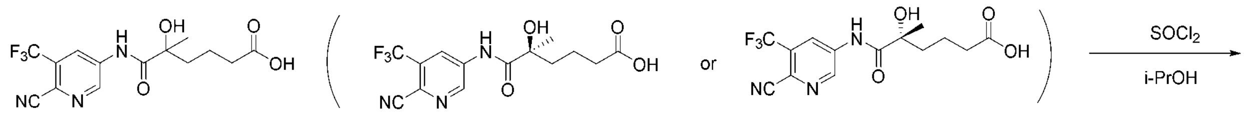


**7-1/8-1**

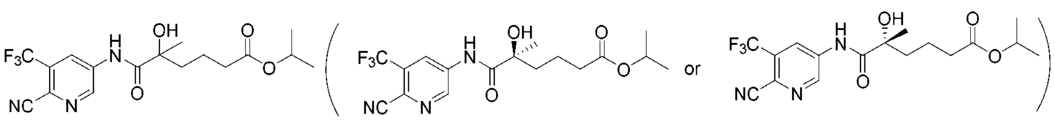


**009/010**

Intermediate compound **7-1** (or **8-1**) (50 mg, 144.81 μmol) was dissolved in isopropanol (4 mL), and thionyl chloride (52 mg, 434.44 μmol) was added dropwise at room temperature. The mixture was heated to 60 °C and stirred for 2 h. After LC-MS indicated the completion of the reaction, the mixture was subjected to rotary evaporation at reduced pressure to remove the solvent, and the residue was purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 45%-75%, elution time: 11 min] to give target compound **009** (10 mg) (or **010** (12 mg)).

Compound **009**:

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 26.011 min.

MS (ESI): m/z =388.10$[M+H]^+$.

$^1H$ NMR (400 MHz, DMSO-$d_6$)δ 10.71(s, 1H), 9.39 (d, *J* = 2.2 Hz, 1H), 8.91 (d, *J* = 2.3 Hz, 1H), 5.91 (s, 1H), 4.93 - 4.79 (m, 1H), 2.28 - 2.17 (m, 2H), 1.83 - 1.53 (m, 3H), 1.50 - 1.38 (m, 1H), 1.36 (s, 3H), 1.18 - 1.12 (m, 6H).

Compound **010**:

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 16.601 min.

MS (ESI): m/z =388.10[M+H]$^+$.

$^1$H NMR (400 MHz, DMSO-$d_6$)δ 10.71 (s, 1H), 9.39 (d, $J$ = 2.2 Hz, 1H), 8.91 (d, $J$ = 2.3 Hz, 1H), 5.91 (s, 1H), 4.93 - 4.80 (m, 1H), 2.28 - 2.17 (m, 2H), 1.81 - 1.54 (m, 3H), 1.49 - 1.39 (m, 1H), 1.36 (s, 3H), 1.18 - 1.11 (m, 6H).

**Examples 11 & 12: Synthesis of tert-butyl (6S)/(6R)-6-((6-cyano-5-(trifluoromethyl)pyridin-3-yl)amino)-5-hydroxy-5-methyl-6-oxohexanoate (compounds 011 & 012)**

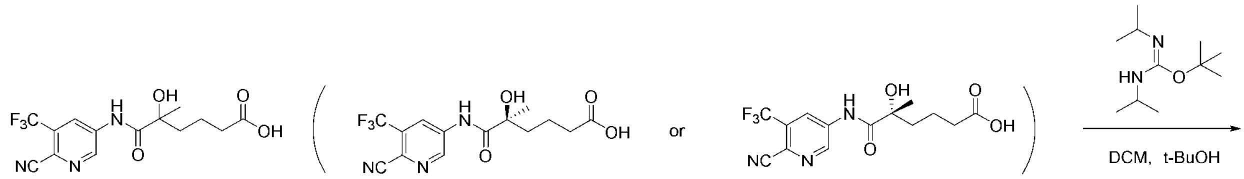


**7-1/8-1**

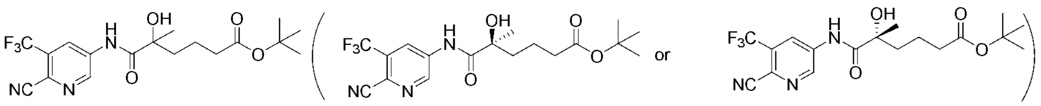

**011/012**

Intermediate compound **7-1 (or 8-1)** (300.00 mg, 868.88 μmol) and O-tert-butyl-N,N'-diisopropylisourea (1.74 g, 8.69 mmol) were added to dichloromethane (7.5 mL) and tert-butanol (644.01 mg, 8.69 mmol), and the mixture was stirred at room temperature for 15 h. After LCMS indicated the completion of the reaction, the reaction mixture was filtered, and the filtrate was concentrated. The residue was purified by Prep-HPLC [Phenomenex C18, 5 μm silica, 30 mm diameter, 80 mm length; a mixture of purified water and acetonitrile (acetonitrile content: 60%-70%) was used as the eluent, elution time: 8 min] to give target compound **011** (150 mg) (or **012** (110 mg)). Compound **011**:
Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 32.009 min.

MS (ESI): m/z=400.1 [M-H]$^-$.

$^1$H NMR (400 MHz, DMSO-$d_6$)δ 10.70 (s, 1H), 9.39 (s, 1H), 9.01 - 8.88 (m, 1H), 5.90 (s, 1H), 2.22 - 2.13 (m, 2H), 1.82 - 1.48 (m, 4H), 1.44 - 1.32 (m, 12H).

Compound **012**:

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 17.147 min.

MS (ESI): m/z = 400.1 [M-H]$^-$.

$^1$H NMR (400 MHz, DMSO-$d_6$)δ 10.70 (s, 1H), 9.39 (s, 1H), 9.00 - 8.84 (m, 1H), 5.89 (s, 1H), 2.22 - 2.11 (m, 2H), 1.83 - 1.49 (m, 4H), 1.46 - 1.32 (m, 12H).

**Examples 13 & 14: Synthesis of ethyl (6S)/(6R)-6-((6-cyano-5-(methylthio)pyridin-3-yl)amino)-5-hydroxy-5-methyl-6-oxohexanoate (compounds 013 & 014)**

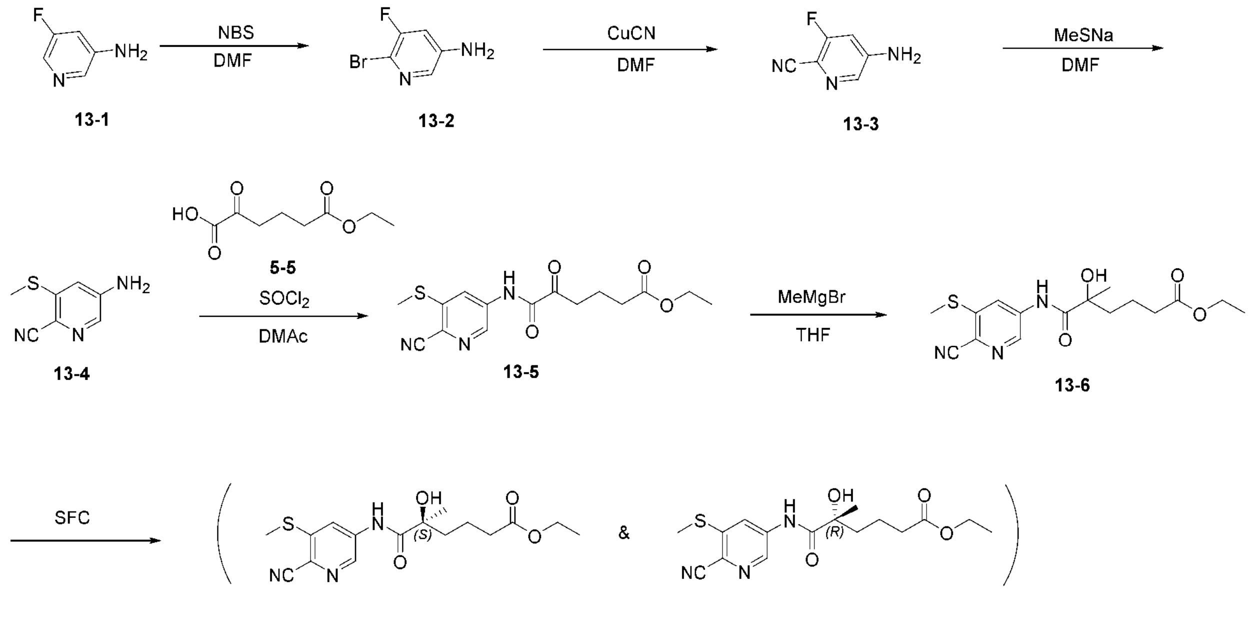


13-1 13-2 13-3

5-5

13-4 13-5 13-6

&

013/014

**Step 1: Synthesis of 6-bromo-5-fluoropyridin-3-amine**

5-Fluoropyridin-3-amine (13.0 g, 116 mmol) was dissolved in N,N-dimethylformamide (60 mL), and then the mixture was cooled in an ice bath. A solution of N-bromosuccinimide (20.64 g, 116 mmol) in N,N-dimethylformamide (50 mL) was slowly and dropwise added to the mixture in the ice bath, and then the mixture was allowed to react for 30 min in the ice bath. After LC-MS monitoring indicated the completion of the reaction, the reaction mixture was poured into 500 mL of water, and the resulting mixture was extracted with ethyl acetate (200 mL × 3). The organic phases were combined, washed with water (300 mL × 2) and saturated brine (300 mL × 2) in sequence, dried over anhydrous sodium sulfate, and filtered. The filtrate was concentrated at reduced pressure to give a brown-yellow crude product, which was purified by column chromatography (silica, petroleum ether/ethyl acetate = 10/1-1/1) to give intermediate **13-2** (8.4 g).
MS (ESI): m/z = 190.90, 192.90 $[M+H]^+$.

**Step 2: Synthesis of 5-amino-3-fluoropyridine-2-carbonitrile**

Intermediate **13-2** (6 g, 31.41 mmol) and cuprous cyanide (11.25 g, 125.64 mmol) were dispersed in N,N-dimethylformamide (100 mL). A nitrogen balloon was provided, and the mixture was purged with nitrogen three times and then transferred into a 130 °C oil bath in a nitrogen atmosphere for 6 h of reaction. After LC-MS indicated the completion of the reaction, the reaction mixture was cooled to room temperature and filtered. The filtrate was concentrated at reduced pressure, and the residue was purified by column chromatography (silica, petroleum ether/ethyl acetate = 1/2) to give title compound **13-3** (2.6 g).
MS (ESI): m/z = 138.0 $[M+H]^+$.

**Step 3: Synthesis of 5-amino-3-(methylthio)pyridine-2-carbonitrile**

Compound **13-3** (2 g, 14.59 mmol) was added to a reaction flask and dissolved in N,N-dimethylformamide (30 mL), and sodium thiomethoxide (2.04 g, 29.11 mmol) was added with stirring at room temperature. The resulting reaction mixture was stirred at room temperature for 12 h. The reaction mixture was poured into water (200 mL), and the resulting mixture was extracted with ethyl acetate (80 mL × 5). The organic phases were combined, washed with water (200 mL × 2) and saturated brine (400 mL) in sequence, dried over anhydrous sodium sulfate, and filtered. The filtrate was concentrated at reduced pressure to give title compound **13-4** (1.7 g).
MS (ESI): m/z = 166.0 $[M+H]^+$.
$^1H$ NMR (400 MHz, DMSO-$d_6$)δ 7.75 (d, $J$ = 2.3 Hz, 1H), 6.84 (d, $J$ = 2.3 Hz, 1H), 6.47 (s, 2H), 2.50 (s, 3 H).

**Step 4: Synthesis of ethyl 6-(6-cyano-5-(methylthio)pyridin-3-yl)amino)-5,6-dioxohexanoate**

Intermediate compound **5-5** (1.37 g, 7.28 mmol) was dissolved in N,N-dimethylacetamide (15 mL). The solution

was cooled to -10 °C, and thionyl chloride (1.04 g, 8.72 mmol) was added dropwise. After the dropwise addition was completed, solid compound **13-4** (1.2 g, 7.26 mmol) was added, and the mixture was stirred for 12 h. LC-MS indicated that the reaction was feasible. The mixture was diluted with ethyl acetate (30 mL), washed three times with water (20 mL × 3), dried over anhydrous sodium sulfate, and filtered, and the filtrate was concentrated. The residue was separated and purified by column chromatography (silica, ethyl acetate:petroleum ether = 0/1-1/1) to give compound **13-5** (1.6 g).

MS (ESI): m/z = 336.0 $[M+H]^+$.

$^1$H NMR (400 MHz, Chloroform-*d*) δ 8.96 (s, 1H), 8.41 (s, 2H), 4.19 - 4.07 (m, 2H), 3.13 - 3.04 (m, 2H), 2.60 (s, 3H), 2.46 - 2.38 (m, 2H), 2.09 - 1.96 (m, 2H), 1.31 - 1.22 (m, 3H).

**Step 5: Synthesis of ethyl 6-((6-cyano-5-(methylthio)pyridin-3-yl)amino)-5-hydroxy-5-methyl-6-oxohexanoate**

Intermediate compound **13-5** (1 g, 2.98 mmol) was dissolved in tetrahydrofuran (20 mL), and the solution was cooled to -78 °C. Methylmagnesium bromide (3 M in $Et_2O$, 2.98 mL) was added dropwise. The mixture was slowly warmed to room temperature and stirred overnight. LC-MS indicated that the reaction was feasible. At 0 °C, a saturated aqueous ammonium chloride solution (50 mL) was added to quench the reaction. The mixture was warmed to room temperature and extracted with ethyl acetate (20 mL × 3). The organic phases were combined, dried over anhydrous sodium sulfate, and filtered, and the filtrate was concentrated. The residue was purified by column chromatography (silica, ethyl acetate:petroleum ether = 1/4) to give compound **13-6** (600 mg).

MS (ESI): m/z = 352.0 $[M+H]^+$.

$^1$H NMR (400 MHz, DMSO-$d_6$) δ 10.32 (s, 1H), 8.95 (d, *J* = 2.1 Hz, 1H), 8.41 (d, *J* = 2.1 Hz, 1H), 5.86 (s, 1H), 4.08 - 3.97 (m, 2H), 2.58 (s, 3H), 2.26 (t, *J* = 7.1 Hz, 2H), 1.80 - 1.66 (m, 2H), 1.64 - 1.53 (m, 1H), 1.44 (d, *J* = 7.6 Hz, 1H), 1.35 (s, 3H), 1.20 - 1.11 (m, 3H).

**Step 6: Synthesis of ethyl (6S)/(6R)-6-((6-cyano-5-(methylthio)pyridin-3-yl)amino)-5-hydroxy-5-methyl-6-oxo-hexanoate**

Compound **13-6** (1 g) was separated by SFC [column: Chiral Cel OJ-H 150 × 4.6 mm I.D., 5 μm; mobile phase: A: carbon dioxide; B: ethanol (0.05% diethanolamine), gradient: mobile phase B: 5% to 40%, elution time: 4.5 min; mobile phase B: 5%, elution for 1.5 min; flow rate: 2.5 mL/min; column temperature: 40 °C] to give compound **013** (350 mg) and compound **014** (370 mg).

Compound **013**:

SFC retention time: 3.468 min.

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 21.615 min.

MS (ESI): m/z =352.00 $[M+H]^+$.

$^1$H NMR (400 MHz, DMSO-$d_6$) δ 10.32 (s, 1H), 8.95 (d, *J* = 2.1 Hz, 1H), 8.41 (d, *J* = 2.1 Hz, 1H), 5.86 (s, 1H), 4.08 - 3.97 (m, 2H), 2.58 (s, 3H), 2.31 - 2.22 (m, 2H), 1.80 - 1.66 (m, 2H), 1.64 - 1.53 (m, 1H), 1.47 - 1.40 (m, 1H), 1.35 (s, 3H), 1.20 - 1.11 (m, 3H).

Compound **014**:

SFC retention time: 4.292 min.

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 15.077 min.

MS (ESI): m/z =352.00 $[M+H]^+$.

$^1$H NMR (400 MHz, DMSO-$d_6$) δ 10.32 (s, 1H), 8.95 (d, *J* = 2.1 Hz, 1H), 8.41 (d, *J* = 2.1 Hz, 1H), 5.86 (s, 1H), 4.08 - 3.97 (m, 2H), 2.58 (s, 3H), 2.31 - 2.22 (m, 2H), 1.80 - 1.66 (m, 2H), 1.64 - 1.53 (m, 1H), 1.47 - 1.40 (m, 1H), 1.35 (s, 3H), 1.20 - 1.11 (m, 3H).

**Examples 15 & 16: Synthesis of methyl (6S)/(6R)-6-((6-cyano-5-(methylthio)pyridin-3-yl)amino)-5-hydroxy-5-methyl-6-oxohexanoate (compounds 015 & 016)**

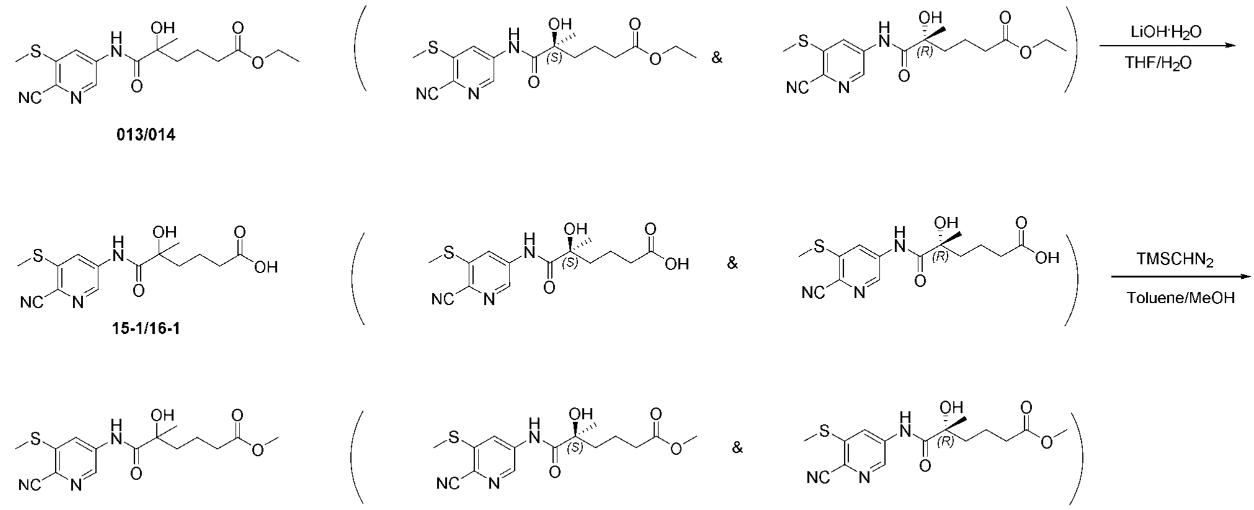


013/014

15-1/16-1

015/016

**Step 1: Synthesis of (6S)/(6R)-6-((6-cyano-5-(methylthio)pyridin-3-yl)amino)-5-hydroxy-5-methyl-6-oxohexanoic acid**

Compound **013** (or compound **014**) (100 mg, 284.56 μmol) was dispersed in a mixed solvent of tetrahydrofuran (3 mL) and water (1 mL), and lithium hydroxide monohydrate (35.82 mg, 853.68 μmol) was added. The reaction mixture was stirred at room temperature for 3 h. After LC-MS indicated the completion of the reaction, the reaction mixture was poured into a 0.1 M hydrochloric acid solution (15 mL), and the mixture was extracted with ethyl acetate (5 mL × 3). The organic phases were combined, dried over anhydrous sodium sulfate, and filtered, and the filtrate was concentrated to give compound **15-1** (90 mg) (or **16-1**).

**Compound 15-1:**

MS (ESI): m/z = 324.10 $[M+H]^+$.

$^1$H NMR (400 MHz, DMSO-$d_6$) δ 11.98 (s, 1H), 10.31 (s, 1H), 8.95 (d, *J* = 2.1 Hz, 1H), 8.41 (d, *J* = 2.1 Hz, 1H), 5.84 (s, 1H), 2.58 (s, 3H), 2.19 (t, *J* = 7.1 Hz, 2H), 1.81 - 1.52 (m, 3H), 1.46 - 1.37 (m, 1H), 1.35 (s, 3H).

Compound 16-1:

MS (ESI): m/z = 324.10 $[M+H]^+$.

$^1$H NMR (400 MHz, DMSO-$d_6$) δ 11.98 (s, 1H), 10.31 (s, 1H), 8.95 (d, *J*= 2.1 Hz, 1H), 8.41 (d, *J*= 2.1 Hz, 1H), 5.84 (s, 1H), 2.58 (s, 3H), 2.19 (t, *J= 7.1* Hz, 2H), 1.81 - 1.52 (m, 2H), 1.47 - 1.38 (m, 1H), 1.35 (s, 3H).

Step 2: Synthesis of methyl (6S)/(6R)-6-((6-cyano-5-(methylthio)pyridin-3-yl)amino)-5-hydroxy-5-methyl-6-oxohexanoate

Compound 15-1 (or 16-1) (90 mg, 278.32 μmol) was dissolved in a mixed solvent of toluene (4 mL) and methanol (1 mL). The solution was cooled to 0 °C, and trimethylsilyldiazomethane (2 M in n-hexane, 278.32 μL) was added dropwise. The mixture was stirred for 2 h with the temperature maintained. After LC-MS indicated the completion of the reaction, two drops of acetic acid were added to quench the reaction. The reaction mixture was diluted with ethyl acetate (20 mL) and washed three times with water (10 mL × 3). The organic phase was dried over anhydrous sodium sulfate, filtered, and concentrated. The residue was purified by column chromatography (silica, ethyl acetate:petroleum ether = 1/1) to give compound 015 (65 mg) (or 016 (65 mg)).

Compound 015:

Chiral-HPLC retention time: 19.582 min.

MS (ESI): m/z =338.0 $[M+H]^+$.

$^1$H NMR (400 MHz, DMSO-$d_6$) δ 10.32 (s, 1H), 8.95 (d, *J*=2.1 Hz, 1H), 8.41 (d, *J* = 2.1 Hz, 1H), 5.86 (s, 1H), 3.56 (s, 3H), 2.58 (s, 3H), 2.33 - 2.22 (m, 2H), 1.81 - 1.64 (m, 2H), 1.64 - 1.52 (m, 1H), 1.48 - 1.38 (m, 1H), 1.35 (s, 3H).

Compound 016:

Chiral-HPLC retention time: 13.855 min.

MS (ESI): m/z =338.0 [M+H]$^+$.

$^1$H NMR (400 MHz, DMSO-$d_6$)δ 10.31 (s, 1H), 8.95 (d, *J* = 2.1 Hz, 1H), 8.41 (d, *J* = 2.1 Hz, 1H), 5.86 (s, 1H), 3.56 (s, 3H), 2.58 (s, 3H), 2.33 - 2.24 (m, 2H), 1.81 - 1.63 (m, 2H), 1.63 - 1.53 (m, 1H), 1.52 - 1.38 (m, 1H), 1.35 (s, 3H).

**Examples 17 & 18: Synthesis of isopropyl (6S)/(6R)-6-((6-cyano-5-(methylthio)pyridin-3-yl)amino)-5-hydroxy-5-methyl-6-oxohexanoate (compounds 017 & 018)**

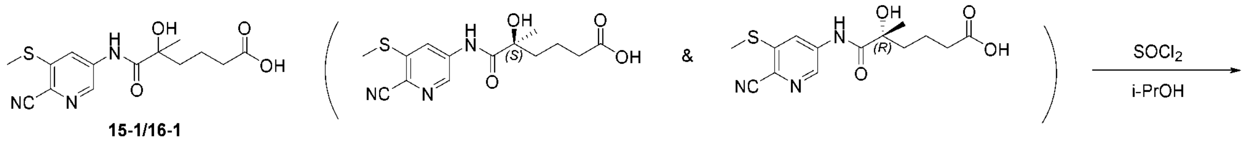


**15-1/16-1**

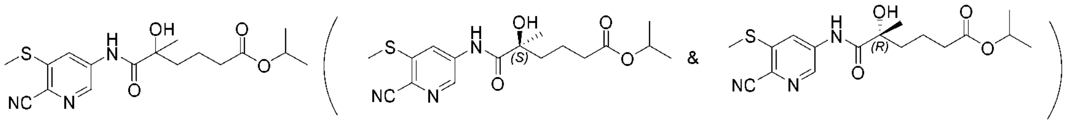

**017/018**

Intermediate compound **15-1** (or **16-1**) (140 mg, 432.94 μmol) was added to isopropanol (4 mL), and thionyl chloride (51.51 mg, 432.94 μmol) was added dropwise. The mixture was heated to 60 °C and stirred for 4 h. After LC-MS indicated the completion of the reaction, the reaction mixture was cooled to room temperature, diluted with ethyl acetate (10 mL × 3), and washed three times with water (10 mL × 3). The organic phase was dried over anhydrous sodium sulfate and filtered. The filtrate was concentrated, and the residue was purified by column chromatography (silica, petroleum ether/ethyl acetate = 1/1) to give compound **017** (130 mg) (or **018** (125 mg)).

Compound **017**:

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 22.544 min.

MS (ESI): m/z =366.10 [M+H]$^+$.

$^1$H NMR (400 MHz, DMSO-$d_6$)δ 10.32 (s, 1H), 8.95 (d, *J* = 2.1 Hz, 1H), 8.41 (d, *J* = 2.1 Hz, 1H), 5.85 (s, 1H), 4.91 - 4.80 (m, 1H), 2.58 (s, 3H), 2.27 - 2.18 (m, 2H), 1.81 - 1.63 (m, 2H), 1.63 - 1.52 (m, 1H), 1.50 - 1.37 (m, 1H), 1.35 (s, 3H), 1.19 - 1.12 (m, 6H).

Compound **18**:

Chiral-HPLC [column: AD-RH (Daicel 150 × 4.6 mm, 5 μm); mobile phase: A: water; B: acetonitrile; gradient: mobile phase B: 10% to 90%; elution for 35 min; flow rate: 1.0 mL/min; column temperature: 25 °C] retention time: 15.406 min.

MS (ESI): m/z =366.10 [M+H]$^+$.

$^1$H NMR (400 MHz, DMSO-$d_6$)δ 10.32 (s, 1H), 8.95 (d, *J* = 2.0 Hz, 1H), 8.41 (d, *J* = 2.1 Hz, 1H), 5.87 (s, 1H), 4.92 - 4.79 (m, 1H), 2.58 (s, 3H), 2.27 - 2.19 (m, 2H), 1.81 - 1.66 (m, 2H), 1.64 - 1.53 (m, 1H), 1.49 - 1.40 (m, 1H), 1.35 (s, 3H), 1.19 - 1.12 (m, 6H).

**Example 19: Synthesis of tert-butyl 6-((6-cyano-5-(methylthio)pyridin-3-yl)amino)-5-hydroxy-5-methyl-6-oxohexanoate (compound 019)**

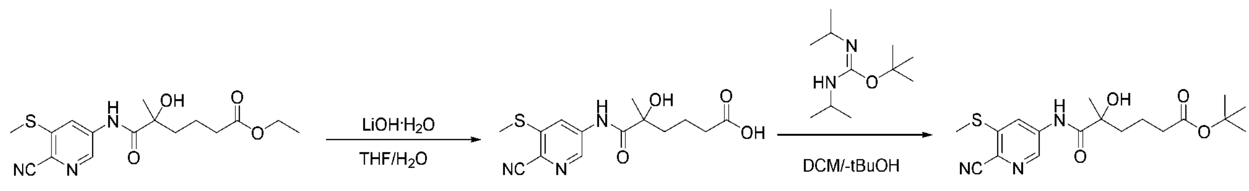


13-6 19-1 019

**Step 1: Synthesis of 6-((6-cyano-5-(trifluoromethyl)pyridin-3-yl)amino)-5-hydroxy-5-methyl-6-oxohexanoic acid**

Intermediate compound **13-6** (300 mg, 853.68 μmol) was dispersed in a mixed solvent of tetrahydrofuran (4 mL) and water (1 mL), and lithium hydroxide monohydrate (38.51 mg, 917.78 μmol) was added. The reaction mixture was stirred at room temperature overnight. After LC-MS indicated the completion of the reaction, the reaction mixture was poured into a 0.1 M hydrochloric acid solution (15 mL), and the mixture was extracted with ethyl acetate (5 mL × 3). The organic phases were combined, dried over anhydrous sodium sulfate, and filtered, and the filtrate was concentrated to give title compound **19-1** (253 mg).

MS (ESI): m/z = 324.10 $[M+H]^+$.

$^1$H NMR (400 MHz, DMSO-$d_6$)δ 10.34 (s, 1H), 8.96 (d, *J* = 2.1 Hz, 1H), 8.41 (d, *J* = 2.1 Hz, 1H), 2.58 (s, 3H), 2.21 - 2.12 (m, 2H), 1.81 - 1.53 (m, 3H), 1.46 - 1.37 (m, 1H), 1.34 (s, 3H).

**Step 2: Synthesis of tert-butyl 6-((6-cyano-5-(methylthio)pyridin-3-yl)amino)-5-hydroxy-5-methyl-6-oxohexanoate**

Intermediate compound **19-1** (100 mg, 309.25 μmol) and O-tert-butyl-N,N'-diisopropylisourea (619.48 mg, 3.09 mmol) were dissolved in a mixed solvent of dichloromethane (5 mL) and tert-butanol (0.5 mL), and the reaction mixture was stirred at room temperature overnight. After LC-MS indicated the completion of the reaction, the reaction mixture was concentrated, and the residue was purified by column chromatography (silica, ethyl acetate/petroleum ether = 1/9-4/5) to give compound **019** (35 mg).

MS (ESI): m/z = 380.10 $[M+H]^+$.

$^1$H NMR (400 MHz, DMSO-$d_6$)δ 10.32 (s, 1H), 8.96 (d, *J* = 2.1 Hz, 1H), 8.41 (d, *J* = 2.1 Hz, 1H), 5.85 (s, 1H), 2.58 (s, 3H), 2.21 - 2.12 (m, 2H), 1.80 - 1.69 (m, 1H), 1.69 - 1.61 (m, 1H), 1.61 - 1.51 (m, 1H), 1.43 - 1.32 (m, 13H)

**Example 20: Synthesis of ethyl 6-[(6-cyano-5-(methoxy)pyridin-3-yl)amino]-5-hydroxy-5-methyl-6-oxohexanoate (compound 020)**

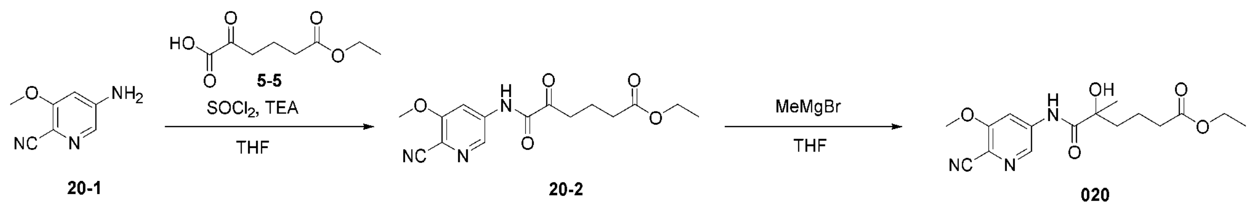


20-1 20-2 020

**Step 1: Synthesis of ethyl 6-[(6-cyano-5-(methoxy)pyridin-3-yl)amino]-5,6-dioxohexanoate**

Intermediate compound **20-1** (946.25 mg, 6.34 mmol) was added to tetrahydrofuran (20 mL) and precooled at 0 °C for 15 min. Thionyl chloride (1.20 g, 10.06 mmol) was added, and the mixture was stirred at 0 °C for 2 h. Then triethylamine (1.36 g, 13.41 mmol) was added, and the mixture was stirred for 10 min in an ice bath. Intermediate compound **5-5** (500 mg, 2.65 mmol) was added, and the mixture was warmed to room temperature and stirred for 12 h. After LCMS indicated the completion of the reaction, the reaction mixture was diluted with water (50 mL) and extracted with ethyl acetate (50 mL × 3). The organic phases were combined and concentrated, and the residue was purified by column chromatography (silica, petroleum ether/ethyl acetate = 1/1) to give title compound **20-2** (940 mg).

MS (ESI): m/z = 320.1 $[M+H]^+$.

$^1$H NMR (400 MHz, DMSO-$d_6$)δ 11.07 (s, 1H), 8.76 (d, *J* = 1.9 Hz, 1H), 8.24 (d, *J* = 1.9 Hz, 1H), 4.08 - 4.01 (m, 2H), 3.94 (s, 3H), 2.97 (t, *J* = 7.2 Hz, 2H), 2.37 (t, *J* = 7.4 Hz, 2H), 1.86 - 1.76 (m, 2H), 1.19 - 1.17 (m, 3H).

**Step 2: Synthesis of ethyl 6-[(6-cyano-5-(methoxy)pyridin-3-yl)amino]-5-hydroxy-5-methyl-6-oxo-hexanoate**

Intermediate compound **20-2** (200 mg, 626.35 μmol) was added to tetrahydrofuran (8 mL), and methylmagnesium bromide (3 M in $Et_2O$, 627 μL) was added at -50 °C. The mixture was stirred at - 40 °C for 12 h. After LCMS indicated the completion of the reaction, the reaction was quenched with a saturated ammonium chloride solution. The reaction mixture was diluted with water (20 mL) and extracted with ethyl acetate (20 mL × 3). The organic phases were combined and concentrated, and the residue was purified by column chromatography (silica, petroleum ether/ethyl acetate = 3/1) to give target compound **020** (90 mg).
MS (ESI): m/z = 336.1 $[M+H]^+$.
$^1H$ NMR (400 MHz, DMSO-$d_6$)δ 10.29 (s, 1H), 8.75 (d, *J* = 1.9 Hz, 1H), 8.29 (d, *J* = 1.9 Hz, 1H), 5.85 (s, 1H), 4.07 - 3.99 (m, 2H), 3.93 (s, 3H), 2.33 - 2.21 (m, 2H), 1.82 - 1.63 (m, 2H), 1.63 - 1.40 (m, 2H), 1.35 (s, 3H), 1.20 - 1.10 (m, 3H).

**Example 21: Synthesis of ethyl 5-[[6-cyano-5-(trifluoromethyl)pyridin-3-yl]carbamoyl]-6,6,6-trifluoro-5-hydroxyhexanoate (compound 021)**

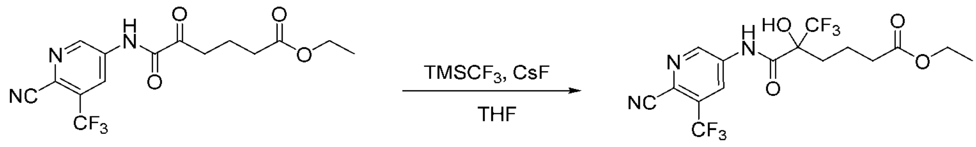


5-6 021

Intermediate compound **5-6** (50 mg, 139.94 μmol) and (trifluoromethyl)trimethylsilane (59 mg, 419.83 μmol) were added to tetrahydrofuran (0.5 mL), and cesium fluoride (5 mg, 32.91 μmol) was added in a nitrogen atmosphere. The reaction mixture was stirred at 25 °C for 3 h. After LCMS indicated the completion of the reaction, the reaction mixture was diluted with water (0.5 mL) and filtered. The filtrate was concentrated, and the resulting residue was purified by Prep-HPLC [Phenomenex $C_{18}$, 5 μm silica, 30 mm diameter, 80 mm length; a mixture of water (containing 7 mmol/L $NH_4HCO_3$) and acetonitrile (acetonitrile content: 60%-70%) was used as the eluent, elution time: 8 min] to give target compound **021** (5 mg).
MS (ESI): m/z = 428.00 $[M+H]^+$.
$^1H$ NMR (400 MHz, Chloroform-*d*)δ 9.45 (s, 1H), 9.00 - 8.93 (m, 1H), 8.83 - 8.75 (m, 1H), 6.16 (s, 1H), 4.36 - 4.08 (m, 2H), 2.59 - 2.38 (m, 3H), 2.01 - 1.89 (m, 1H), 1.71 - 1.58 (m, 2H), 1.39 - 1.24 (m, 3H).

**Example 22: Synthesis of $N^1$-(6-cyano-5-(trifluoromethyl)pyridin-3-yl)-2-hydroxy-$N^6$,2-dimethyladipamide (compound 022)**

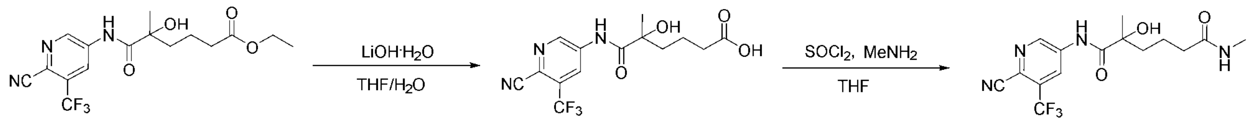


5-7 22-1 022

**Step 1: Synthesis of 6-((6-cyano-5-(trifluoromethyl)pyridin-3-yl)amino)-5-hydroxy-5-methyl-6-oxohexanoic acid**

Compound **5-7** (100 mg, 267.86 μmol) was dispersed in a mixed solvent of tetrahydrofuran (3 mL) and water (1 mL), and lithium hydroxide monohydrate (12.84 mg, 306.01 μmol) was added. The reaction mixture was stirred at room temperature overnight. After LC-MS indicated the completion of the reaction, the reaction mixture was poured into a 0.1 M aqueous hydrogen chloride solution (15 mL), and the mixture was extracted with ethyl acetate (5 mL × 3). The organic phases were combined, dried over anhydrous sodium sulfate, and filtered, and the filtrate was concentrated to give compound **22-1** (80 mg).
MS (ESI): m/z = 346.10 $[M+H]^+$.
$^1H$ NMR (400 MHz, DMSO-$d_6$)δ 9.40 (d, *J* = 2.2 Hz, 1H), 8.91 (d, *J* = 2.3 Hz, 1H), 2.22 - 2.13 (m, 2H), 1.81 - 1.54 (m, 3H), 1.48 - 1.36 (m, 1H), 1.36 (s, 3H).

**Step 2: Synthesis of N[1]-(6-cyano-5-(trifluoromethyl)pyridin-3-yl)-2-hydroxy-N[6],2-dimethyladipamide**

Intermediate compound **22-1** (50 mg, 144.81 μmol) was dissolved in tetrahydrofuran (4 mL). The solution was cooled to 0 °C, and thionyl chloride (18 mg, 151.31 μmol) was added dropwise. The mixture was left to react at room temperature for 6 h. A methylamine solution (1 M in THF, 1.5 mL) was added to the reaction mixture, and the mixture was stirred at room temperature for 3 h. After LC-MS indicated the completion of the reaction, the reaction mixture was poured into an icy 1 M hydrochloric acid solution (30 mL), and the mixture was extracted with ethyl acetate (10 mL × 3). The organic phases were combined, dried over anhydrous sodium sulfate, and filtered, and the filtrate was concentrated. The residue was separated and purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 30%-60%, elution time: 7 min] to give target compound **022** (15 mg).

MS (ESI): m/z= 359.10 $[M+H]^+$.

$^1$H NMR (400 MHz, DMSO-$d_6$)δ 9.39 (s, 1H), 8.91 (d, *J* = 2.3 Hz, 1H), 7.69 (s, 1H), 5.90 (s, 1H), 2.55-2.51 (m, 3H), 2.10-2.01 (m, 2H), 1.74- 1.50 (m, 3H), 1.42-1.32 (m, 4H).

**Example 23: Synthesis of N[1]-(6-cyano-5-(trifluoromethyl)pyridin-3-yl)-2-hydroxy-2-methyladipamide (compound 023)**

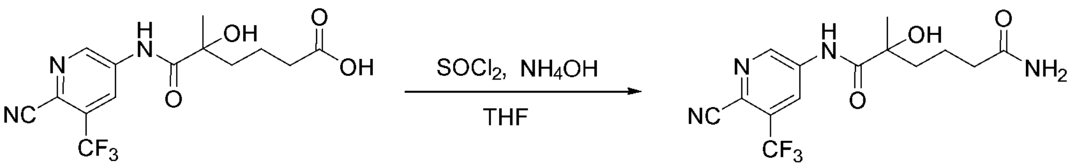


**22-1** **023**

Compound **22-1** (50 mg, 144.81 μmol) was dissolved in tetrahydrofuran (4 mL), and the resulting solution was cooled to 0 °C. Thionyl chloride (18 mg, 151.31 μmol) was added dropwise, and the mixture was left to react at room temperature for 6 h. Ammonia water (2 mL, content: 35%) was added to the reaction mixture, and the resulting mixture was stirred at room temperature for 3 h. After LC-MS indicated the completion of the reaction, the reaction mixture was poured into an icy 1 M hydrochloric acid solution (30 mL), and the mixture was extracted with ethyl acetate (10 mL × 3). The organic phases were combined, dried over anhydrous sodium sulfate, and filtered, and the filtrate was concentrated. The residue was separated and purified by Prep-HPLC [YMC-TAR column, 5 μm silica, 30 mm diameter, 150 mm length; a mixture of water (containing 7 mmol/L $NH_4HCO_3$) and acetonitrile with decreasing polarity was used as the eluent, acetonitrile gradient: 40%-70%, elution time: 12 min] to give target compound **023** (15 mg).

MS (ESI): m/z = 345.10$[M+H]^+$.

$^1$H NMR (400 MHz, DMSO-$d_6$)δ 10.70 (s, 1H), 9.40 (d, *J* = 2.3 Hz, 1H), 8.91 (d, *J* = 2.3 Hz, 1H), 7.23 (s, 1H), 6.69 (s, 1H), 5.92 (s, 1H), 2.06-1.97 (m, 2H), 1.79-1.52 (m, 3H), 1.43-1.33 (m, 4H).

**Example 24: Ethyl 6-[(6-cyano-5-methylselanylpyridin-3-yl)amino]-5-hydroxy-5-methyl-6-oxohexanoate (compound 024)**

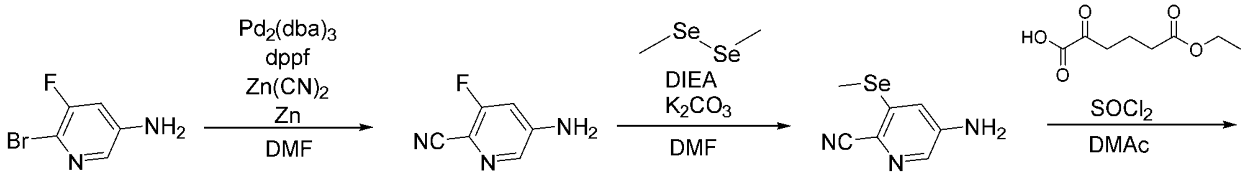


24-1 24-2 24-3

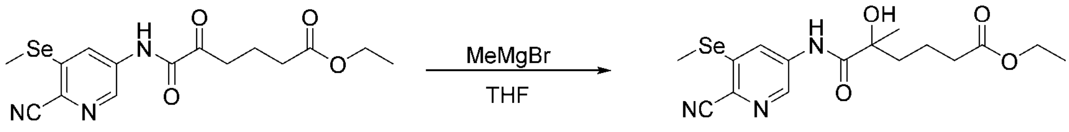


24-4 024

**Step 1: Synthesis of 5-amino-3-fluoropyridine-2-carbonitrile**

6-Bromo-5-fluoropyridin-3-amine (5 g, 26.18 mmol), zinc cyanide (6.15 g, 52.36 mmol), zinc powder (166.47 mg, 2.54 mmol), tris(dibenzylideneacetone)dipalladium (1.2 g, 1.31 mmol), and 1,1'-bis(diphenylphosphino)ferrocene (1.45 g, 2.62 mmol) were added to N,N-dimethylformamide (50 mL), and the reaction mixture was left to react in an argon atmosphere at 100 °C for 16 h. After LCMS indicated the completion of the reaction, the reaction mixture was poured into water (500 mL), and ethyl acetate (200 mL × 4) was added for extraction. The organic phases were combined, washed with saturated brine (400 mL × 2), dried over anhydrous sodium sulfate, and filtered, and the filtrate was concentrated. The crude product was purified by column chromatography (silica, petroleum ether/ethyl acetate = 10/1-1/1) to give title compound **24-2** (2.9 g).

MS (ESI): m/z = 138.10 $[M+H]^+$.

**Step 2: Synthesis of 5-amino-3-(methylselanyl)pyridine-2-carbonitrile**

5-Amino-3-fluoropyridine-2-carbonitrile (1.6 g, 11.67 mmol), dimethyl diselenide (6.68 g, 35.53 mmol), N,N-diisopropylethylamine (4.52 g, 35.01 mmol), and potassium carbonate (4.83 g, 35.01 mmol) were added to N,N-dimethylformamide (20 mL), and the reaction mixture was stirred at 100 °C for 18 h. After LCMS indicated the completion of the reaction, the reaction mixture was poured into water, and ethyl acetate (200 mL × 4) was added for extraction. The organic phases were combined, washed with saturated brine (500 mL), dried over anhydrous sodium sulfate, and filtered, and the filtrate was concentrated. The crude product was purified by column chromatography (silica, petroleum ether/ethyl acetate = 10/1-1/1) to give title compound **24-3** (2 g).

MS (ESI): m/z = 214.10 $[M+H]^+$.

$^1$H NMR (400 MHz, DMSO-$d_6$)δ 7.79 (d, *J* = 2.3 Hz, 1H), 6.95 (d, *J* = 2.4 Hz, 1H), 6.49 (s, 2H), 2.39 (s, 3H).

**Step 3: Synthesis of ethyl 6-[(6-cyano-5-methylselanylpyridin-3-yl)amino]-5,6-dioxohexanoate**

6-Ethoxy-2,6-dioxohexanoic acid (166.34 mg, 883.94 μmol) and 5-amino-3-(methylselanyl)pyridine-2-carbonitrile (100 mg, 471.45 μmol) were dissolved in N,N-dimethylacetamide (3 mL), and thionyl chloride (112.18 mg, 942.91 μmol) was added dropwise at - 10 °C. The reaction mixture was stirred at -10 °C for 18 h. After LCMS indicated the completion of the reaction, the reaction mixture was poured into water (50 mL), and ethyl acetate (30 mL × 4) was added for extraction. The organic phases were combined, washed with saturated brine (60 mL), dried over anhydrous sodium sulfate, and filtered, and the filtrate was concentrated to give title compound **24-4** (220 mg).

MS (ESI): m/z = 384.10 $[M+H]^+$.

**Step 4: Synthesis of ethyl 6-[(6-cyano-5-methylselanylpyridin-3-yl)amino]-5-hydroxy-5-methyl-6-oxohexanoate**

Intermediate **24-4** (200 mg, 523.18 μmol) was added to tetrahydrofuran (5 mL), and a solution of methylmagnesium bromide in tetrahydrofuran (3 M, 524 μL) was added dropwise at -78 °C. The reaction mixture was stirred at -78 °C for 18 h. After LCMS indicated the completion of the reaction, the reaction mixture was diluted with ethyl acetate (10 mL), and a saturated ammonium chloride solution (10 mL) was added at a low temperature to quench the reaction. The resulting

mixture was extracted with ethyl acetate (20 mL × 4). The organic phases were combined, washed with saturated brine, dried over anhydrous sodium sulfate, and filtered, and the filtrate was concentrated. The crude product was separated and purified by prep-HPLC [Phenomenex C18, 5 μm silica, 30 mm diameter, 80 mm length; a mixture of water (containing 0.05% ammonium bicarbonate) and acetonitrile (acetonitrile content: 38%-68%) was used as the eluent] to give target compound **024** (60 mg). MS (ESI): m/z = 400.10 $[M+H]^+$.

$^1H$ NMR (400 MHz, Chloroform-*d*) δ 9.15 (s, 1H), 8.59 (d, *J* = 2.3 Hz, 1H), 8.40 (d, *J* = 2.3 Hz, 1H), 4.18- 4.12 (m, 2H), 3.55 (s, 1H), 2.48 (s, 3H), 2.42 - 2.35 (m, 2H), 2.10 - 2.01 (m, 1H), 1.82 - 1.62 (m, 3H), 1.51 (s, 3H), 1.26 (t, *J* = 7.2 Hz, 3H).

**Example 25: Ethyl (5R)-6-[(6-cyano-5-methylsulfonylpyridin-3-yl)amino]-5-hydroxy-5-methyl-6-oxo-hexanoate or ethyl (5S)-6-[(6-cyano-5-methylsulfonylpyridin-3-yl)amino]-5-hydroxy-5-methyl-6-oxo-hexanoate (compound 025)**

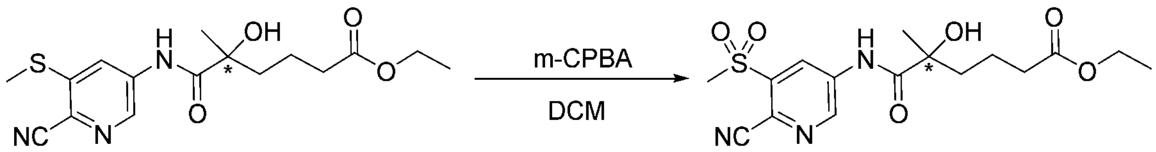


**014** **025**

Compound **014** (90.00 mg, 256.10 μmol) was added to dichloromethane (1 mL), and m-chloroperoxybenzoic acid (155.99 mg, 768.31 μmol, content: 85%) was added in an ice bath. The reaction mixture was stirred at room temperature for 4 h and filtered, and the filtrate was washed with water (1 mL × 2). The organic phase was dried over anhydrous sodium sulfate and filtered, and the filtrate was concentrated. The residue was purified by column chromatography (silica, petroleum ether/ethyl acetate = 2/1) to give compound **025** (59 mg).

MS (ESI): m/z = 384.2 $[M+H]^+$.

$^1H$ NMR (400 MHz, DMSO-$d_6$) δ 10.74 (s, 1H), 9.39 - 9.30 (m, 1H), 9.13 - 9.05 (m, 1H), 5.89 (s, 1H), 4.10 - 3.95 (m, 2H), 3.44 (s, 3H), 2.31 - 2.23 (m, 2H), 1.81 - 1.66 (m, 2H), 1.64 - 1.54 (m, 1H), 1.50 - 1.39 (m, 1H), 1.36 (s, 3H), 1.20 - 1.11 (m, 3H).

**Example 26: Ethyl (5R)-6-[(6-cyano-5-methylsulfinylpyridin-3-yl)amino]-5-hydroxy-5-methyl-6-oxo-hexanoate or ethyl (5S)-6-[(6-cyano-5-methylsulfinylpyridin-3-yl)amino]-5-hydroxy-5-methyl-6-oxo-hexanoate (compound 026)**

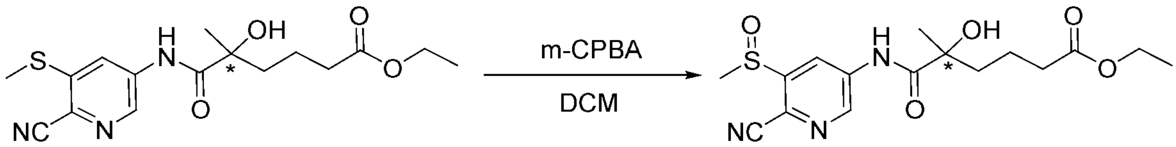


**014** **026**

Compound **014** (50 mg, 142.28 μmol) was added to dichloromethane (0.5 mL), and m-chloroperoxybenzoic acid (23.11 mg, 113.82 μmol, content: 85%) was added in an ice bath. The reaction mixture was stirred at room temperature for 4 h and filtered, and the filtrate was washed with water (1 mL × 2). The organic phase was dried over anhydrous sodium sulfate and filtered, and the filtrate was concentrated and purified by column chromatography (silica, petroleum ether/ethyl acetate = 2/1) to give compound **026** (5 mg).

MS (ESI): m/z = 368.1 $[M+H]^+$.

$^1H$ NMR (400 MHz, DMSO-$d_6$) δ 10.64 (s, 1H), 9.21 - 9.11 (m, 1H), 9.03 - 8.90 (m, 1H), 5.85 (s, 1H), 4.14 - 3.91 (m, 2H), 2.91 (s, 3H), 2.32 - 2.23 (m, 2H), 1.84 - 1.65 (m, 2H), 1.65 - 1.54 (m, 1H), 1.50 - 1.41 (m, 1H), 1.36 (s, 3H), 1.20 - 1.11 (m, 3H).

**Example 27: Ethyl 7-((6-cyano-5-(trifluoromethyl)pyridin-3-yl)amino)-6-hydroxy-6-methyl-7-oxoheptanoate (compound 027)**

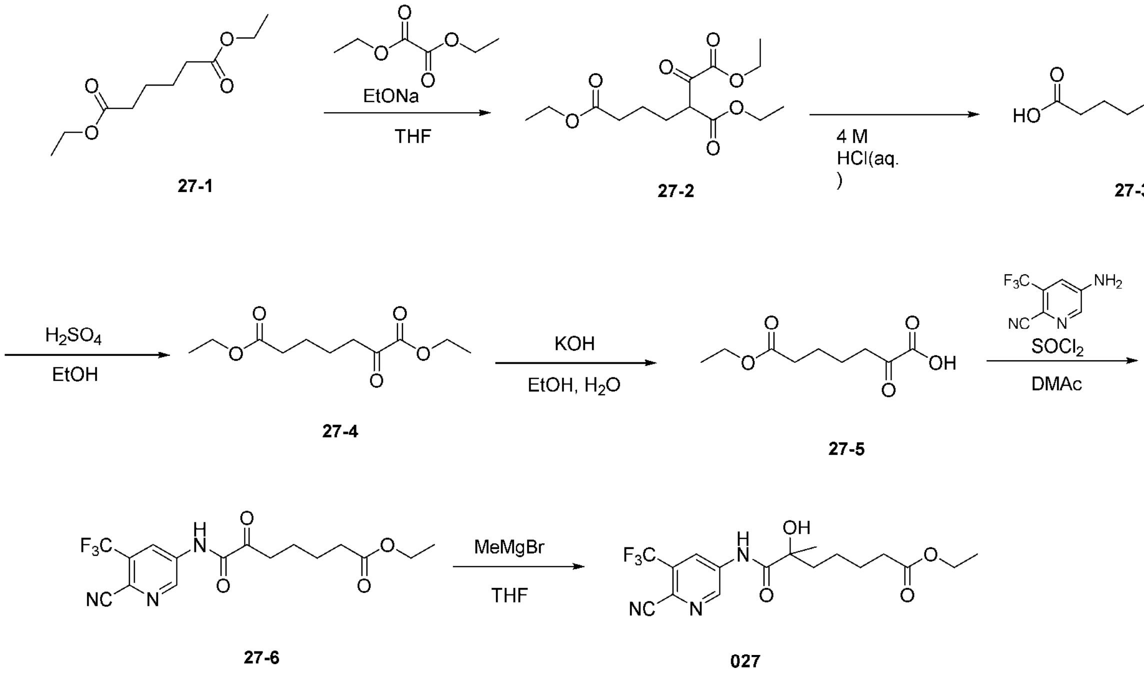


27-1 27-2 27-3 27-4 27-5 27-6 027

**Step 1: Synthesis of triethyl 1-oxopentane-1,2,5-tricarboxylate**

The starting material **27-1** (2 g, 9.89 mmol) was added to tetrahydrofuran (10 mL), and sodium ethoxide (672.45 mg, 9.89 mmol) was added in an ice-water bath. After the mixture was stirred at room temperature for 30 min, diethyl oxalate (1.45 g, 9.89 mmol) was added. The reaction mixture was stirred at room temperature for 18 h and diluted with 20 mL of water. The mixture was extracted with ethyl acetate (20 mL × 3), and the organic phases were combined, washed with water (30 mL × 2) and saturated brine (50 mL), dried over anhydrous sodium sulfate, filtered, and concentrated at reduced pressure to give title compound **27-2** (2.99 g).

$^1$H NMR (400 MHz, Chloroform-*d*)δ 4.45 - 4.29 (m, 2H), 4.25 - 4.09 (m, 4H), 4.02 (t, *J* = 7.0 Hz, 1H), 2.42 - 2.27 (m, 2H), 2.04 - 1.85 (m, 2H), 1.79 - 1.59 (m, 2H), 1.44 - 1.17 (m, 9H)

**Step 2: Synthesis of 2-oxoheptanedioic acid**

Intermediate **27-2** (6 g, 19.85 mmol) was added to a 4 M aqueous HCl solution (60 mL), and the reaction mixture was stirred at 70 °C for 18 h. After LCMS indicated the completion of the reaction, water (20 mL) was added for dilution, and the mixture was extracted with ethyl acetate (20 mL × 3). The organic phases were combined, washed with water (30 mL × 2) and saturated brine (50 mL), dried over anhydrous sodium sulfate, filtered, and concentrated at reduced pressure to give title compound **27-3** (3 g).

MS (ESI): m/z =173.00 [M-H]$^-$.

**Step 3: Synthesis of diethyl 2-oxoheptanedioate**

Intermediate **27-3** (2.8 g, 16.08 mmol) and concentrated sulfuric acid (3.15 g, 32.16 mmol, 1.71 mL) were added to ethanol (25 mL). The reaction mixture was stirred at 50 °C for 18 h. After LCMS indicated the completion of the reaction, the reaction mixture was cooled and poured into water (20 mL). The mixture was extracted with ethyl acetate (30 mL × 3), and the organic phases were combined, washed with water (40 mL × 2) and saturated brine (40 mL), dried over anhydrous sodium sulfate, filtered, concentrated at reduced pressure, and purified by column chromatography (silica, ethyl acetate/petroleum ether = 3/1) to give title compound **27-4** (1.6 g).

MS (ESI): m/z =231.1 [M+H]$^+$.

$^1$H NMR (400 MHz, Chloroform-*d*)δ 4.37 - 4.22 (m, 2H), 4.18 - 4.08 (m, 2H), 2.92 - 2.81 (m, 2H), 2.37 - 2.24 (m,

2H), 1.75 - 1.55 (m, 4H), 1.44 - 1.19 (m, 6H).

**Step 4: Synthesis of 7-ethoxy-2,7-dioxoheptanoic acid**

Intermediate **27-4** (166 mg, 720.93 μmol) was added to ethanol (2 mL) and water (1 mL), and potassium hydroxide (40.45 mg, 720.93 μmol) was added at 0 °C. The reaction mixture was stirred at room temperature for 1 h. After LCMS indicated the completion of the reaction, the reaction mixture was extracted and washed once with ethyl acetate (20 mL). The aqueous phase was adjusted to acidity (pH 2-3) with diluted hydrochloric acid (10 mL) and extracted with ethyl acetate (20 mL × 3). The organic phases were combined, dried over anhydrous sodium sulfate, filtered, and concentrated at reduced pressure to give title compound **27-5** (130 mg).
MS (ESI): m/z =201.20 $[M-H]^-$.
$^1H$ NMR (400 MHz, Chloroform-*d*)δ 4.27 - 4.07 (m, 2H), 3.01 - 2.92 (m, 2H), 2.39 - 2.31 (m, 2H), 1.78 - 1.59 (m, 2H), 1.35 - 1.18 (m, 3H).

**Step 5: Synthesis of ethyl 7-((6-cyano-5-(trifluoromethyl)pyridin-3-yl)amino)-6,7-dioxoheptanoate**

Intermediate **27-5** (324.18 mg, 1.60 mmol) and 5-amino-3-(trifluoromethyl)pyridine-2-carbonitrile (200 mg, 1.07 mmol) were added to N,N-dimethylacetamide (3 mL). Thionyl chloride (216.17 mg, 1.82 mmol) was added in an ice-water bath. The reaction mixture was stirred in an ice-water bath for 18 h. After LCMS indicated the completion of the reaction, the reaction mixture was poured into ice water, and the resulting mixture was extracted with ethyl acetate (5 mL × 3). The organic phases were combined, washed with water (40 mL × 2) and saturated brine (40 mL), dried over anhydrous sodium sulfate, filtered, and concentrated at reduced pressure to give title compound **27-6** (400 mg).
MS (ESI): m/z =372.10 $[M+H]^+$.

**Step 6: Synthesis of ethyl 7-((6-cyano-5-(trifluoromethyl)pyridin-3-yl)amino)-6-hydroxy-6-methyl-7-oxoheptanoate**

Intermediate **27-6** (200 mg, 538.63 μmol) was added to tetrahydrofuran (3 mL). A solution of methylmagnesium bromide in tetrahydrofuran (3 M, 540 μL) was added to the reaction mixture at - 78 °C. The reaction mixture was stirred at -78 °C for 18 h. After LCMS indicated the completion of the reaction, 30 mL of a saturated aqueous ammonium chloride solution was added to the reaction mixture, and the mixture was extracted with ethyl acetate (50 mL × 3). The organic phases were combined, dried over anhydrous sodium sulfate, filtered, and concentrated at reduced pressure. The residue was purified by column chromatography (silica, ethyl acetate/petroleum ether = 5/1) to give title compound **027** (40 mg).
MS (ESI): m/z =388.10 $[M+H]^+$.
$^1H$ NMR (400 MHz, DMSO-$d_6$)δ 10.70 (s, 1H), 9.39 (d, *J* = 2.2 Hz, 1H), 8.91 (d, *J* = 2.3 Hz, 1H), 5.87 (s, 1H), 4.01 (q, *J* = 7.1 Hz, 2H), 2.30 - 2.22 (m, 2H), 1.82 - 1.70 (m, 1H), 1.68 - 1.41 (m, 3H), 1.36 (s, 3H), 1.29 - 1.06 (m, 5H).

**Example 28: Ethyl 5-[(6-cyano-5-methylthiopyridin-3-yl)carbamoyl]-6,6,6-trifluoro-5-hydroxyhexanoate (compound 028)**

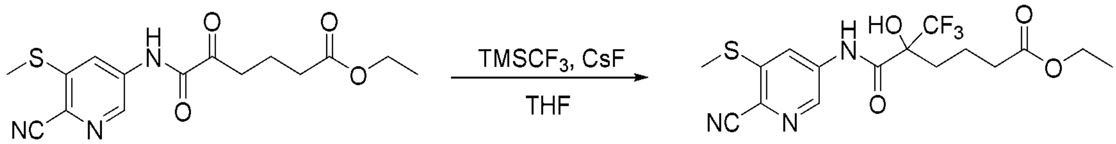


13-5 028

Intermediate **13-5** (1 g, 2.98 mmol) and (trifluoromethyl)trimethylsilane (2.12 g, 14.91 mmol) were added to tetrahydrofuran (20 mL), and cesium fluoride (9.85 mg, 64.84 μmol) was added in a nitrogen atmosphere. The reaction mixture was stirred at 25 °C for 3 h. After LCMS indicated the completion of the reaction, the reaction mixture was poured into ethyl acetate (20 mL) and water (20 mL), and the phases were separated. The aqueous phase was extracted twice with ethyl acetate (20 mL). The organic phases were combined, washed with saturated brine (30 mL), dried over anhydrous sodium sulfate, filtered, and concentrated. The crude product was purified by silica gel column chromatography (silica, petroleum ether/ethyl acetate = 3/2) to give compound **028** (120 mg).
MS (ESI): m/z = 406.1 $[M+H]^+$.
$^1H$ NMR (400 MHz, DMSO-$d_6$)δ 10.66 (s, 1H), 9.01 - 8.89 (m, 1H), 8.48 - 8.31 (m, 1H), 7.65 (s, 1H), 4.09 - 3.94 (m,

2H), 2.60 (s, 3H), 2.37 - 2.29 (m, 2H), 2.20 - 2.06 (m, 1H), 1.89 - 1.68 (m, 2H), 1.47 - 1.34 (m, 1H), 1.20 - 1.12 (m, 3H).

**Example 29: Ethyl 6-(4-cyano-3-methylthio-anilino)-5-hydroxy-5-methyl-6-oxohexanoate (compound 029)**

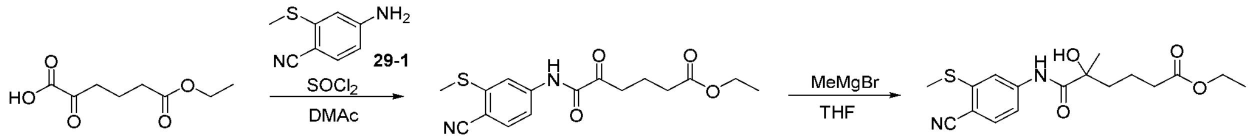


5-5 29-2 029

**Step 1: Synthesis of ethyl 6-(4-cyano-3-methylthio-anilino)-5,6-dioxo-hexanoate**

Intermediate **5-5** (1.01 g, 5.36 mmol) was added to N,N-dimethylacetamide (5 mL), and thionyl chloride (724.42 mg, 6.09 mmol) was added at -10 °C. 4-Amino-2-methylthio-benzonitrile (**29-1**, 500 mg, 3.04 mmol) was then added, and the reaction mixture was stirred at -78 °C for 15 h. After LCMS indicated the completion of the reaction, the reaction mixture was diluted with ethyl acetate (50 mL), and a saturated sodium bicarbonate solution (50 mL) was added to quench the reaction. The mixture was extracted with ethyl acetate (50 mL × 4). The organic phase was washed with saturated brine, dried over anhydrous sodium sulfate, and filtered. The filtrate was concentrated at reduced pressure, and the crude product was purified by column chromatography (silica, petroleum ether/ethyl acetate = 1/1) to give intermediate **29-2** (600 mg).

MS (ESI): m/z =335.20 $[M+H]^+$.

$^1H$ NMR (400 MHz, DMSO-$d_6$)δ 10.84 (s, 1H), 7.96 (d, *J* = 1.9 Hz, 1H), 7.84 (dd, *J* = 8.6, 1.9 Hz, 1H), 7.77 (d, *J* = 8.5 Hz, 1H), 4.06 (q, *J* = 7.1 Hz, 2H), 2.97 (t, *J* = 7.2 Hz, 2H), 2.56 (s, 3H), 2.36 (t, *J* = 7.4 Hz, 2H), 1.85 - 1.75 (m, 2H), 1.18 (t, *J* = 7.1 Hz, 3H).

**Step 2: Synthesis of ethyl 6-(4-cyano-3-methylthio-anilino)-5-hydroxy-5-methyl-6-oxohexanoate**

Intermediate **29-2** (400 mg, 1.20 mmol) was added to tetrahydrofuran (4 mL). The mixture was cooled to -78 °C, and a solution of methylmagnesium bromide in diethyl ether (3 M, 1.2 mL) was added with the temperature maintained. The reaction mixture was stirred at -78 °C for 15 h. After LCMS indicated the completion of the reaction, the reaction mixture was diluted with ethyl acetate (50 mL), and a saturated ammonium chloride solution (50 mL) was added at a low temperature to quench the reaction. The resulting mixture was extracted with ethyl acetate (50 mL × 4). The organic phase was washed with saturated brine, dried over anhydrous sodium sulfate, and filtered, and the filtrate was concentrated at reduced pressure. The crude product was separated and purified by Prep-HPLC [Phenomenex C18, 5 μm silica, 30 mm diameter, 80 mm length; a mixture of water (containing 0.05% ammonium bicarbonate) and acetonitrile (acetonitrile content: 50%-80%) was used as the eluent] to give target compound **029** (110 mg).

MS (ESI): m/z =351.20 $[M+H]^+$.

$^1H$ NMR (400 MHz, DMSO-$d_6$)δ 10.02 (s, 1H), 7.88 (d, *J* = 1.9 Hz, 1H), 7.79 (dd, *J* = 8.6, 1.9 Hz, 1H), 7.65 (d, *J* = 8.5 Hz, 1H), 5.76 (s, 1H), 3.97 (q, *J* = 7.1 Hz, 2H), 2.50 (s, 3H), 2.21 (t, *J* = 7.1 Hz, 2H), 1.73 - 1.58 (m, 2H), 1.55 - 1.47 (m, 1H), 1.41 - 1.31 (m, 1H), 1.28 (s, 3H), 1.10 (t, *J* = 7.1 Hz, 3H).

**Example 30: Ethyl 6-(4-cyano-3-methoxy-anilino)-5-hydroxy-5-methyl-6-oxohexanoate (compound 030)**

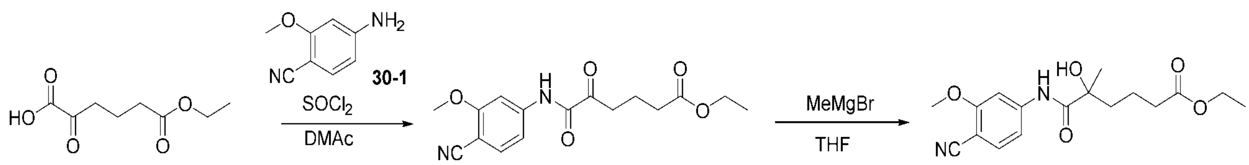


5-5 30-2 030

**Step 1: Synthesis of ethyl 6-(4-cyano-3-methoxy-anilino)-5,6-dioxo-hexanoate**

6-Ethoxy-2,6-dioxohexanoic acid (971.24 mg, 5.16 mmol) was added to N,N-dimethylacetamide (5 mL), and the

reaction mixture was cooled to -10 °C. Thionyl chloride (802.97 mg, 6.75 mmol) was added dropwise, and 4-amino-2-methoxy-benzonitrile (500 mg, 3.37 mmol) was then added. The reaction mixture was stirred at -78 °C for 15 h. After LCMS indicated the completion of the reaction, a saturated sodium bicarbonate solution (50 mL) was added to quench the reaction. The mixture was extracted with ethyl acetate (50 mL × 4). The organic phase was washed with saturated brine, dried over anhydrous sodium sulfate, and filtered. The filtrate was concentrated at reduced pressure, and the crude product was purified by column chromatography (silica, petroleum ether/ethyl acetate = 1/1) to give intermediate **30-2** (600 mg).

MS (ESI): m/z =319.20 $[M+H]^+$.

$^1$H NMR (400 MHz, DMSO-$d_6$)δ 10.77 (s, 1H), 7.79 (d, *J* = 1.8 Hz, 1H), 7.69 (d, *J* = 8.5 Hz, 1H), 7.59 (dd, *J* = 8.6, 1.8 Hz, 1H), 4.05 (q, *J* = 7.1 Hz, 2H), 3.87 (s, 3H), 2.95 (t, *J* = 7.2 Hz, 2H), 2.35 (t, *J* = 7.4 Hz, 2H), 1.84 - 1.73 (m, 2H), 1.17 (t, *J* = 7.1 Hz, 3H).

**Step 2: Synthesis of ethyl 6-(4-cyano-3-methoxy-anilino)-5-hydroxy-5-methyl-6-oxohexanoate**

Intermediate **30-2** (500 mg, 1.57 mmol) was added to tetrahydrofuran (5 mL), and a solution of methylmagnesium bromide in tetrahydrofuran (3 M, 1.57 mL) was added at -78 °C. The reaction mixture was stirred at -78 °C for 15 h. After LCMS indicated the completion of the reaction, the reaction mixture was diluted with ethyl acetate (50 mL), and a saturated ammonium chloride solution (50 mL) was added at a low temperature to quench the reaction. The resulting mixture was extracted with ethyl acetate (50 mL × 4). The organic phase was washed with saturated brine, dried over anhydrous sodium sulfate, and filtered, and the filtrate was concentrated at reduced pressure. The crude product was separated and purified by Prep-HPLC [Phenomenex C18, 5 μm silica, 30 mm diameter, 80 mm length; a mixture of water (containing 0.05% ammonium bicarbonate) and acetonitrile (acetonitrile content: 50%-80%) was used as the eluent] to give target compound **030** (110 mg).

MS (ESI): m/z =335.20 $[M+H]^+$.

$^1$H NMR (400 MHz, DMSO-$d_6$)δ 9.99 (s, 1H), 7.80 (d, *J* = 1.7 Hz, 1H), 7.66 - 7.56 (m, 2H), 5.81 (s, 1H), 4.02 (q, *J* = 7.1 Hz, 2H), 3.86 (s, 3H), 2.26 (t, *J* = 7.1 Hz, 2H), 1.79 - 1.65 (m, 2H), 1.61 - 1.38 (m, 2H), 1.33 (s, 3H), 1.15 (t, *J* = 7.1 Hz, 3H).

**Example 31: Ethyl 6-((6-cyano-5-(methylthio)pyridin-3-yl)amino)-5-hydroxy-6-oxohexanoate (compound 031)**

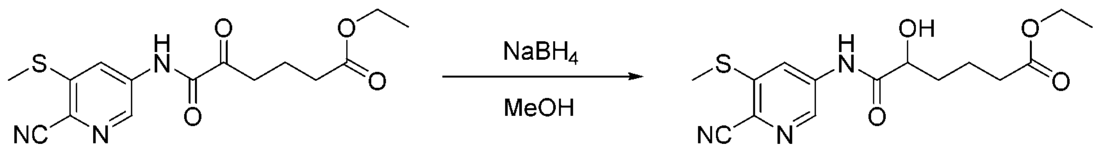


13-5 031

Compound **13-5** (10 mg, 29.82 μmol) was added to methanol (1 mL). Sodium borohydride (2.26 mg, 59.63 μmol) was added in an ice-water bath. The reaction mixture was stirred at room temperature for 1 h. After LCMS indicated the completion of the reaction, 10 mL of water was added to the reaction mixture in an ice-water bath to quench the reaction, and the resulting mixture was extracted with ethyl acetate (200 mL × 3). The organic phases were combined, washed with saturated brine (10 mL × 2), dried over anhydrous sodium sulfate, and filtered. The filtrate was concentrated at reduced pressure to give title compound **031** (9 mg).

MS (ESI): m/z =338.20 $[M+H]^+$.

**Example 32: Isopropyl 6-((6-cyano-5-(methylthio)pyridin-3-yl)amino)-5-hydroxy-6-oxohexanoate (compound 032)**

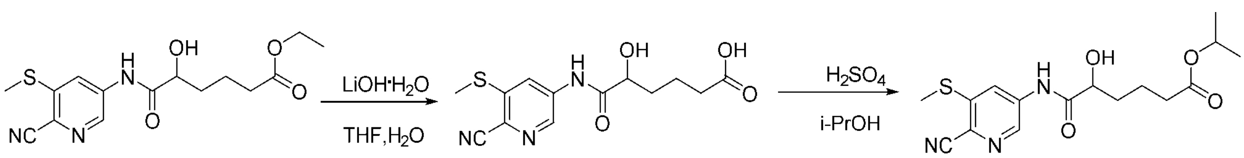


031 32-1 032

**Step 1: Synthesis of 6-(6-cyano-5-(methylthio)pyridin-3-yl)amino)-5-hydroxy-6-oxohexanoic acid**

Compound **031** (50 mg, 148.19 μmol) and lithium hydroxide monohydrate (7.11 mg, 169.45 μmol) were added to tetrahydrofuran (1 mL) and deionized water (1 mL). The reaction mixture was stirred at room temperature for 2 h. After LCMS indicated the completion of the reaction, the mixture was diluted with water (10 mL) and extracted with ethyl acetate (50 mL). A 1 M aqueous HCl solution (10 mL) was added to the aqueous phase to adjust the system to acidity (pH 2-3), and the mixture was extracted with ethyl acetate (20 mL × 3). The organic phases were combined, washed with saturated brine (20 mL × 2), dried over anhydrous sodium sulfate, and filtered. The filtrate was concentrated at reduced pressure to give title compound **32-1** (35 mg).
MS (ESI): m/z =309.6 $[M+H]^+$.
$^1H$ NMR (400 MHz, DMSO-$d_6$)δ 8.89 (d, *J* = 2.1 Hz, 1H), 8.36 (d, *J* = 2.1 Hz, 1H), 4.11 - 4.03 (m, 1H), 2.57 (s, 3H), 2.14 - 2.05 (m, 2H), 1.78 - 1.71 (m, 1H), 1.65 - 1.55 (m, 3H).

**Step 2: Synthesis of isopropyl 6-((6-cyano-5-(methylthio)pyridin-3-yl)amino)-5-hydroxy-6-oxohexanoate**

Intermediate **32-1** (50 mg, 161.63 μmol) and concentrated sulfuric acid (15.85 mg, 161.63 μmol) were added to isopropanol (1 mL). The reaction mixture was stirred at room temperature for 6 h. After LCMS indicated the completion of the reaction, the reaction mixture was filtered, and the filtrate was purified by Prep-HPLC [Phenomenex C18, 5 μm silica, 30 mm diameter, 80 mm length; a mixture of purified water and acetonitrile (acetonitrile content: 60%-70%) was used as the eluent] to give target compound **032** (10 mg).
MS (ESI): m/z =351.7 $[M+H]^+$.
$^1H$ NMR (400 MHz, DMSO-$d_6$)δ 8.89 (d, *J* = 2.1 Hz, 1H), 8.35 (d, *J* = 2.1 Hz, 1H), 6.03 (s, 1H), 4.93 - 4.82 (m, 1H), 4.13 - 4.05 (m, 1H), 2.58 (s, 3H), 2.33 - 2.22 (m, 2H), 1.79 - 1.68 (m, 1H), 1.70 - 1.53 (m, 3H), 1.20 - 1.14 (m, 6H).

**Experimental Example 1: Effect of compounds disclosed herein on AR antagonistic activity**

**(1). Materials and reagents:**

| Product | Supplier | Cat. No. |
|---|---|---|
| LNCaP cell | ATCC | CRL-1740 |
| RPMI-1640 | ATCC | 30-2001 |
| Serum | Ausgenex | FBS500-S |
| 96-Well plate | Corning | 3603 |
| Lipofectamine™ 3000 | Invitrogen | L3000015 |
| Bright-Lite Luciferase Assay System | Vazyme | DD1204-03 |
| 6-Well plate | Corning | 3516 |

**(2). Instrument:**

| Product | Supplier | Cat. No. |
|---|---|---|
| Biosafety cabinet | ESCO | AC2-6S1-TC |
| Carbon dioxide incubator | ESCO | CLM-240B-8-TC |
| Countess™ II cell counter | NanoEnTeK | EVE-MC2 |
| Microplate centrifuge | Monad | PlatePro 3200 |
| Microplate reader | BMG LABTECH | PHERAstar FSX |

**(3) Procedures:**

Day one: cell plating

LNCaP cells were plated in a 6-well plate (Corning, 3516) at $8 \times 10^5$ cells/well.

Day two: transfection

Transfection reagents Lipo 3000 and P3000 (Lipofectamine™ 3000) and AR and PSA ARE1 reporter gene plasmids (Bio (Anhui) Co., Ltd.) (a total of 2.5 μg of plasmids, i.e., 1.25 μg of each plasmid, were added to each well) were left to stand and incubated at room temperature for 10 min, and then 250 μL of the above transfection reagents were added dropwise to each well containing cells.

Day three: cell treatment, plating, and drug addition

The cells were washed with PBS, and the medium was replaced with a phenol red-free RPMI-1640 medium containing 5% carbon adsorption serum. The cells were re-plated onto a 96-well plate (Corning, 3603) according to the number of cells plated ($2 \times 10^4$ cells/well).

After cell plating, the test compound (or DMSO or positive control) was added. After at least 30 min of incubation (incubator, 37 °C, 5% $CO_2$), DHT (dihydrotestosterone) was added to the culture system at a final concentration of 1 nM. The culture system was then incubated in a carbon dioxide incubator. The final volume of the system in the plate was 100 μL.

Test compound: 10 mM stock solution, with a final concentration of 3.3 μM or 6.7 μM. Negative control: 0.1% DMSO. Positive control: 30 μM enzalutamide.

Day four: luciferase signal detection and data processing

After the test compound (or DMSO or positive control) and DHT were co-incubated for 6 or 24 h, the luciferase signal was detected using the Vazyme Bright-Lite substrate. The medium was added at 70 μL/well, and the plate was shaken for 2 min, followed by reading of the luciferase signal.

**(4). Data processing**:

Assay robustness was confirmed using the DMSO and low concentration control data:

$$\text{Ave_H} = \text{Ave (DMSO)}$$

$$\text{Ave_L} = \text{Ave (30000 nM enzalutamide)}$$

$$\text{Inhibition rate (\%)} = (\text{Ave_H} - \text{Sample})/(\text{Ave_H} - \text{Ave_L}) \times 100\%$$

Ave_H: the measurement of the negative control (0.1% DMSO);
Ave_L: the measurement of the positive control (30 μM enzalutamide);
Sample: the measurement of the test compound;

The corresponding activity test results for the test compounds were specified in Table 1.

Table 1. Corresponding activity test results for compounds disclosed herein

| Compound | AR antagonism (inhibition rate, %) |
|---|---|
| 001 | 87.89[a] |
| 002 | 59.75[a] |
| 005 | 92.96[b] |
| 006 | 90.51[c] |
| 006 | 88.13[a] |
| 007 | 100.00[c] |

(continued)

| Compound | AR antagonism (inhibition rate, %) |
|---|---|
| 008 | 92.41[b] |
| 009 | 97.66[c] |
| 010 | 85.37[c] |
| 014 | 64.39[c] |
| 015 | 92.46[a] |
| 018 | 63.12[c] |
| 019 | 57.98[b] |
| 024 | 88.18[a] |
| 027 | 76.55[a] |
| 029 | 98.41[a] |
| 030 | 98.45[a] |
| 032 | 92.25[a] |
| Note: a denotes incubation time: 24 h, test concentration: 3.3 μM; b denotes incubation time: 6 h, test concentration: 6.7 μM; c denotes incubation time: 24 h, test concentration: 6.7 μM. | |

As can be seen from the above results, the compounds disclosed herein have AR antagonistic activity.

**Experimental Example 2: Metabolic stability assay of compounds disclosed herein in liver microsomes**

The metabolic stability of compounds disclosed herein in liver microsomes was determined using the following method.

I. Materials and instruments

1. Liver microsome source: Human liver microsome (Corning, 452117)
2. $Na_2HPO_4$ (Tianjin Guangfu Fine Chemical Research Institute, 20180130)
3. $KH_2PO_4$ (Tianjin Guangfu Fine Chemical Research Institute, 20180920)
4. $MgCl_2$ (Tianjin Guangfu Fine Chemical Research Institute, 20191216)
5. NADPH (Solarbio, 1216C022)
6. Positive control compound: verapamil (Sigma, MKBV4993V)
7. AB Sciex Triple Quad 4000 liquid chromatography-mass spectrometry system

II. Procedures

1. Preparation of 100 mM phosphate-buffered saline (PBS): 7.098 g $Na_2HPO_4$ was dissolved in 500 mL of purified water by sonication to give solution A. 3.400 g of $KH_2PO_4$ was dissolved in 250 mL of purified water by sonication to give solution B. Solution A was placed on a stirrer, and solution B was added slowly until the pH reached 7.4, so as to give a 100 mM PBS buffer.
2. Preparation of reaction system
The reaction system was prepared according to the table below.

| Reagent | Stock solution concentration | Volume | Final concentration |
|---|---|---|---|
| Liver microsome | 20 mg/mL | 10 μL | 0.5 mg/mL |
| Phosphate-buffered saline | 100 mM | 346 μL | 100 mM |

3. The reaction system was pre-incubated in a 37 °C water bath for 10 min. 40 μL of 10 mM NADPH solution (in 100 mM phosphate-buffered saline) was added to the reaction system, with the final NADPH concentration being 1 mM. A negative control was prepared by replacing the NADPH solution with 40 μL of phosphate-buffered saline. The negative control was used to exclude the effect of the chemical stability of the compound itself.
4. The reaction was initiated by adding 4 μL of 100 μM compound disclosed herein or positive control compound verapamil to the reaction system at a final concentration of 1 μM.
5. After 0.5 min, 15 min, 30 min, 45 min, and 60 min of mixing well by using a vortex oscillator, 50 μ L of the incubated sample was taken out, and glacial acetonitrile containing an internal standard of 4 folds was added to terminate the reaction. The sample was centrifuged at 3,220 g for 45 min. 90 μL of the supernatant was transferred to a feeding plate after the centrifugation was completed, and 90 μL ultrapure water was added. The mixture was well mixed for LC-MS/MS analysis.

All data were calculated by using Microsoft Excel software. The peak areas were determined by using an extracted ion chromatogram. The *in vitro* half-life ($T_{1/2}$) of the test compound was determined by linear fitting of the natural logarithm of the elimination proportion of the test compound over time. *In vitro* half-life ($T_{1/2}$) was calculated by the slope k:

$$\textit{in vitro}\ T_{1/2} = 0.693/k$$

The *in vitro* intrinsic clearance (*in vitro* $CL_{int}$, unit: μL/min/mg protein) was calculated using the following formula:

$$\textit{in vitro}\ CL_{int} = k \times \text{volume of incubation solution/enzyme protein content;}$$

The calculated $T_{1/2}$ and $CL_{int}$ according to the above formulas are shown in Table 2.

Table 2. Half-life values and intrinsic clearance values of compounds disclosed herein in liver microsomes

| Compound | In vitro $T_{1/2}$ (min) | In vitro $Cl_{int}$ (μL/min/mg protein) |
|---|---|---|
| 005 | 2.1 | 670.7 |
| 006 | 6.9 | 199.5 |
| 007 | 6.1 | 226.3 |
| 008 | 10.6 | 131.2 |
| 009 | 5.9 | 234.1 |
| 010 | 11.1 | 124.8 |
| 013 | 2.8 | 487.2 |
| 015 | 5.12 | 270.78 |
| 016 | 1.14 | 1216.15 |
| 017 | 5.7 | 242.7 |
| 018 | 5.6 | 247.8 |

As can be seen from the above results, the compounds disclosed herein are characterized by high clearance and rapid metabolism, which may avoid the accumulation of the compound in the body caused by long-term treatment and thus the systemic impact on the androgen receptor signaling pathway, thereby further improving the safety of the treatment.

**Experimental Example 3: Whole blood stability**

Procedures:

1. Preparation of stock solution

1 mM stock solutions of the test compounds were prepared in DMSO, and a 1 mM stock solution of the reference compound propantheline was prepared in acetonitrile.

2. Stability assay

4 μL of test compound stock solution (or reference compound stock solution) was added to 796 μL of pre-

incubated whole blood (CD-1 mouse whole blood or human whole blood) at a final concentration of 5 μM. The final concentration of the solvent was 0.5%. At different time points (including 5 min, 15 min, 30 min, 60 min, and 120 min), a 50 μL aliquot of the whole blood containing the sample was added to a new tube and incubated in a 37 °C water bath with shaking at 60 rpm. The treatment was performed in duplicate. At designated time points, 300 μL of room-temperature quenching solution (a solution of acetonitrile containing internal standards (100 nM alprazolam, 500 nM labetalol, and 2 μM ketoprofen)) was added to the whole blood sample containing the sample to terminate the reaction. 50 μL of whole blood containing the sample was added to a new tube containing 300 μL of room-temperature quenching solution to prepare a sample at 0 min. All samples were vortexed for 5 min. The samples in the plate were centrifuged at 3220 g for 30 min at 4 °C to precipitate the proteins. 100 μL of the supernatant was transferred to a new 96-well plate containing 100 μL of water for LC-MS/MS analysis.

3. Data analysis

All calculations were performed using Microsoft Excel. The peak area ratio was determined by using an extracted ion chromatogram. The proportion of remaining compound at each time point was calculated according to the following formula:

$$\text{Remaining proportion t min (\%)} = \text{peak area ratio t min/peak area ratio 0 min} \times 100\%$$

Peak area ratio t min: peak area ratio of reference compound to test compound at t min;
Peak area ratio 0 min: peak area ratio of reference compound to test compound at time zero.

The slope value was determined by linear regression on the curve of the natural logarithm of the remaining proportion of the test compound versus the incubation time.

Determination of *in vitro* half-life (in vitro $t_{1/2}$) from the slope value:

$$in\ vitro\ t_{1/2} = 0.693/k$$

in the formula, k is the rate constant (k = -slope value)

The determined half-lives of the compounds disclosed herein in CD-1 mouse or human whole blood are shown in Table 3.

Table 3. Stability of compounds disclosed herein in CD-1 mouse or human whole blood

| Compound | Half-life $T_{1/2}$ in CD-1 mouse whole blood (min) | Half-life $T_{1/2}$ in human whole blood (min) |
|---|---|---|
| 005 | 3.54 | 119.01 |
| 006 | 3.61 | 183.42 |
| 007 | 2.70 | 82.65 |
| 008 | 3.94 | 141.44 |
| 009 | 10.03 | 210.07 |
| 010 | 5.97 | 351.20 |
| 013 | <0.5 | 118.50 |
| 014 | 1.13 | 89.49 |
| 015 | 4.34 | 164.02 |
| 016 | 9.44 | 70.19 |
| 017 | 4.64 | 206.18 |
| 018 | 4.93 | 194.81 |

As can be seen from the above results, the compounds disclosed herein are characterized by short half-life, high clearance, and rapid metabolism in CD-1 mouse or human whole blood, which may avoid the accumulation of the compound in the body caused by long-term treatment and thus the systemic impact on the androgen receptor signaling pathway, thereby further improving the safety of the treatment.

### Experimental Example 4: *In vivo* pharmacodynamic study

#### 4.1 Reagents

Vehicle: propylene glycol/ethanol (30:70, v/v)

#### 4.2 Procedures

Animal information: C57BL/6 mice (male, aged 6 weeks, about 18-20 g), purchased from Shanghai Lingchang Biotechnology Co., Ltd. The mice were bred in an SPF-level environment within independently ventilated cages. All animals had free access to a certified, commercial standard laboratory diet and drinking water.

Skin preparation: After 1-2 weeks of acclimation, the hairs in a 2 cm × 3 cm area of the back of the mice were shaved to confirm that the hairs of the mice were in the telogen phase (the skin was pink) and the skin was not damaged.

Treatment: On day 3 after shaving, the mice were grouped for treatment. Compound **010** disclosed herein was applied to the shaved area at a concentration of 0.5 wt% twice daily in the morning and evening for 32 days. The control group received the vehicle. 12 mice were in each group.

#### 4.3 Mouse state observation and hair growth scoring

During the experiment, the states of the mice in each group were observed for erythema, dehiscence, desquamation, and the like on the skin.

The mice were weighed on days 10, 13, 16, 20, 23, 27, and 32 of compound **010** treatment, and the hair growth state was scored and photographed.

The scoring criteria are as follows:

0: no growth, with pink skin in the shaved area;
1: the skin in the shaved area was gray (an increase of less than 20%);
2: the skin in the shaved area was black (more than 20% but less than 40%);
3: the skin in the shaved area was black with a little hair growth (more than 40% but less than 60%);
4: the skin in the shaved area was black with partial hair growth (more than 60% but less than 80%);
5: basically complete hair growth (80%-100% growth) in the shaved area.

#### 4.4 Results

The effects of the compounds disclosed herein on mouse hair growth are shown in Table 4.

Table 4. Scoring of effect of compounds disclosed herein on hair growth in mice

| Compound | Score (mean±SD) | | | | | | | |
|---|---|---|---|---|---|---|---|---|
| | Day 0 | Day 10 | Day 13 | Day 16 | Day 20 | Day 23 | Day 27 | Day 32 |
| **Vehicle control group** | 0 | 0.0±0.0 | 0.0±0.0 | 0.1±0.3 | 0.3±0.5 | 0.3±0.5 | 0.6±0.7 | 1.1±0.8 |
| **010** | 0 | 0.2±0.4 | 0.2±0.4 | 0.3±0.5 | 0.7±0.7 | 0.8±0.6 | 1.1±0.7 | 1.9±0.7** |
| *** *P* < 0.05, ** *P* < 0.01 vs. vehicle control group** | | | | | | | | |

#### 4.5 Conclusion

In mice with telogen hair growth, compound **010** disclosed herein at a concentration of 0.5 wt%, administered BID topically at 20 μL/$cm^2$, exhibited a significant promoting effect on hair growth. Compound **010** significantly promoted the hair growth ($P < 0.01$) from day 32 of the treatment. Compound **010** exhibited no significant impact on the body weight of the mice at the investigated doses and no signs of erythema, dehiscence, desquamation, and the like on the skin. The other compounds disclosed herein also have significant promoting effects on hair growth.

Although the specific embodiments of the present disclosure have been illustrated and described, those skilled in the art will appreciate that, where feasible, the technical features described in one embodiment can be applied to or combined with the technical features described in another embodiment. Therefore, those skilled in the art can make various changes and modifications to the embodiments of the present disclosure without departing from the spirit and scope of the present disclosure.

**Claims**

1. A compound of formula (I) or a stereoisomer thereof or a pharmaceutically acceptable salt thereof,

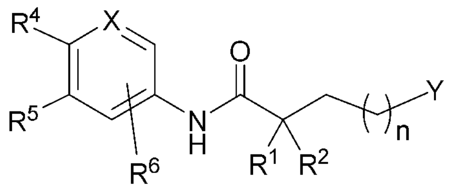

(I)

wherein

X is selected from CH or N;
Y is selected from OH, COOH, $-CONH_2$, $-COOR^3$, or $-CONR^{3'}R^3$;
$R^1$ is selected from OH or $-O-C_1-C_6$ alkyl;
$R^2$ is selected from H, deuterium, $C_1-C_6$ alkyl, $C_1-C_6$ deuterated alkyl, or $C_1-C_6$ haloalkyl;
$R^3$ is selected from $C_1-C_6$ alkyl, $C_1-C_6$ deuterated alkyl, $C_3-C_6$ cycloalkyl, 4- to 10-membered heterocyclyl, $C_6-C_{10}$ aryl, or 5- to 10-membered heteroaryl, wherein the $C_1-C_6$ alkyl, $C_3-C_6$ cycloalkyl, 4- to 10-membered heterocyclyl, $C_6-C_{10}$ aryl, or 5- to 10-membered heteroaryl is optionally substituted with $R^{3a}$;
$R^{3a}$ is selected from halogen, OH, CN, $NH_2$, $-COR^{3b}$, $-COOR^{3b}$, $-NHCOR^{3b}$, $-CONHR^{3b}$, $-O-C_1-C_6$ alkyl, $C_1-C_6$ alkyl, phenyl, 4- to 6-membered heterocyclyl, or 5- to 6-membered heteroaryl, wherein the $C_1-C_6$ alkyl, phenyl, 4- to 6-membered heterocyclyl, or 5- to 6-membered heteroaryl is optionally substituted with $R^{3c}$;
$R^{3b}$ is selected from H or $C_1-C_6$ alkyl;
$R^{3c}$ is selected from halogen, OH, $C_1-C_6$ alkyl, or $-O-C_1-C_6$ alkyl;
$R^{3'}$ is selected from H or $C_1-C_6$ alkyl;
$R^4$ is selected from $NO_2$, CN, halogen, $C_1-C_6$ alkyl, $C_1-C_6$ haloalkyl, or $-O-C_1-C_6$ alkyl;
$R^5$ is selected from halogen, $C_1-C_6$ alkyl, $C_1-C_6$ haloalkyl, $-COOC_1-C_6$ alkyl, $-S-C_1-C_6$ alkyl, $-O-C_1-C_6$ alkyl, $-Se-C_1-C_6$ alkyl, $-S(O)-C_1-C_6$ alkyl, or $-S(O)_2-C_1-C_6$ alkyl;
$R^6$ is selected from H, halogen, $C_1-C_6$ alkyl, $C_1-C_6$ haloalkyl, or $-O-C_1-C_6$ alkyl;
n is selected from 1, 2, 3, 4, 5, 6, 7, 8, or 9;
with the provision that, when Y is selected from OH, n is selected from 1, 2, or 3.

2. The compound of formula (I) or the stereoisomer thereof or the pharmaceutically acceptable salt thereof according to claim 1, wherein Y is selected from COOH, $-CONH_2$, $-COOR^3$, or $-CONR^{3'}R^3$; or Y is selected from $CONH_2$, $-COOR^3$, or $-CONR^{3'}R^3$;

or Y is selected from $-COOR^3$ or $-CONR^{3'}R^3$;
or Y is $-COOR^3$;
or Y is selected from OH, COOH, $-COOCH_2CH_3$, $-COOCH_3$, $-COOCH(CH_3)_2$, $-COOC(CH_3)_3$, $-CONHCH_3$, or $-CONH_2$;
or Y is selected from $-COOCH_2CH_3$, $-COOCH_3$, $-COOCH(CH_3)_2$, $-COOC(CH_3)_3$, or $-CONHCH_3$;
or Y is selected from $-COOCH_2CH_3$, $-COOCH_3$, $-COOCH(CH_3)_2$, or $-COO(CH_3)_3$.

3. The compound of formula (I) or the stereoisomer thereof or the pharmaceutically acceptable salt thereof according to claim 1 or 2, wherein $R^1$ is OH.

4. The compound of formula (I) or the stereoisomer thereof or the pharmaceutically acceptable salt thereof according to any one of claims 1-3, wherein $R^2$ is selected from H, $C_1-C_6$ alkyl, or $C_1-C_6$ haloalkyl; or

$R^2$ is selected from $C_1-C_6$ alkyl, $C_1-C_6$ deuterated alkyl, or $C_1-C_6$ haloalkyl;
or $R^2$ is selected from $C_1-C_6$ alkyl or $C_1-C_6$ haloalkyl;
or $R^2$ is selected from H, $CH_3$, or $CF_3$;
or $R^2$ is selected from $CH_3$ or $CF_3$.

**5.** The compound of formula (I) or the stereoisomer thereof or the pharmaceutically acceptable salt thereof according to any one of claims 1-4, wherein $R^3$ is selected from $C_1$-$C_6$ alkyl, $C_1$-$C_6$ deuterated alkyl, $C_3$-$C_6$ cycloalkyl, 4- to 6-membered heterocyclyl, $C_6$-$C_{10}$ aryl, or 5- to 6-membered heteroaryl, wherein the $C_1$-$C_6$ alkyl, $C_3$-$C_6$ cycloalkyl, 4- to 6-membered heterocyclyl, $C_6$-$C_{10}$ aryl, or 5- to 6-membered heteroaryl is optionally substituted with $R^{3a}$;

or $R^3$ is selected from $C_1$-$C_6$ alkyl or $C_1$-$C_6$ deuterated alkyl, wherein the $C_1$-$C_6$ alkyl is optionally substituted with $R^{3a}$;
or $R^3$ is unsubstituted $C_1$-$C_6$ alkyl;
or $R^3$ is selected from unsubstituted methyl, unsubstituted ethyl, unsubstituted isopropyl, or unsubstituted tert-butyl.

**6.** The compound of formula (I) or the stereoisomer thereof or the pharmaceutically acceptable salt thereof according to any one of claims 1-5, wherein $R^{3a}$ is selected from halogen, OH, CN, $NH_2$, $-COR^{3b}$, $-COOR^{3b}$, $-NHCOR^{3b}$, $-CONHR^{3b}$, -O-$C_1$-$C_6$ alkyl, or $C_1$-$C_6$ alkyl, wherein the $C_1$-$C_6$ alkyl is optionally substituted with $R^{3c}$.

**7.** The compound of formula (I) or the stereoisomer thereof or the pharmaceutically acceptable salt thereof according to any one of claims 1-6, wherein $R^{3'}$ is H or $C_1$-$C_4$ alkyl; or $R^{3'}$ is H.

**8.** The compound of formula (I) or the stereoisomer thereof or the pharmaceutically acceptable salt thereof according to any one of claims 1-7, wherein $R^4$ is selected from halogen or CN; or $R^4$ is selected from F, Cl, or CN; or $R^4$ is CN.

**9.** The compound of formula (I) or the stereoisomer thereof or the pharmaceutically acceptable salt thereof according to any one of claims 1-8, wherein $R^5$ is selected from halogen, $C_1$-$C_6$ alkyl, $C_1$-$C_6$ haloalkyl, -$COOC_1$-$C_6$ alkyl, -S-$C_1$-$C_6$ alkyl, or -O-$C_1$-$C_6$ alkyl;

or $R^5$ is selected from $C_1$-$C_6$ haloalkyl, -S-$C_1$-$C_6$ alkyl, -O-$C_1$-$C_6$ alkyl, -Se-$C_1$-$C_6$ alkyl, -S(O)-$C_1$-$C_6$ alkyl, or -$S(O)_2$-$C_1$-$C_6$ alkyl;
or $R^5$ is selected from $C_1$-$C_3$ haloalkyl, -S-$C_1$-$C_3$ alkyl, -O-$C_1$-$C_3$ alkyl, or -Se-$C_1$-$C_3$ alkyl;
or $R^5$ is selected from $C_1$-$C_6$ haloalkyl, -S-$C_1$-$C_6$ alkyl, or -O-$C_1$-$C_6$ alkyl;
or $R^5$ is selected from $CF_3$, -S-$CH_3$, -O-$CH_3$, -Se-$CH_3$, -S(O)-$CH_3$, or -$S(O)_2$-$CH_3$;
or $R^5$ is selected from $CF_3$, -S-$CH_3$, -O-$CH_3$, or -Se-$CH_3$.

**10.** The compound of formula (I) or the stereoisomer thereof or the pharmaceutically acceptable salt thereof according to any one of claims 1-9, wherein $R^6$ is selected from H or halogen; or $R^6$ is H.

**11.** The compound of formula (I) or the stereoisomer thereof or the pharmaceutically acceptable salt thereof according to any one of claims 1-10, wherein n is selected from 1, 2, or 3;

or n is selected from 1 or 2;
or n is 2.

**12.** The compound of formula (I) or the stereoisomer thereof or the pharmaceutically acceptable salt thereof according to any one of claims 1-11, wherein X is N.

**13.** The compound of formula (I) or the stereoisomer thereof or the pharmaceutically acceptable salt thereof according to any one of claims 1-12, wherein the compound of formula (I) or the stereoisomer thereof or the pharmaceutically acceptable salt thereof is selected from a compound of formula (II) or a stereoisomer thereof or a pharmaceutically acceptable salt thereof,

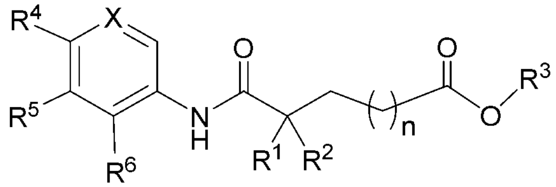

(II)

wherein X, $R^1$, $R^2$, $R^3$, $R^4$, $R^5$, $R^6$, and n are as defined in any one of claims 1-12.

**14.** The compound of formula (I) or the stereoisomer thereof or the pharmaceutically acceptable salt thereof according to claim 1, wherein the compound of formula (I) or the stereoisomer thereof or the pharmaceutically acceptable salt thereof is selected from the following compounds or stereoisomers thereof or pharmaceutically acceptable salts thereof:

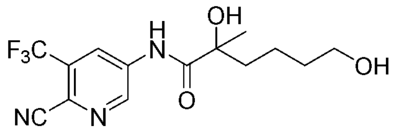 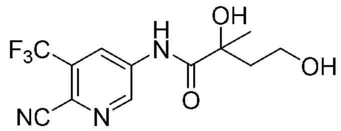 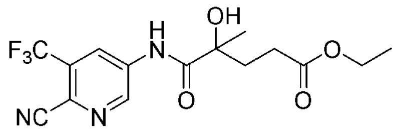

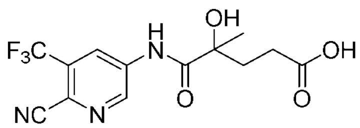 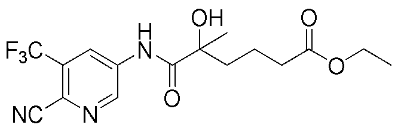 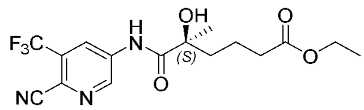

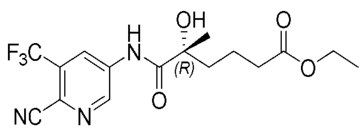 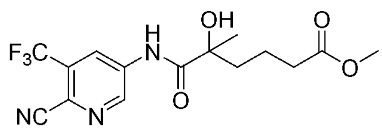 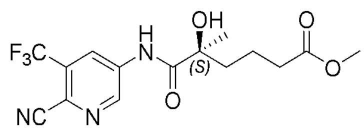

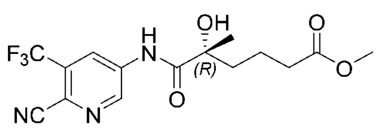 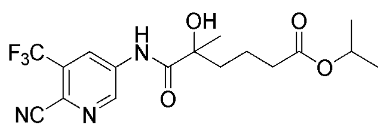 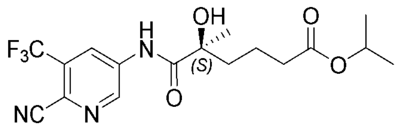

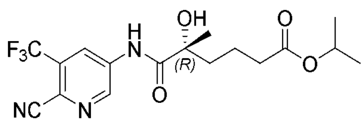 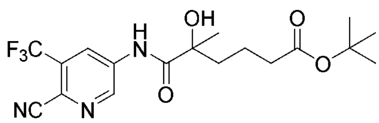 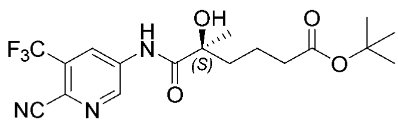

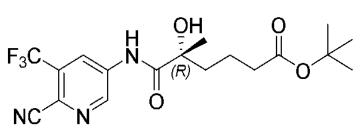 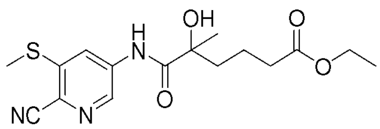 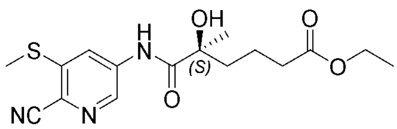

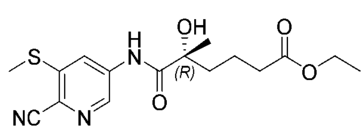 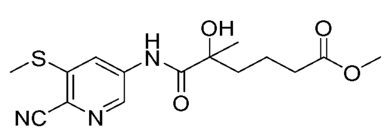 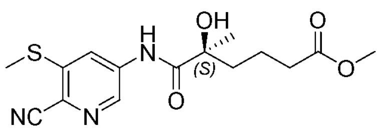

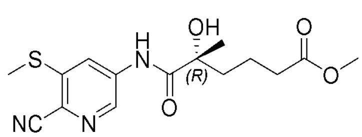 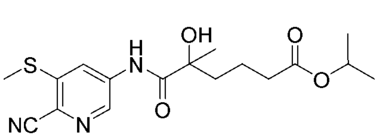 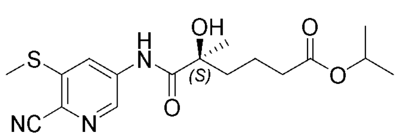

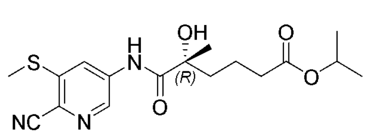 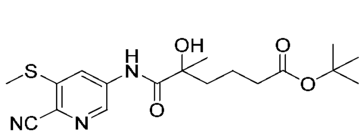 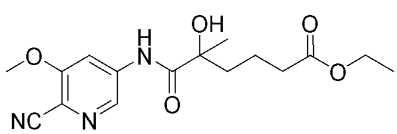

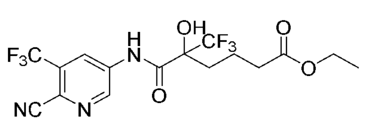
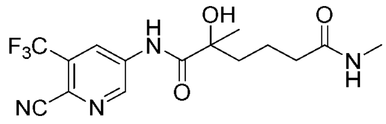
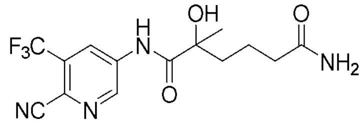
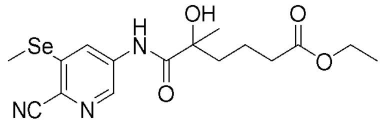
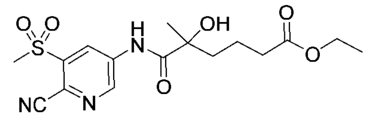
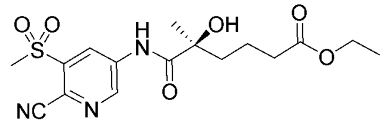
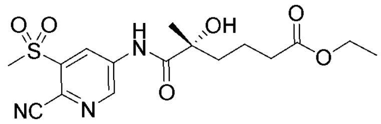
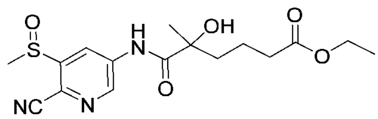
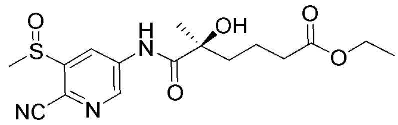
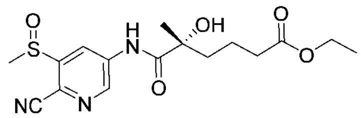
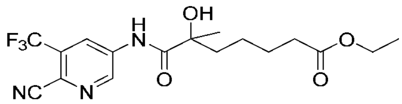
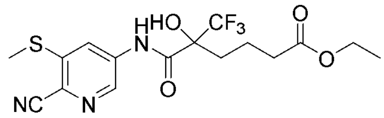
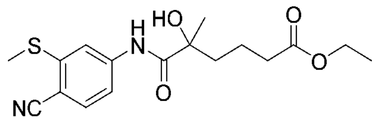
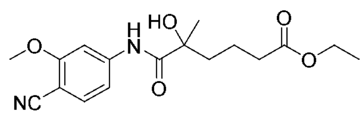
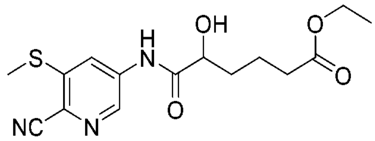

or

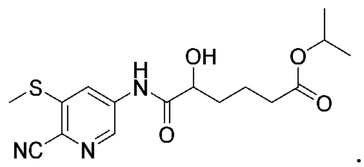

.

**15.** A pharmaceutical composition, comprising the compound of formula (I) or the stereoisomer thereof or the pharmaceutically acceptable salt thereof according to any one of claims 1-14 and a pharmaceutically acceptable excipient.

**16.** Use of the compound of formula (I) or the stereoisomer thereof or the pharmaceutically acceptable salt thereof according to any one of claims 1-14 or the pharmaceutical composition according to claim 15 in preparing a medicament for preventing or treating an androgen receptor-mediated disease.

**17.** A method for preventing or treating an androgen receptor-mediated disease in a mammal, comprising administering to a mammal, preferably a human, in need of the treatment a therapeutically effective amount of the compound of formula (I) or the stereoisomer thereof or the pharmaceutically acceptable salt thereof according to any one of claims 1-14 or the pharmaceutical composition according to claim 15.

**18.** The compound of formula (I) or the stereoisomer thereof or the pharmaceutically acceptable salt thereof according to any one of claims 1-14, or the pharmaceutical composition according to claim 15 for use in preventing or treating an androgen receptor-mediated disease in a mammal.

**19.** The use according to claim 16, the method according to claim 17, or the compound of formula (I) or the stereoisomer thereof or the pharmaceutically acceptable salt thereof or the pharmaceutical composition according to claim 18, wherein the androgen receptor-mediated disease is selected from androgenetic alopecia.

## INTERNATIONAL SEARCH REPORT

International application No. **PCT/CN2024/121910**

### A. CLASSIFICATION OF SUBJECT MATTER

C07D213/84(2006.01)i; C07D213/75(2006.01)i; C07C255/60(2006.01)i; C07C235/38(2006.01)i; A61K31/277(2006.01)i; A61K31/44(2006.01)i; A61P5/28(2006.01)i; A61P17/14(2006.01)i

According to International Patent Classification (IPC) or to both national classification and IPC

### B. FIELDS SEARCHED

Minimum documentation searched (classification system followed by classification symbols)

IPC:C07D,C07C,A61K,A61P

Documentation searched other than minimum documentation to the extent that such documents are included in the fields searched

Electronic data base consulted during the international search (name of data base and, where practicable, search terms used)

CNABS, CNTXT, DWPI, ENTXTC, STN (REGISTRY, CAPLUS, CASREACT): 中国药科大学, 酰胺, 吡啶, 苯, 氰基, 雄激素受体, 雄激素性脱发, +amide, pyridin+, phenyl, cyano, AR, androgen receptor, AGA, androgenitic alopecia, STN中结构式检索, search for structural formula in STN

### C. DOCUMENTS CONSIDERED TO BE RELEVANT

| Category* | Citation of document, with indication, where appropriate, of the relevant passages | Relevant to claim No. |
|---|---|---|
| X | WO 2008044033 A1 (ASTRAZENECA AB et al.) 17 April 2008 (2008-04-17) claims 1-18, and description, pages 44-45, 55, 66, 77, 89, 100, 117, 139 and 142-143 | 1-11, 13-19 |
| Y | WO 2008044033 A1 (ASTRAZENECA AB et al.) 17 April 2008 (2008-04-17) claims 1-18, and description, pages 44-45, 55, 66, 77, 89, 100, 117, 139 and 142-143 | 1-19 |
| Y | CN 109310664 A (UNIV TENNESSEE RES FOUNDATION) 05 February 2019 (2019-02-05) claims 1-50 | 1-19 |
| X | HALLINGER, S. et al. "Levels of 3,4-dichloroaniline in White Rot Fungal Cultures" *Chemosphere*. Vol. 17, No. (3), 31 December 1988 (1988-12-31), pages 543-550 page 547, table 1 | 1-11, 18 |
| X | WO 2023145769 A1 (SUMITOMO CHEMICAL COMPANY, LIMITED) 03 August 2023 (2023-08-03) description, page 137 | 1-9, 11, 18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 January 2025** | **10 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121910**

## C. DOCUMENTS CONSIDERED TO BE RELEVANT

| Category* | Citation of document, with indication, where appropriate, of the relevant passages | Relevant to claim No. |
|---|---|---|
| A | EP 0100172 A1 (IMPERIAL CHEMICAL INDUSTRIES PLC) 08 February 1984 (1984-02-08)<br>entire document | 1-19 |
| A | CN 1416416 A (BIOPHYSICA, INC.) 07 May 2003 (2003-05-07)<br>entire document | 1-19 |
| A | WO 2023011596 A1 (CHINA PHARMACEUTICAL UNIVERSITY et al.) 09 February 2023 (2023-02-09)<br>entire document | 1-19 |

Form PCT/ISA/210 (second sheet) (July 2022)

**INTERNATIONAL SEARCH REPORT**

International application No.
**PCT/CN2024/121910**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17, 19**
because they relate to subject matter not required to be searched by this Authority, namely:

The subject matter of claims 17 and 19 (when referring to claim 17) belongs to a method for treating diseases in a human body as defined in PCT Rule 39.1(iv). The search is carried out on the basis of the use of the compound or pharmaceutical composition in the preparation of a drug for treating corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/121910**

| Patent document cited in search report | | | Publication date (day/month/year) | Patent family member(s) | | | Publication date (day/month/year) |
|---|---|---|---|---|---|---|---|
| WO | 2008044033 | A1 | 17 April 2008 | None | | | |
| CN | 109310664 | A | 05 February 2019 | BR | 112018075347 | A2 | 19 March 2019 |
| | | | | RU | 2018139930 | A | 10 July 2020 |
| | | | | RU | 2018139930 | A3 | 29 September 2020 |
| | | | | MX | 2018015179 | A | 14 October 2019 |
| | | | | IL | 263613 | A | 31 January 2019 |
| | | | | IL | 263613 | B | 29 April 2021 |
| | | | | KR | 20230005416 | A | 09 January 2023 |
| | | | | US | 2017368003 | A1 | 28 December 2017 |
| | | | | US | 10314797 | B2 | 11 June 2019 |
| | | | | AU | 2017278314 | A1 | 13 December 2018 |
| | | | | AU | 2017278314 | B2 | 30 June 2022 |
| | | | | JP | 2021138780 | A | 16 September 2021 |
| | | | | JP | 7382987 | B2 | 17 November 2023 |
| | | | | CA | 3024615 | A1 | 14 December 2017 |
| | | | | AU | 2022235644 | A1 | 20 October 2022 |
| | | | | AU | 2022235644 | B2 | 13 June 2024 |
| | | | | JP | 2019522644 | A | 15 August 2019 |
| | | | | JP | 6956747 | B2 | 02 November 2021 |
| | | | | EP | 3468552 | A1 | 17 April 2019 |
| | | | | EP | 3468552 | A4 | 08 July 2020 |
| | | | | KR | 20190016513 | A | 18 February 2019 |
| | | | | KR | 102481319 | B1 | 26 December 2022 |
| | | | | WO | 2017214634 | A1 | 14 December 2017 |
| | | | | MX | 2021003784 | A | 27 May 2021 |
| WO | 2023145769 | A1 | 03 August 2023 | AR | 128338 | A1 | 17 April 2024 |
| | | | | TW | 202340171 | A | 16 October 2023 |
| EP | 0100172 | A1 | 08 February 1984 | JPS | 5933250 | A | 23 February 1984 |
| | | | | JPH | 0432061 | B2 | 28 May 1992 |
| | | | | CS | 399991 | A3 | 17 June 1992 |
| | | | | ES | 8607936 | A1 | 01 June 1986 |
| | | | | LU | 88769 | I2 | 05 November 1996 |
| | | | | US | 4636505 | A | 13 January 1987 |
| | | | | IE | 831732 | L | 23 January 1984 |
| | | | | IE | 55941 | B1 | 27 February 1991 |
| | | | | ZA | 8305182 | B | 30 May 1984 |
| | | | | NL | 950029 | I1 | 01 February 1996 |
| | | | | NL | 950029 | I2 | 03 March 1997 |
| | | | | NO | 1996014 | I1 | 10 December 1996 |
| | | | | FI | 832644 | A0 | 20 July 1983 |
| | | | | FI | 832644 | A | 24 January 1984 |
| | | | | FI | 83770 | B | 15 May 1991 |
| | | | | FI | 83770 | C | 26 August 1991 |
| | | | | ATE | 28864 | T1 | 15 August 1987 |
| | | | | HU | 191296 | B | 27 February 1987 |
| | | | | AU | 1693783 | A | 26 January 1984 |
| | | | | AU | 556328 | B2 | 30 October 1986 |
| | | | | ES | 544189 | A0 | 16 September 1986 |
| | | | | GR | 79232 | B | 22 October 1984 |
| | | | | JPH | 02131462 | A | 21 May 1990 |

Form PCT/ISA/210 (patent family annex) (July 2022)

**INTERNATIONAL SEARCH REPORT**
**Information on patent family members**

International application No.
**PCT/CN2024/121910**

| Patent document cited in search report | Publication date (day/month/year) | Patent family member(s) | Publication date (day/month/year) |
|---|---|---|---|
| | | ZA 835182 B | 30 May 1984 |
| | | ES 8607915 A1 | 01 June 1986 |
| | | EP 0100172 B1 | 12 August 1987 |
| | | ES 524392 A0 | 01 November 1985 |
| | | IL 69217 A0 | 30 November 1983 |
| | | IL 69217 A | 31 March 1987 |
| | | NO 832599 L | 24 January 1984 |
| | | NO 164974 B | 27 August 1990 |
| | | NO 164974 C | 05 December 1990 |
| | | PT 77087 A | 01 August 1983 |
| | | PT 77087 B | 27 January 1986 |
| | | HK 92690 A | 16 November 1990 |
| | | DK 322783 D0 | 13 July 1983 |
| | | CA 1249823 A | 07 February 1989 |
| | | MX 9203451 A | 01 August 1992 |
| | | NZ 204995 A | 30 August 1985 |
| | | DE 3372965 D1 | 17 September 1987 |
| CN 1416416 A | 07 May 2003 | AU 3685601 A | 20 August 2001 |
| | | IL 145728 A0 | 25 July 2002 |
| | | JP 2003522752 A | 29 July 2003 |
| | | JP 4806508 B2 | 02 November 2011 |
| | | DE 10084380 T1 | 20 June 2002 |
| | | AU 2001236856 B2 | 11 August 2005 |
| | | CA 2400185 A1 | 16 August 2001 |
| | | CA 2400185 C | 05 July 2011 |
| | | MXPA 01006376 A | 06 June 2003 |
| | | WO 0158854 A1 | 16 August 2001 |
| | | HK 1051849 A1 | 22 August 2003 |
| | | ATE 259781 T1 | 15 March 2004 |
| | | DE 60008360 D1 | 25 March 2004 |
| | | DE 60008360 T2 | 09 December 2004 |
| | | BR 0008439 A | 23 April 2002 |
| | | BRPI 0008439 B1 | 19 April 2016 |
| | | ES 2215672 T3 | 16 October 2004 |
| | | EP 1169301 A1 | 09 January 2002 |
| | | EP 1169301 B1 | 18 February 2004 |
| | | IL 145728 A | 13 April 2008 |
| | | KR 20020089347 A | 29 November 2002 |
| | | KR 100508681 B1 | 17 August 2005 |
| | | RU 2225390 C2 | 10 March 2004 |
| | | ES 2187390 A1 | 01 June 2003 |
| | | ES 2187390 B2 | 01 April 2004 |
| | | WO 0158855 A1 | 16 August 2001 |
| | | WO 0158855 A9 | 31 October 2002 |
| | | US 6472415 B1 | 29 October 2002 |
| | | HK 1043692 A1 | 20 September 2002 |
| | | CZ 20012398 A3 | 14 November 2001 |
| | | CZ 301910 B6 | 28 July 2010 |
| | | AU 5302600 A | 20 August 2001 |
| | | ZA 200206497 B | 14 August 2003 |

Form PCT/ISA/210 (patent family annex) (July 2022)

**INTERNATIONAL SEARCH REPORT**
**Information on patent family members**

International application No.
**PCT/CN2024/121910**

| Patent document cited in search report | Publication date (day/month/year) | Patent family member(s) | Publication date (day/month/year) |
|---|---|---|---|
| | | PL 350356 A1 | 02 December 2002 |
| | | PL 201173 B1 | 31 March 2009 |
| WO 2023011596 A1 | 09 February 2023 | TW 202312993 A | 01 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311282820X
- CN 202410900706


### Non-patent literature cited in the description


- **MAEHR**. *J. Chem. Ed*, 1985, vol. 62, 114-120